Europäisches Patentamt
European Patent Office
Office européen des brevets

(19)

(11) Publication number: **0 265 185 A2**

# EUROPEAN PATENT APPLICATION

(12)

(21) Application number: 87309153.2

(22) Date of filing: 15.10.87

(51) Int. Cl.[4]: C 07 D 501/26
C 07 D 501/36,
C 07 D 501/38,
C 07 D 501/57,
A 61 K 31/545,
C 07 D 498/04, C 07 D 471/04
//(C07D498/04,265:00,205:00),
(C07D471/04,221:00,205:00)

(30) Priority: 21.10.86 GB 8625188
25.10.86 GB 8625595
10.02.87 GB 8702961
17.03.87 GB 8706294

(43) Date of publication of application:
27.04.88 Bulletin 88/17

(84) Designated Contracting States:
AT BE CH DE ES FR GB GR IT LI LU NL SE

(71) Applicant: BEECHAM GROUP PLC
Beecham House Great West Road
Brentford Middlesex TW8 9BD (GB)

(72) Inventor: Corbett, David Francis
Beecham Pharmaceuticals Brockham Park
Betchworth Surrey, RH3 7AJ (GB)

Frydrych, Colin Henry
Beecham Pharmaceuticals Brockham Park
Betchworth Surrey, RH3 7AJ (GB)

Southgate, Robert
Beecham Pharmaceuticals Brockham Park
Betchworth Surrey, RH3 7AJ (GB)

(74) Representative: Lockwood, Barbara Ann et al
Beecham Pharmaceuticals Biosciences Research
Centre Great Burgh Yew Tree Bottom Road
Epsom Surrey KT18 5XQ (GB)

The title of the invention has been amended (Guidelines for Examination in the EPO, A-III, 7.3).

(54) **Cephalosporins, processes for their preparation and pharmaceutical compositions containing them.**

(57) Antibacterial agents have the formula (Ia) or are pharmaceutically acceptable salts or in vivo hydrolysable esters thereof:

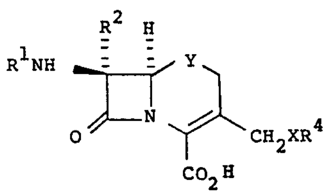

(Ia)

wherein $R^1$ is an acyl group, in particular that of an antibacterially active cephalosporin; $R^2$ is hydrogen, methoxy, or formamido; Y is S, SO, $SO_2$, O or $CH_2$; X is oxygen, sulphur, or -NH- and $R^4$ is a group of the formula CO Z $R^5$ wherein Z is -CH=CH-, $-(CH_2)n-$ or -NH-; n is 0, 1 or 2; and $R^5$ is:

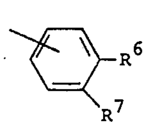
,
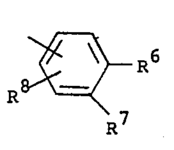
or
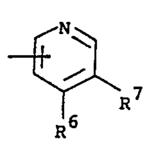

wherein $R^6$ and $R^7$ are the same or different, each representing hydroxy or protected hydroxy and $R^8$ is hydroxy, amino, halogen or carboxy.

The use of the compounds of formula (Ia) and intermediates for their preparation are also disclosed.

EP 0 265 185 A2

**Description**

NOVEL COMPOUNDS

This invention relates to a class of novel cephalosporins which have antibacterial activity and are of value in the treatment of infections in animals, especially mammals including man, caused by a wide range of organisms, particularly Gram-negative organisms. The invention also relates to a process for the preparation of such compounds, intermediates for use in the preparation of the compounds and to pharmaceutical compositions comprising the antibacterially active compounds.

European Patent Application Publication Number 0071395 discloses a class of β-lactam antibiotics having an α-formamido (formamidyl) substituent on the carbon atom adjacent to the carbonyl group of the β-lactam ring.

In addition, an antibacterial cephalosporin known as cefotaxime has the formula (A):

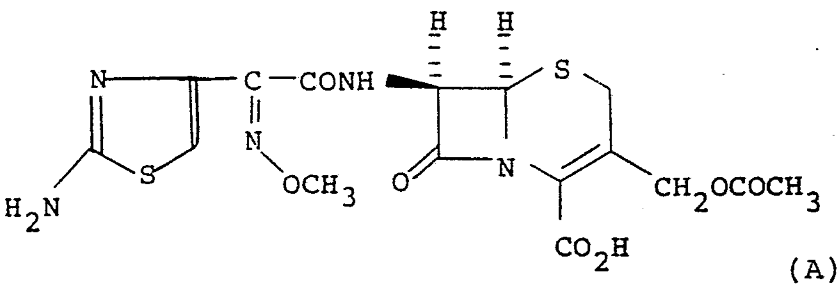

(A)

Compound (A) and related cephem compounds are described in U.K. Patent Specification No. 1 536 281.

The present invention provides a cephalosporin of the formula (I) or a salt thereof:

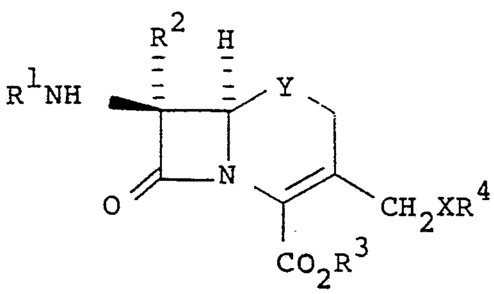

(I)

wherein $R^1$ is an acyl group, in particular that of an antibacterially active cephalosporin; $R^2$ is hydrogen, methoxy, or formamido; $R^3$ is hydrogen or a readily removable carboxy protecting group; Y is S, SO, $SO_2$, OO or $CH_2$; X is oxygen, sulphur, or -NH- and $R^4$ is a group of the formula-CO-Z-$R^5$ wherein Z is -CH=CH-, $-(CH_2)_n-$ or -NH-; n is 0, 1 or 2; and $R^5$ is:

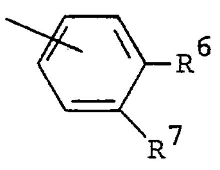

,

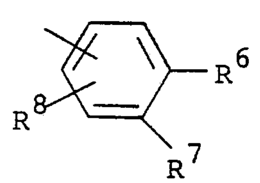

or

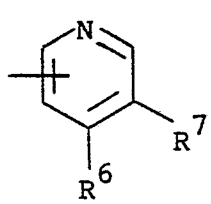

wherein $R^6$ and $R^7$ are the same or different, each representing hydroxy or protected hydroxy and $R^8$ is hydroxy, amino, halogen or carboxy.

The compounds of the invention have been found to possess outstanding antibacterial properties.

The term 'halogen' refers to fluorine, chlorine, bromine and iodine.

In compounds of formula (I), the formamido group can exist in conformations wherein the hydrogen atoms of the -NH-CHO moiety are cis- or trans-; of these the cis conformation normally predominates.

Since the β-lactam antibiotic compounds of the present invention are intended for use in pharmaceutical compositions, it will be readily appreciated that preferred compounds within formula (I) are pharmaceutically acceptable, i.e. are compounds of formula (Ia) or pharmaceutically acceptable salts or pharmaceutically acceptable in vivo hydrolysable esters thereof:

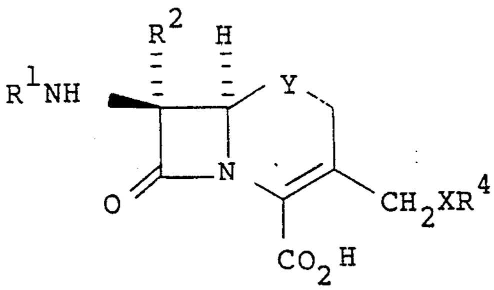

(Ia)

wherein $R^1$, $R^2$, Y, X and $R^4$ are as defined with respect to formula (I).

Those compounds of the formula (I) wherein $R^3$ is a readily removable carboxy protecting group or a non-pharmaceutically acceptable salt are primarily useful as intermediates in the preparation of compounds of the formula (Ia) or a pharmaceutically acceptable salt or pharmaceutically acceptable in vivo hydrolysable ester thereof.

Suitable readily removable carboxy protecting groups for the group $R^3$ include groups forming ester derivatives of the carboxylic acid, including in vivo hydrolysable esters. The derivative is preferably one which may readily be cleaved.

Suitable ester-forming carboxyl-protecting groups are those which may be removed under conventional conditions. Such groups for $R^3$ include benzyl, p-methoxybenzyl, benzoylmethyl, p-nitrobenzyl, 4-pyridylmethyl, 2,2,2-trichloroethyl, 2,2,2-tribromoethyl, t-butyl, t-amyl, allyl, diphenylmethyl, triphenylmethyl, adamantyl, 2-benzyloxyphenyl, 4-methylthiophenyl, tetrahydrofur-2-yl, tetrahydropyran-2-yl, pentachlorophenyl, acetonyl, p-toluenesulphonylethyl, methoxymethyl, a silyl, stannyl or phosphorus-containing group, an oxime radical of formula $-N=CHR^9$ where $R^9$ is aryl or heterocyclic, or an in vivo hydrolysable ester radical such as defined below.

When used herein the term 'aryl' includes phenyl and naphthyl, each optionally substituted with up to five, preferably up to three, groups selected from halogen, mercapto, $C_{1-6}$ alkyl, phenyl, $C_{1-6}$ alkoxy, hydroxy($C_{1-6}$)alkyl, mercapto($C_{1-6}$)alkyl, halo($C_{1-6}$) alkyl, hydroxy, amino, nitro, carboxy, $C_{1-6}$alkylcarbonyloxy, formyl, or $C_{1-6}$ alkylcarbonyl groups.

The terms 'heterocyclyl' and 'heterocyclic' as used herein include aromatic and non-aromatic, single and fused, rings suitably containing up to four hetero-atoms in each ring selected from oxygen, nitrogen and sulphur, which rings may be unsubstituted or substituted by, for example, up to three groups selected from halogen, ($C_{1-6}$)alkyl, ($C_{1-6}$)alkoxy, halo($C_{1-6}$)alkyl, hydroxy, carboxy, carboxy salts, carboxy esters, ($C_{1-6}$)alkoxycarbonyl, ($C_{1-6}$)alkoxycarbonyl($C_{1-6}$)alkyl, aryl, and oxo groups.

Each heterocyclic ring suitably has from 4 to 7, preferably 5 or 6, ring atoms. A fused heterocyclic ring system may include carbocyclic rings and need include only one heterocyclic ring. Compounds within the invention containing a heterocyclyl group may occur in two or more tautometric forms depending on the nature of the heterocyclyl group; all such tautomeric forms are included within the scope of the invention.

When used herein the term 'alkyl' (or 'lower alkyl') includes straight and branched chain alkyl groups containing from 1 to 6 carbon atoms, such as methyl, ethyl, propyl and butyl. A particular alkyl group is methyl.

A carboxyl group may be regenerated from any of the above esters by usual methods appropriate to the particular $R^3$ group, for example, acid- and base-catalysed hydrolysis, or by enzymically-catalysed hydrolysis, or by hydrogenolysis under conditions wherein the remainder of the molecule is substantially unaffected.

Examples of suitable pharmaceutically acceptable in vivo hydrolysable ester groups include those which break down readily in the human body to leave the parent acid or its salt. Suitable ester groups of this type include those of part formula (i), (ii), (iii) and (iv):

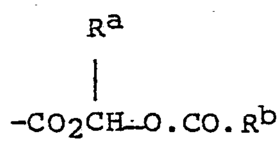

(i)

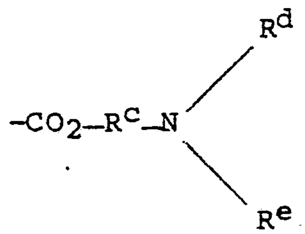

(ii)

$-CO_2CH_2-OR^f$ (iii)

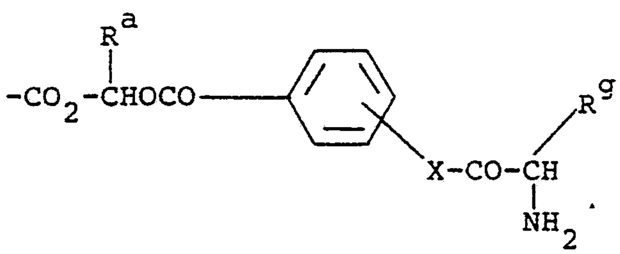

(iv)

wherein $R^a$ is hydrogen, $C_{1-6}$ alkyl, $C_{3-7}$ cycloalkyl, methyl, or phenyl, $R^b$ is $C_{1-6}$ alkyl, $C_{1-6}$ alkoxy, phenyl, benzyl, $C_{3-7}$ cycloalkyl, $C_{1-6}$ alkyl $C_{3-7}$cycloalkyl, 1-amino $C_{1-6}$ alkyl, or 1-($C_{1-6}$ alkyl)amino $C_{1-6}$ alkyl; or $R^a$ and $R^b$ together form a 1,2-phenylene group optionally substituted by one or two methoxy groups; $R^c$ represents $C_{1-6}$ alkylene optionally substituted with a methyl or ethyl group and $R^d$ and $R^e$independently represent $C_{1-6}$ alkyl; $R^f$ represents $C_{1-6}$alkyl; $R^g$ represents hydrogen or phenyl optionally substituted by up to three groups selected from halogen, $C_{1-6}$ alkyl, or $C_{1-6}$ alkoxy; and Q is oxygen or NH.

Examples of suitable in vivo hydrolysable ester groups include, for example, acyloxyalkyl groups such as acetoxymethyl, pivaloyloxymethyl, α-acetoxyethyl, α-pivaloyloxyethyl, 1-(cyclohexylcarbonyloxy)prop-1-yl, and (1-aminoethyl)carbonyloxymethyl; alkoxycarbonyloxyalkyl groups, such as ethoxycarbonyloxymethyl and α-ethoxycarbonyloxyethyl; dialkylaminoalkyl especially di-loweralkylamino alkyl groups such as dimethylaminomethyl, dimethylaminoethyl, diethylaminomethyl or diethylaminoethyl; lactone groups such as phthalidyl and dimethoxyphthalidyl; and esters linked to a second β-lactam antibiotic or to a β-lactamase inhibitor.

A further suitable pharmaceutically acceptable in vivo hydrolysable ester group is that of the formula:

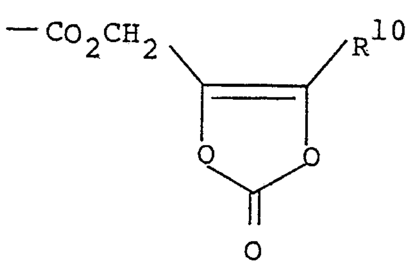

wherein $R^{10}$ is hydrogen, $C_{1-6}$ alkyl or phenyl.

Suitable pharmaceutically acceptable salts of the carboxy group of the compound of formula (I) include metal salts, eg aluminium, alkali metal salts such as sodium or potassium, alkaline earth metal salts such as calcium or magnesium, and ammonium or substituted ammonium salts, for example those with lower alkylamines such as triethylamine, hydroxy-lower alkylamines such as 2-hydroxyethylamine, bis-(2-hydroxyethyl)-amine or tris-(2-hydroxyethyl)amine, cycloalkylamines such as dicyclohexylamine, or with procaine,

dibenzylamine, N,N-dibenzylethylenediamine, 1-ephenamine, N-methylmorpholine, N-ethylpiperidine, N-benzyl-β- phenethylamine, dehydroabietylamine, N,N'-bisdehydro-abietylamine, ethylenediamine, or bases of the pyridine type such as pyridine, collidine or quinoline, or other amines which have been used to form salts with known penicillins and cephalosporins. Other useful salts include the lithium salt and silver salt. Salts within compounds of formula (I), may be prepared by salt exchange in conventional manner.

In one preferred aspect of the invention the group $R^5$ is dihydroxyphenyl, for example 2,3-dihydroxyphenyl or 3,4-dihydroxyphenyl, preferably 3,4-dihydroxyphenyl, in which one or both of the hydroxy groups may optionally be protected. When both hydroxy groups are protected it will be understood that a different protecting group may be used for each hydroxy group, although, more conveniently, the protecting groups used will be the same.

Examples of suitable hydroxy protecting groups include formyl and optionally substituted $C_{1-6}$ alkylcarbonyl and arylcarbonyl groups such as acetyl, chloroacetyl, dichloroacetyl and benzoyl; optionally substituted $C_{1-6}$alkoxycarbonyl and aryl $C_{1-6}$ alkoxycarbonyl, for example ethoxycarbonyl, trimethylsilylethoxycarbonyl, benzyloxycarbonyl and 4-nitrobenzyloxycarbonyl; and optionally substituted $C_{2-6}$ alkenyloxycarbonyl such as allyloxycarbonyl.

Further examples of suitable hydroxy-protecting groups include aryl, aryl $C_{1-6}$ alkyl, optionally substituted $C_{1-6}$ alkyl, optionally substituted $C_{2-6}$ alkenyl, optionally substituted $C_{3-7}$ cycloalkyl, and silyl groups.

Some examples of optional substituents in groups mentioned hereinabove as being optionally substituted include up to three groups (which may be the same or different) chosen from halogen, hydroxy, $C_{1-6}$ alkoxy, $C_{1-6}$ alkylthio, cyano, nitro, carboxy, carboxylic acid $C_{1-6}$ alkyl ester, carbamoyl, amino, mono ($C_{1-6}$) alkylamino, and di ($C_{1-6}$) alkylamino.

Preferred alkyl protecting groups for a hydroxy group in $R^5$ include, for example, methyl or ethyl, optionally substituted with ($C_{1-6}$) alkoxy or ($C_{1-6}$) alkylthio, for example with methoxy, ethoxy, or methylthio. Further useful protecting groups are methoxyethoxymethyl and (trimethylsilyl)ethoxymethyl. In addition, when $R^5$ is 3,4-dihydroxyphenyl, the hydroxy groups may be protected by an alkylene bridge so that $R^5$ becomes, for example, 3,4-[1,1-dimethyl-(methylenedioxy)]phenyl.

Suitable aryl $C_{1-6}$ alkyl protecting groups for a hydroxy group in $R^5$ include benzyl, 4-nitrobenzyl and 4-methoxybenzyl.

The silyl protecting groups mentioned above may be substituted with $C_{1-6}$ alkyl and/or phenyl groups and include, for example, trimethylsilyl, t-butyldimethylsilyl, t-butyldiphenylsilyl, triethylsilyl, isopropyldimethylsilyl, triphenylmethyldimethylsilyl and the like. The resulting silyl ethers may be cleaved by methods known in the art, such as those described by T. W. Greene in "Protective Groups in Organic Synthesis" (Wiley-Interscience, New York, 1981), or by M. Lalonde and T.H. Chan in Synthesis, 1985 (September), pages 817-845 and references therein.

Particularly preferred hydroxy protecting groups are acetyl and trimethylsilyl.

It will be appreciated that the bond connecting the group $R^5$ to the group Z may be attached to any carbon atom in the substituted benzene or pyridine ring which is not substituted by groups $R^6$, $R^7$ or $R^8$.

In another preferred aspect of the invention the group $R^5$ is a group of formula:

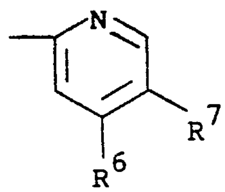

wherein $R^6$ and $R^7$ are as hereinabove defined.

When $R^6$ and $R^7$ are hydroxy, it will be understood that the 4,5-dihydroxypyridin-2-yl group may exist in different tautomeric forms, such as the 5-hydroxy-4-oxo-1,4-dihydropyridin-2-yl group of formula:

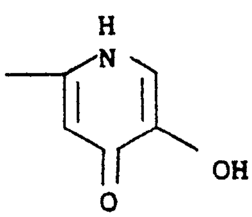

Any of such tautomeric forms, and mixtures thereof, may be present in compounds of the invention.

When $R^6$ and $R^7$ are protected hydroxy, suitable hydroxy protecting groups include those listed above.

In compounds of formula (I) or (Ia), the group Y may be sulphur or an oxidised sulphur atom, i.e. a sulphoxide (S-O) or sulphone ($SO_2$) group. When Y is a sulphoxide group it will be understood that α- and

β-isomers may exist; both such isomers are encompassed within the scope of the present invention.

Preferably Y is sulphur.

Preferably the group X is oxygen or -NH.

When the group Z is $-(CH_2)_n-$ where n is as hereinbefore defined, n is preferably zero.

When $R^4$ has the formula $-COCH=CHR^5$ geometric isomers can exist depending on the arrangement of the groups attached to the double bond. Both cis and trans isomers are included within the scope of the invention.

From the foregoing, it may be seen that in one preferred aspect of the invention there is provided a compound of the formula (II) or a pharmaceutically acceptable salt or pharmaceutically acceptable in vivo hydrolysable ester thereof:

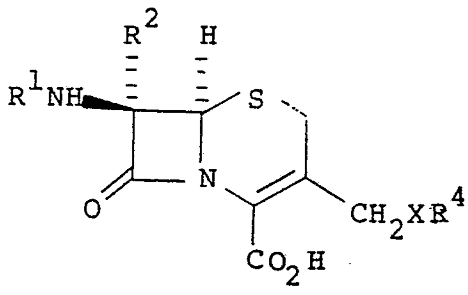

(II)

wherein $R^1$, $R^2$, X and $R^4$ are as hereinbefore defined.

Suitable acyl groups $R^1$ for inclusion in the compounds of formula (I) include those of the formula $R^{11}CO-$ wherein $R^{11}CO-$ has one of the subformulae (a)-(e):

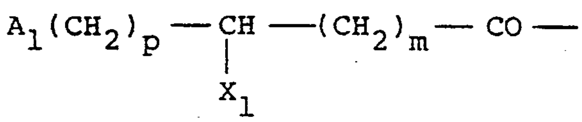

(a)

$A_2CO-$ (b)

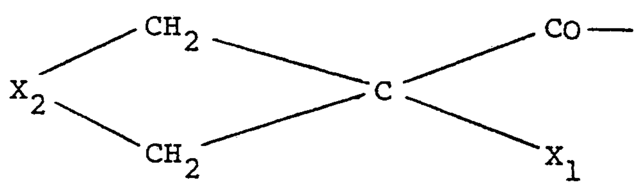

(c)

$A_2-X_3-(CH_2)_p-CO-$ (d)

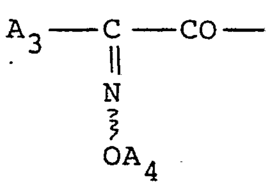

(e)

wherein p is 0, 1 or 2; m is 0, 1 or 2; $A_1$ is $C_{1-6}$alkyl, substituted $C_{1-6}$ alkyl, $C_{3-6}$ cycloalkyl, cyclohexenyl, cyclohexadienyl, an aromatic (including heteroaromatic) group, such as phenyl, substituted phenyl, thienyl, pyridyl, or an optionally substituted thiazolyl group, a $C_{1-6}$ alkylthio group or $C_{1-6}$alkyloxy; $X_1$ is a hydrogen or halogen atom, a carboxylic acid, carboxylic ester, sulphonic acid, azido, tetrazolyl, hydroxy, acyloxy, amino, ureido, acylamino, heterocyclylamino, guanidino or acylureido group; $A_2$ is an aromatic group, for example a

phenyl, 2,6-dimethoxyphenyl,2-alkoxy-1- naphthyl, 3-arylisoxazolyl, or a 3-aryl-5-methylisoxazolyl group, such as 3-(2-chloro-6-fluorophenyl) 5-methylisoxazol-4-yl; a substituted alkyl group; or a substituted dithietane; $X_2$is a $CH_2OCH_2$, $CH_2SCH_2$ or alkylene group; $X_3$ is an oxygen or sulphur atom; $A_3$ is an aryl or heteroaryl group such as phenyl, substituted phenyl or aminothiazolyl; and $A_4$ is hydrogen, $C_{1-6}$alkyl, $C_{3-6}$ cycloalkyl, arylaminocarbonyl, $C_{1-6}$alkylaminocarbonyl, $C_{1-6}$ alkanoyl, $C_{1-6}$ alkoxycarbonyl, $C_{2-6}$ alkenyl, carboxy $C_{1-6}$ alkyl, $C_{1-6}$ alkylsulphonyl, di-$C_{1-6}$ alkylphosphatomethyl, and aryl.

The term 'heteroaryl' as used herein means a heteroaromatic heterocyclic ring or ring system, suitably having 5 or 6 ring atoms in each ring.

More suitably $A_1$ is $C_{1-6}$ alkyl, $C_{3-6}$ cycloalkyl, cyclohexenyl, cyclohexadienyl, phenyl, hydroxyphenyl, thienyl or pyridyl; and $X_1$ is hydrogen, or halogen, carboxy, carboxylic ester, azido, tetrazolyl, hydroxy, acyloxy, amino, ureido, guanidino or acylureido.

In compounds of formula (I) in which $R^1$ is an acyl group of formula $R^{11}CO$, preferred values of the group $R^{11}$ depend on the value of the group $R^2$.

When $R^2$ is hydrogen, $R^{11}$ is preferably a group of the subformula:

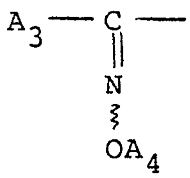

wherein $A_3$ and $A_4$ are as defined with respect to the group of subformula (e) above.

Suitable values for the group $A_3$ include those commonly found in antibacterially active cephalosporins containing a hydroxyimino or substituted hydroxyimino group in the side chain attached to C-7, for example phenyl, thien-2-yl, thien-3-yl, fur-2-yl, fur-3-yl, pyrid-2-yl, pyrid-3-yl, pyrid-4-yl, and 2-aminothiazol-4-yl in which the amino group is optionally protected.

Preferred groups for $A_3$ include phenyl, 2-aminothiazol-4-yl, fur-2-yl, thien-2-yl, and 2-(2-chloroacetamido)thiazol-4-yl.

A particularly preferred group for $A_3$ is 2-aminothiazol-4-yl.

Suitable values for the group $A_4$ include methyl, ethyl, cyclopentyl, cyclohexyl, phenyl and carboxypropyl.

Preferred values of $A_4$ include methyl, cyclopentyl and 2-carboxypropan-2-yl.

It will be appreciated that compounds of formula (I) in which $R^1$ is an acyl group $R^{11}CO$ having the formula (e):

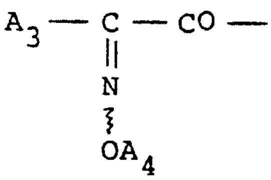

(e)

can exist as syn and anti isomers or mixtures thereof. Both isomers are encompassed within the scope of this invention.

Preferably the compounds of the invention have the syn configuration (i.e. have the group $OA_4$ syn to the amide linkage) or are enriched in that isomer.

From the foregoing it will be seen that one preferred subclass of compounds within the invention may be represented by the formula (III) or a pharmaceutically acceptable salt or pharmaceutically acceptable in vivo hydrolysable ester thereof:

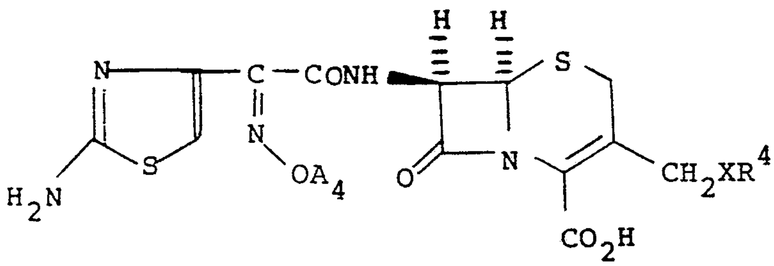

(III)

wherein $R^4$, X, and $A^4$ are as hereinbefore defined,

In a particularly preferred aspect of the invention the group $R^4$ in a compound of the formula (III) is 3,4-dihydroxybenzoyl and the group X is oxygen.

When $R^2$ in compounds of formula (I) is methoxy or formamido, and $R^1$ is an acyl group of formula $R^{11}CO$, favoured values for the group $R^{11}$ include those of subformulae (f) and (g) below:

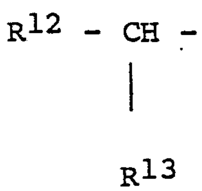

(f)

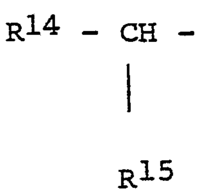

(g)

wherein $R^{12}$ is a phenyl, thienyl or phenoxy group; $R^{13}$ is a hydrogen atom or methyl group; $R^{14}$ is a phenyl, substituted phenyl, substituted thiazolyl, thienyl or cyclohexadienyl group; and $R^{15}$ is a hydroxyl, carboxylic acid group or lower alkyl or phenyl, tolyl or indanyl ester thereof, amino or a substituted amino group.

Suitably the substituted phenyl group for $R^{14}$ is a phenyl group substituted with up to three groups selected from $C_{1-6}$ alkyl, phenyl, halogen, $C_{1-6}$ alkoxy, amino, nitro, hydroxy, $C_{1-6}$ alkylamido, $C_{1-6}$alkylcarbonyloxy, carboxy, $C_{1-6}$ alkoxycarbonyl, halo ($C_{1-6}$) alkyl, oxo ($C_{1-6}$) alkyl, $C_{1-6}$ alkylcarbonyl, aryloxy, aralkyloxy, arylcarbonyl, $C_{1-6}$ alkylamino or di($C_{1-6}$)alkylamino.

Preferably $R^{14}$ is a phenyl or thienyl group.

Preferably $R^{15}$ is a substituted amino group.

More preferably the substituted amino group $R^{15}$ is a ureido, acylamino or acylureido group.

One suitable sub-group within the present invention provides a compound of formula (IV) or a pharmaceutically acceptable salt or in vivo hydrolysable ester thereof:

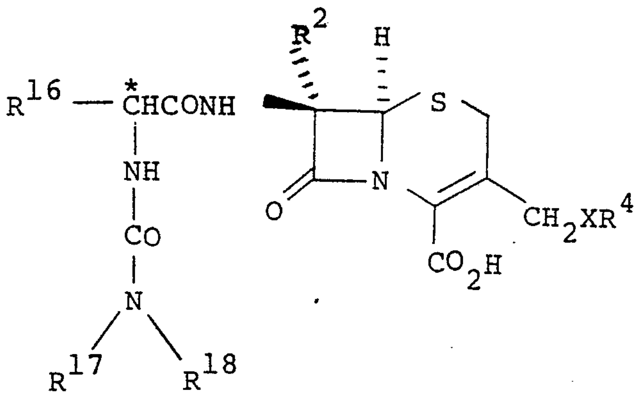

(IV)

wherein $R^2$ is methoxy or formamido; $R^4$ and X are as defined with respect to formula (I); $R^{16}$ is phenyl, substituted phenyl, cyclohexenyl, cyclohexadienyl, or a 5- or 6-membered heterocyclic ring containing up to three hetero-atoms selected from oxygen, sulphur or nitrogen, optionally substituted with hydroxy, amino, halogen, $C_{1-6}$ alkylamino, di($C_{1-6}$alkyl)amino, or $C_{1-6}$alkoxy; $R^{17}$ is hydrogen or a $C_{1-6}$ alkyl group and $R^{18}$ is an optionally substituted 5- or 6- membered heterocyclic group containing one or two nitrogen heteroatoms; or $R^{17}$ and $R^{18}$ together with the nitrogen atom to which they are attached form an optionally substituted five- or six-membered heterocyclic group containing one or two nitrogen heteroatoms.

The carbon atom marked * in formulae herein is asymmetric and thus compounds of formula (IV) may exist as two optically active diastereoisomers. In general the isomer prepared from the D-side chain exhibits the highest antibacterial activity and accordingly the D compound or the DL mixtures are preferred, with the D compound being particularly preferred.

Compounds with the preferred D-side- chain can be separated from a mixture of both diastereoisomers by conventional methods, or prepared from intermediates that bear a D- side chain.

Suitably the substituted phenyl group for $R^{16}$ is a phenyl group substituted with up to three groups selected from $C_{1-6}$ alkyl, phenyl, halogen, $C_{1-6}$ alkoxy, amino, nitro, hydroxy, $C_{1-6}$ alkylamido, $C_{1-6}$alkylcarbonyloxy, carboxy, $C_{1-6}$ alkoxycarbonyl, halo ($C_{1-6}$) alkyl, oxo ($C_{1-6}$) alkyl, $C_{1-6}$ alkylcarbonyl, aryloxy, aralkyloxy, arylcarbonyl, $C_{1-6}$ alkylamino or di($C_{1-6}$) alkylamino.

In one favoured group of compounds according to the invention $R^{16}$ is phenyl, 3-hydroxyphenyl, 3-tri($C_{1-6}$alkyl)silyloxyphenyl, 3-($C_{1-6}$ alkylcarbonyloxy)phenyl, 3-($C_{1-6}$ alkyloxycarbonyloxy)phenyl, 3-(benzyloxycarbonyloxy)phenyl, 3-(4-nitrobenzyloxycarbonyloxy)phenyl, 3-aminophenyl, 4-hydroxyphenyl, 4-tri($C_{1-6}$alkyl)silyloxyphenyl, 4-($C_{1-6}$ alkylcarbonyloxy)phenyl, 4-($C_{1-6}$ alkyloxycarbonyloxy)phenyl, 4-(benzyloxycarbonyloxy)phenyl, 4-(4-nitrobenzyloxy-carbonyloxy)phenyl, 4-aminophenyl, 3,4-dihydroxyphenyl, 3,4-bis-[tri($C_{1-6}$alkyl)silyloxy]phenyl, 3,4-di($C_{1-6}$alkylcarbonyloxy)phenyl, 3,4-di-($C_{1-6}$alkyloxy-carbonyloxy)phenyl, 3,4-di-(benzyloxycarbonyloxy)- phenyl, or 3,4-di-(4-nitrobenzyloxycarbonyloxy)phenyl.

Preferred groups for $R^{16}$ in formula (IV) are phenyl, thien-2-yl, thien-3-yl, 4-hydroxyphenyl and 4-acetoxyphenyl.

Particularly preferred groups $R^{16}$ are phenyl and thien-2-yl.

Suitably $R^{17}$ is hydrogen.

Suitable substituents for the 5- or 6- membered heterocyclic group of $R^{18}$ or $R^{17}$ and $R^{18}$ together include the optionally substituted alkyl, alkenyl, alkynyl, cycloalkyl or cycloalkenyl group; optionally substituted phenyl, oxo; the hydroxy group optionally substituted by alkyl, alkenyl, cycloalkyl, phenyl, pyridyl, pyrimidyl or benzyl; the optionally substituted mercapto group, the alkylsulphonyl group; the substituted imino group; or the amino group optionally substituted by an alkyl, alkenyl, cycloalkyl, phenyl, substituted phenyl or benzyl group. Alternatively two substituents on the ring may form the residue of a further carbocyclic or heterocyclic ring.

In a preferred aspect of the invention, compounds within formula (IV) have a formamido (-NHCHO) group as the group $R^2$.

A preferred sub-group within the present invention provides a compound of formula (V) or a pharmaceutically acceptable salt or in vivo hydrolysable ester thereof:

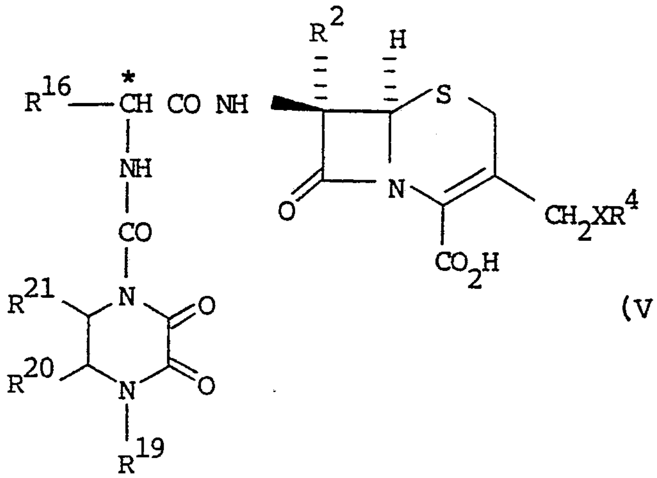

(V)

wherein $R^2$ is methoxy or formamido, $R^4$ and X are as defined with respect to formula (I), $R^{16}$ is as defined with respect to formula (IV) and $R^{19}$ represents hydrogen, $C_{1-6}$ alkyl, substituted alkyl, aryl, or aralkyl; $R^{20}$ and $R^{21}$ are the same or different and represent hydrogen, $C_{1-6}$ alkyl, substituted alkyl, halogen, amino, hydroxy or $C_{1-6}$ alkoxy or $R^{20}$ and $R^{21}$ form the residue of 5- or 6- membered carbocyclic or heterocyclic ring.

Suitable $C_{1-6}$ alkyl groups for the groups $R^{19}$, $R^{20}$ and $R^{21}$ in formula (V) include methyl, ethyl, n- and iso-propyl, n, sec-, iso- and tert-butyl. Preferably $R^{19}$ is ethyl. Preferably $R^{20}$ and $R^{21}$ are hydrogen.

From the foregoing it may be seen that further particularly preferred compounds within the invention are compounds of formula (VI) or a pharmaceutically acceptable salt or in vivo hydrolysable ester thereof:

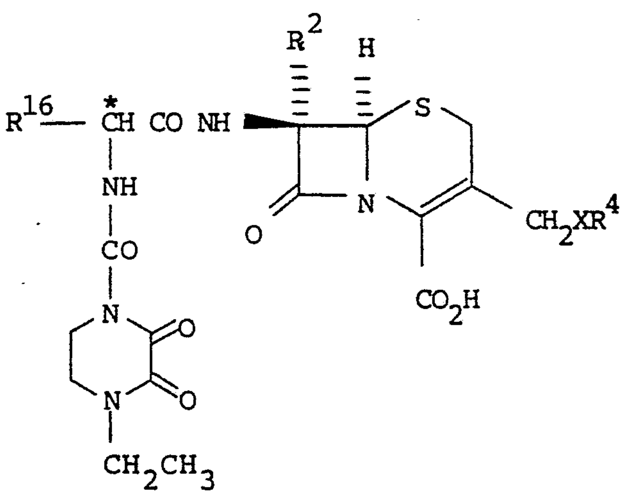

(VI)

wherein $R^2$ is methoxy or formamido, and $R^4$, X, $R^{16}$ and * are as hereinabove defined.

Preferred values of $R^4$ in compounds of formula (VI) include 3,4-dihydroxybenzoyl and (5-hydroxy-4-oxo-1,4-dihydropyridin-2-yl)carbonyl.

Preferably the group X is oxygen or NH.

Specific compounds within this invention of formula (Ia) include the following and pharmaceutically acceptable salts and pharmaceutically acceptable in vivo hydrolysable esters thereof:

(6R, 7R)-3-(3,4-Dihydroxybenzoyloxy)methyl-7-[(R)-2-(4-ethyl-2,3-dioxopiperazin-1-yl)carbonylamino-2-phenylacetamido]-7-formamidoceph-3-em-4-carboxylic acid;

(6R, 7R)-3-(3,4-Dihydroxycinnamoyloxy)methyl-7-[(R)-2-(4-ethyl-2,3-dioxopiperazin-1-yl)carbonylamino-2-phenylacetamido]-7-formamidoceph-3-em-4-carboxylic acid;

(6R,7R)-3-(3,4-Diacetoxybenzoyl)aminomethyl-7-[(R)-2-(4-ethyl-2,3-dioxopiperazin-1-ylcarbonylamino)-2-phenylacetamido]-7-formamidoceph-3-em-4-carboxylic acid;

(6R, 7R)-3-[(E)-3,4-Diacetoxycinnamoyl]aminomethyl-7-[(R)-2-(4-ethyl-2,3-dioxopiperazin-1-ylcarbonylamino)-2-phenylacetamido]-7-formamido-ceph-3-em-4-carboxylic acid;

(6R,7R)-3-(3,4-Dihydroxybenzoyl)aminomethyl-7-[(R)-2-(4-ethyl-2,3-dioxopiperazin-1-ylcarbonylamino)-2-phenylacetamido]-7-formamido-ceph-3-em-4-carboxylic acid;

(6R,7R)-3-(5-Hydroxy-4-oxo-1,4-dihydropyridin-2-yl)carbonylaminomethyl-7-[(R)-2-(4-ethyl-2,3-dioxopiperazin-1-ylcarbonylamino)-2-phenylacetamido]-7-formamidoceph-3-em-4-carboxylic acid;
(6R,7R)-3-(2,3-Dihydroxybenzoyl)aminomethyl-7-[(R)-2-(4-ethyl-2,3-dioxopiperazin-1-ylcarbonyl amino)-2-phenylacetamido]-7-formamidoceph-3-em-4-carboxylic acid;
[6R, 7R]-7-[2-(2-amino-4-thiazolyl)-2((Z)-methoxyimino)acetamido]-3-(3,4-dihydroxybenzoyloxy)methylceph-3-em-4-carboxylic acid;
(6R, 7S)-3-[(3,4-Dihydroxybenzoyloxy)methyl]-7-[(R)-2-(4-ethyl-2,3-dioxopiperazin-1-yl)carbonylamino-2-phenylacetamido]-7-methoxyceph-3-em-4-carboxylic acid;
(6R, 7R)-3-(3,4-Dihydroxybenzoyloxy)methyl-7-[(S)-2-(4-ethyl-2,3-dioxopiperazin-1-yl)carbonylamino-2-(2-thienyl)acetamido]-7-formamidoceph-3-em-4-carboxylic acid;
(6R, 7R)-3-[(3,4-dihydroxyphenyl)acetoxymethyl]-7-[(R)-2-(4-ethyl-2,3-dioxopiperazin-1-yl)carbonylamino-2-phenylacetamido]-7-formamidoceph-3-em-4-carboxylic acid;
(6R, 7R)-7-[(R)-2-(4-Ethyl-2,3-dioxopiperazin-1-yl)carbonylamino-2-phenylacetamido]-7-formamido-3-(3,4,5-trihydroxybenzoyloxy)methylceph-3-em-4-carboxylic acid;
(6R,7R)-3-[(3,4-Dihydroxyphenyl)carbamoyloxymethyl]-7-[(R)-2-(4-ethyl-2,3-dioxopiperazin-1-yl)carbonylamino-2-phenylacetamido]-7-formamidoceph-3-em-4-carboxylic acid;
(6R,7R)-3-(2,3-Dihydroxycinnamoyloxy)methyl-7-[(R)-2-(4-ethyl-2,3-dioxopiperazin-1-yl)carbonylamino-2-phenylacetamido]-7-formamidoceph-3-em-4-carboxylic acid;
(6R,7R)-7-[(R)-2-(4-Ethyl-2,3-dioxopiperazin-1-yl)carbonylamino-2-phenylacetamido]-7-formamido-3-(5-hydroxy-4-oxo-1,4-dihydropyridin-2-yl)carbonyloxymethylceph-3-em-4-carboxylic acid;
(6R,7R)-7-[(R)-2-(4-Ethyl-2,3-dioxopiperazin-1-ylcarbonylamino)-2-phenylacetamido]-7-formamido-3-[(E)-3-(5-hydroxy-4-oxo-1,4-dihydropyridin-2-yl)prop-2-enoyloxymethyl]ceph-3-em-4-carboxylic acid;
[6R,7R]-3-(3,4-Dihydroxybenzoyloxy)methyl-7-(2-phenylacetamido)ceph-3-em-4-carboxylic acid;
[6R,7R]-7-[Z-2-(2-aminothiazol-4-yl)-2-cyclopentyloxyiminoacetamido]3-(3,4-dihydroxybenzoyloxy)methylceph-3-em-4-carboxylic acid;
[6R,7R]-7-[(R)-2-azido-2-phenylacetamido]-3-(3,4-dihydroxybenzoyloxy)methylceph-3-em-4-carboxylic acid;
[6R,7R]-7-[Z-2-(2-Aminothiazol-4-yl)-2-(2-carboxypropan-2-yloxyimino)acetamido]-3-(3,4-dihydroxybenzoyloxy)methylceph-3-em-4-carboxylic acid;
[6R,7R]-7-[2-(2-amino-4-thiazolyl)-2-([Z]-methoxyimino)acetamido]-3-(3,4-dihydroxycinnamoyloxy)methylceph-3-em-4-carboxylic acid;
(6R,7R)-3-[(3,4-Dihydroxyphenyl)carbamoyloxy]methyl-7-[2-(2-furyl)-2-(Z)-methoxyiminoacetamido]ceph-3-em-4-carboxylic acid;
(6R,7R)-7-[3-(2-Chloro-6-fluorophenyl)-5-methylisoxazol-4-yl]carbonylamino-3-(3,4-dihydroxybenzoyloxy)methylceph-3-em-4-carboxylic acid;
(6R,7R)-3-(3,4-Dihydroxybenzoyloxy)methyl-7-(2-(Z)-methoxyimino-2-phenylacetamido)ceph-3-em-4-carboxylic acid;
(6R,7R)-3-(3,4-Dihydroxybenzoyloxy)methyl-7-[2-(E)-methoxyimino-2-(2-thienyl)acetamido]ceph-3-em-4-carboxylic acid;
(6R,7R)-7-[(Z)-2-[2-(2-Chloroacetamido)thiazol-4-yl]-2-methoxyiminoacetamido]-3-[(E)-3-(5-hydroxy-4-oxo-1,4-dihydropyridin-2-yl)propenoyloxy]methylceph-3-em-4-carboxylic acid;
(6R,7R)-7-[(Z)-2-(2-Aminothiazol-4-yl)-2-methoxyiminoacetamido]-3-[(E)-3-(5-hydroxy-4-oxo-1,4-dihydropyridin-2-yl)propenoyloxymethyl]ceph-3-em-4-carboxylic acid; and
(6R,7R)-7-[(Z)-2-[2-(2-Chloroacetamido)thiazol-4-yl]-2-methoxyiminoacetamido]-3-(5-hydroxy-4-oxo-1,4-dihydro pyridin-2-yl)carbonyloxymethylceph-3-em-4-carboxylic acid.

Since the β-lactam antibiotic compounds of the present invention are intended for use in pharmaceutical compositions it will readily be understood that they are each provided in substantially pure form, for example at least 50% pure, more suitably at least 75% pure and preferably at least 95% pure (% are on a wt/wt basis). Impure preparations of the compounds may be used for preparing the more pure forms used in the pharmaceutical compositions. Although the purity of intermediate compounds of the present invention is less critical it will readily be understood that the substantially pure form is preferred as for the β-lactam antibiotic compounds. Preferably, whenever possible, the compounds of the present invention are obtained in crystalline form.

When some of the compounds of this invention are allowed to crystallise, or are recrystallised, from organic solvents, solvent of crystallisation may be present in the crystalline product. This invention includes within its scope such solvates. Similarly, some of the compounds of this invention may be crystallised or recrystallised from solvents containing water. In such cases water of hydration may be formed. This invention includes within its scope stoichiometric hydrates as well as compounds containing variable amounts of water that may be produced by processes such as lyophilisation.

The antibiotic compounds according to the invention may be formulated for administration in any convenient way for use in human or veterinary medicine, according to techniques and procedures per se known in the art with reference to other antibiotics, and the invention therefore includes within its scope a pharmaceutical composition comprising an antibiotic compound according to the present invention such as, for example a pharmaceutically acceptable compound of formula (Ia) or a pharmaceutically acceptable salt or pharmaceutically acceptable in vivo hydrolysable ester thereof together with a pharmaceutically acceptable carrier or excipient.

The compositions may be formulated for administration by any suitable route, such as oral or parenteral, or by topical application. The compositions may be in the form of tablets, capsules, powders, granules, lozenges, creams or liquid preparations, such as oral or sterile parenteral solutions or suspensions.

Tablets and capsules for oral administration may be in unit dose presentation form, and may contain conventional excipients such as binding agents, for example syrup, acacia, gelatin, sorbitol, tragacanth, or polyvinylpyrollidone; fillers, for example lactose, sugar, maize-starch, calcium phosphate, sorbitol or glycine, tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, methyl cellulose, glucose syrup, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and if desired conventional flavouring or colouring agents.

Suppositories will contain conventional suppository base, eg cocoa-butter or other glyceride.

For parenteral administration, fluid unit dosage forms are prepared utilising the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing.

Advantageously, agents such as local anaesthetic, preservative and buffering agents can be dissolved in the vehicle. To enhance the stability, the composition can be frozen after filling into the vial and the water removed under vacuum. The dry lyophilised powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilisation cannot be accomplished by filtration. The compound can be sterilised by exposure to ethylene oxide before suspending in the sterile vehicle. Advantageously, a surfactant or wetting agent is included in the composition to facilitate uniform distribution of the compound.

The composition may contain from 0.1% to 99.5% by weight, preferably from 10-60% by weight, of the active material, depending on the method of administration. Where the compositions comprise dosage units, each unit will preferably contain from 50-500 mg of the active ingredient. The dosage as employed for adult human treatment will preferably range from 100 mg to 12 g per day for an average adult patient (70 kg.), for instance 1500 mg per day, depending on the route and frequency of administration. Such dosages correspond to approximately 1.5 to 170 mg/kg per day. Suitably the dosage is from 1 to 6g. per day.

The daily dosage is suitably given by administering a compound of the invention several times in a 24-hour period. Typically, 250 mg. is administered 4 times a day although, in practice, the dosage and frequency of administration which will be most suitable for an individual patient will vary with the age, weight and response of the patients, and there will be occasions when the physician will choose a higher or lower dosage and a different frequency of administration. Such dosage regimens are within the scope of this invention.

No toxicological effects are indicated when a pharmaceutically acceptable compound of the invention of formula (Ia) or a salt or in vivo hydrolysable ester thereof is administered in the above mentioned dosage range.

The antibiotic compounds according to the present invention may be the sole therapeutic agent in the compositions of the invention or a combination with other antibiotics and/or β-lactamase inhibitor may be employed.

Advantageously the compositions also comprise a compound of formula (VII) or a pharmaceutically acceptable salt or ester thereof:

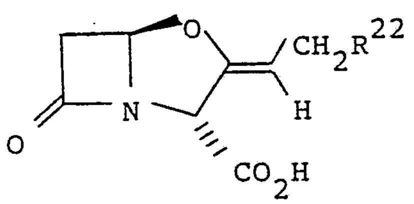

(VII)

wherein $R^{22}$ is hydroxyl; substituted hydroxyl; thiol; a group of formula $SO_2R^{23}$ wherein $R^{23}$ is $C_{1-6}$ alkyl; substituted thiol; amino; mono- or di-hydrocarbyl substituted amino; mono- or di-acylamino; an optionally substituted triazolyl group; or an optionally substituted tetrazolyl group as described in EP 0 053 893.

A further advantageous composition comprises a pharmaceutically acceptable antibiotic compound of the formula (Ia) or a salt or in vivo hydrolysable ester thereof and a pharmaceutically acceptable carrier or excipient together with a β-lactamase inhibitor of formula (VIII) or a pharmaceutically acceptable salt or in vivo

hydrolysable ester thereof:

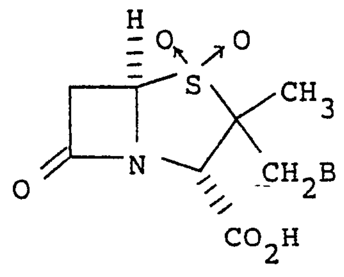

(VIII)

wherein B is hydrogen, halogen or a group of formula:

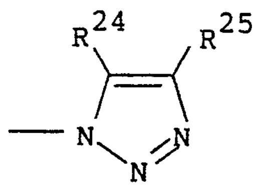

in which $R^{24}$ and $R^{25}$ are the same or different and each is hydrogen, $C_{1-6}$ alkoxycarbonyl, or carboxy or a pharmaceutically acceptable salt thereof.

Further suitable β-lactamase inhibitors include 6-alkylidene penems as described in European Patent Application No. 81301683.9 (Publication Number 0 041 768), and European Patent Application No. 85100521.5 (Publication Number 0 154 132) corresponding to laid open published Danish Patent Application No. 324/85.

Further suitable β-lactamase inhibitors include 6β-bromopenicillanic acid and salts and in vivo hydrolysable esters thereof and 6β-iodopenicillanic acid and salts and in vivo hydrolysable esters thereof.

Such compositions of this invention comprising a β-lactamase inhibitor are formulated in conventional manner.

The present invention also includes a method of treating bacterial infections in humans and animals which comprises the administration of a therapeutically effective amount of an antibiotic compound of this invention of the formula (Ia) or a salt or in vivo hydrolysable ester thereof.

The pharmaceutically acceptable antibiotic compounds of the present invention of formula (Ia) or salts or in vivo hydrolysable esters thereof are active against a broad range of Gram positive and Gram negative bacteria, and may be used to treat a wide range of bacterial infections including those in immunocompromised patients.

Amongst many other uses, the pharmaceutically acceptable compounds of the invention of formula (Ia) or salts or in vivo hydrolysable esters thereof are of value in the treatment of respiratory tract and urinary tract infections in humans and may also be used to treat mastitis in cattle. A particular advantage of the antibacterially active compounds of this invention is their stability to β-lactamase enzymes and they are therefore effective against β-lactamase producing organisms.

The present invention further provides a process for the preparation of a compound of formula (I) which process comprises treating a compound of formula (IX) or a salt thereof:

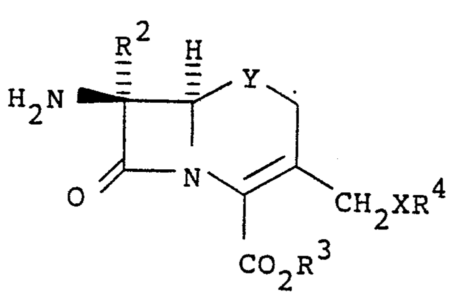

(IX)

wherein $R^2$, $R^3$, Y, X, and $R^4$ are as hereinbefore defined, wherein any reactive groups may be protected, and wherein the amino group is optionally substituted with a group which permits acylation to take place; with an N-acylating derivative of an acid of formula (X):

$R^1OH$ (X)

wherein $R^1$ is as defined with respect to formula (I) and wherein any reactive groups may be protected; and thereafter, if necessary or desired, carrying out one or more of the following steps:

i) removing any protecting groups;

ii) converting the group $R^3$ into a different group $R^3$;

iii) converting the group Y into a different group Y;

iv) converting the product into a salt.

Acids of formula (X) may be prepared by methods known in the art, or methods analogous to such processes. Suitable processes include those described, for example, in UK Patent GB 2 107 307 B, UK Patent Specification No. 1,536,281, and U.K. Patent Specification No. 1,508,064.

Suitable compounds of the formula (IX) include salts and esters, for example in which $R^3$ is diphenylmethyl or trimethylsilyl.

Suitable groups which permit acylation to take place and which are optionally present on the amino group of the starting material of the formula (IX) include N-silyl, N-stannyl and N-phosphorus groups, for example trialkylsilyl groups such as trimethylsilyl, trialkyltin groups such as tri-n-butyltin, groups of formula $-P.R^{26}R^{27}$ wherein $R^{26}$ is an alkyl, haloalkyl, aryl, aralkyl, alkoxy, haloalkyl, aryl, aralkyl, alkoxy, haloalkoxy, aryloxy, aralkyloxy or dialkylamino group, $R^{27}$ is the same as $R^{26}$ or is halogen or $R^{26}$ and $R^{27}$ together form a ring; suitable such phosphorus groups being $-P(OC_2H_5)_2$, $-P(C_2H_5)_2$,

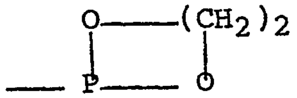 and 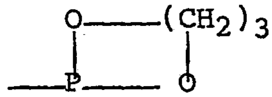

A group which may optionally be introduced onto the amino group in the compound of formula (IX) is trimethylsilyl.

Advantageously the silylation reaction may be carried out in situ, prior to the acylation reaction, with a silylating agent that does not require concomitant addition of base. Suitable silylating agents include, for example, N-(trimethylsilyl)-acetamide, N,O-bis (trimethylsilyl)acetamide, N,O-bis(trimethylsilyl) trifluoroacetamide, N-methyl-N-trimethylsilylacetamide, N-methyl-N-trimethylsilyl- trifluoroacetamide, N,N'-bis(trimethylsilyl)urea, and N,O-bis(trimethylsilyl)carbamate. A preferred silylating agent is N,O-bis(trimethylsilyl) acetamide. The silylation reaction may suitably be carried out in an inert, anhydrous organic solvent such as dichloromethane at room temperature or at an elevated temperature, for example 30 - 60°C, preferably 40 - 50°C.

The above process may optionally be carried out in the presence of a small quantity, for example 0.1 equivalents, of a silyl halide, for example a tri($C_{1-6}$)alkylsilyl halide, especially trimethylsilyl chloride.

A reactive N-acylating derivative of the acid (X) is employed in the above process. The choice of reactive derivative will of course be influenced by the chemical nature of the substituents of the acid.

Suitable N-acylating derivatives include an acid halide, preferably the acid chloride or bromide. Acylation with an acid halide may be effected in the presence of an acid binding agent for example, tertiary amine (such as pyridine or dimethylaniline), molecular sieves, an inorganic base (such as calcium carbonate or sodium bicarbonate) or an oxirane, which binds hydrogen halide liberated in the acylation reaction. The oxirane is preferably a ($C_{1-6}$)- 1,2-alkylene oxide -such as ethylene oxide or propylene oxide. The acylation reaction using an acid halide may be carried out at a temperature in the range -50°C to +50°C, preferably -20°C to +20°C, in aqueous or non-aqueous media such as water, acetone, tetrahydrofuran, ethyl acetate, dimethylacetamide, dimethylformamide, acetonitrile, dichloromethane, 1,2-dichloroethane, or mixtures thereof. Alternatively, the reaction may be carried out in an unstable emulsion of water-immiscible solvent, especially an aliphatic ester or ketone, such as methyl isobutyl ketone or butyl acetate.

The acid halide may be prepared by reacting the acid (X) or a salt or a reactive derivative thereof with a halogenating (eg chlorinating or brominating) agent such as phosphorus pentachloride, thionyl chloride, oxalyl chloride or phosgene.

Alternatively, the N-acylating derivative of the acid (X) may be a symmetrical or mixed anhydride. Suitable mixed anhydrides are alkoxyformic anhydrides, or anhydrides with, for example, carbonic acid monoesters, trimethyl acetic acid, thioacetic acid, diphenylacetic acid, benzoic acid, phosphorus acids (such as phosphoric, phosphorous, and phosphinic acids) or aromatic or aliphatic sulphonic acids (such as p-toluenesulphonic acid or methanesulphonic acid). When a symmetrical anhydride is employed, the reaction may be carried out in the presence of 2,6-lutidine as catalyst.

Alternative N-acylating derivatives of acid (X) are the acid azide, or activated esters such as esters with 2-mercaptopyridine, cyanomethanol, p-nitrophenol, 2,4-dinitrophenol, thiophenol, halophenols, including pentachlorophenol, monomethoxyphenol, N-hydroxy succinimide, N-hydroxybenzotriazole, or 8-hydroxyquinoline; or amides such as N-acylsaccharins, N-acylthiazolidin-2-thione or N-acylphthalimides; or an alkylidene iminoester prepared by reaction of the acid (X) with an oxime.

Other reactive N-acylating derivatives of the acid (X) include the reactive intermediates formed by reaction in situ with a condensing agent such as a carbodiimide, for example, N,N'-diethyl-, dipropyl- or diisopropylcarbo-

diimide, N,N′-di-cyclohexylcarbodiimide, or N-ethyl-N′-[3-(dimethylamino)propyl]carbodiimide; a suitable carbonyl compound, for example, N,N′-carbonyldiimidazole or N,N′-carbonylditriazole; an isoxazolinium salt, for example, N-ethyl-5-phenylisoxazolinium-3-sulphonate or N-t-butyl-5-methylisoxazolinium perchlorate; or an N-alkoxycarbonyl 2-alkoxy-1,2-dihydroquinoline, such as N-ethoxycarbonyl 2-ethoxy-1,2-dihydroquinoline. Other condensing agents include Lewis acids (for example $BBr_3$ - $C_6H_6$); or a phosphoric acid condensing agent such as diethylphosphorylcyanide. The condensation reaction is preferably carried out in an organic reaction medium, for example, methylene chloride, dimethylformamide, acetonitrile, alcohol, benzene, dioxan or tetrahydrofuran.

A further method of forming the N-acylating derivative of the acid of formula (X) is to treat the acid of formula (X) with a solution or suspension preformed by addition of a carbonyl halide, preferably oxalyl chloride, or a phosphoryl halide such as phosphorus oxychloride, to a halogenated hydrocarbon solvent, preferably dichloromethane, containing a lower acyl tertiary amide, preferably N,N-dimethylformamide. The N-acylating derivative of the acid of formula (X) so derived may then be caused to react with a compound of formula (IX). The acylation reaction may conveniently be carried out at -40° to +30°C, if desired in the presence of an acid binding agent such as pyridine. A catalyst such as 4-dimethylaminopyridine may optionally also be added. A preferred solvent for the above acylation reaction is dichloromethane.

When the group Y is S, SO, or $SO_2$, the group Y may be converted into a different group Y by methods of oxidation or reduction well known in the art of cephalosporin and penicillin synthesis, as described, for example, in European Patent Application Publication No. 0 114 752. For example, sulphoxides (in which Y is SO) may be prepared from the corresponding sulphide (in which Y is S) by oxidation with a suitable oxidising agent, for example an organic peracid such as m-chloroperbenzoic acid.

In the process described hereinabove, and in the processes described hereinbelow, it may be necessary to remove protecting groups. Deprotection may be carried out by any convenient method known in the art that does not cause unwanted side reactions to occur to any appreciable extent. Methods that are particularly suitable for converting an acetoxy group in $R^5$ into a hydroxy group include treatment with aqueous sodium sulphite solution or aqueous sodium hydrogen carbonate solution, or treatment with an esterase, especially citrus acetylesterase.

A preferred process within the invention for the preparation of a compound of formula (I) comprises treating a compound of formula (XI):

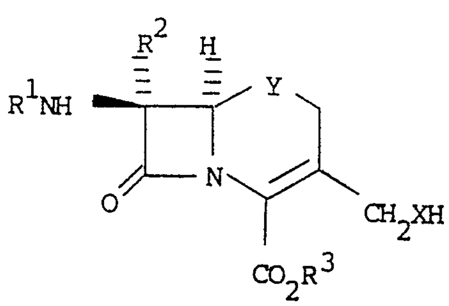

(XI)

wherein $R^1$, $R^2$, $R^3$, Y and X are as hereinbefore defined and any reactive groups may be protected; with either

a) (when Z is not NH) an acylating derivative of an acid of formula (XII):

$R^4$OH (XII)

wherein $R^4$ is as defined in respect of formula (I) and any reactive groups may be protected; or

b) (when Z is NH) an isocyanate of the formula (XIIA):

$R^5$NCO (XIIA)

wherein $R^5$ is as defined in respect of formula (I) and any reactive groups may be protected;

and after step a) or step b), if necessary, carrying out one or more of the following steps: (i) converting the group $R^3$ into a different group $R^3$;

(ii) removing any protecting groups;

(iii) converting the group Y into a different group Y;

(iv) converting the product into a salt.

A particular group $R^3$ in compounds of formula (XI) is diphenylmethyl.

Suitable acylating derivatives of the acid of formula (XII) include an acid halide, preferably the acid chloride or bromide. The acid halides may be prepared by reacting the acid (XII) or a salt or suitable derivative thereof with a halogenating agent as described hereinabove with respect to the acid of formula (X). Acylation with an acid halide may be effected as hereinbefore described.

When X is NH in compounds of formula (XI), a suitable group which permits acylation to take place may optionally be present on the amino group of the 3-aminomethylcephem. Suitable groups include those listed hereinabove as suitable for permitting acylation to take place on the 7β-amino group of the intermediate of formula (IX).

Acylation of the compounds of formula (XI) wherein X is NH is suitably carried out under conventional Schotten-Baumann conditions, i.e. shaking with dilute alkali, for example sodium hydrogen carbonate solution, optionally in the presence of an organic solvent, for example tetrahydrofuran.

Acylation of the compound of formula (XI) wherein X is oxygen is normally carried out at ambient temperature in a chlorinated hydrocarbon solvent, for example dichloromethane.

The compounds of formula (IX) and salts thereof which are, inter alia intermediates for the compounds of formula (I) as hereinbefore defined may be prepared by reacting a compound of formula (XIII):

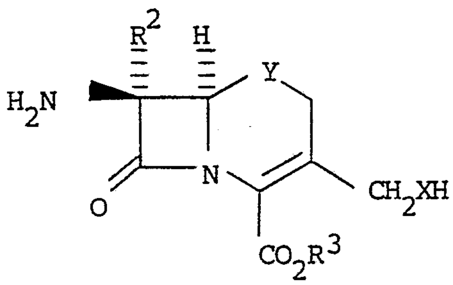

(XIII)

wherein $R^2$, $R^3$, Y and X are as hereinbefore defined and any reactive groups may be protected with an acylating derivative of an acid of formula (XII) or an isocyanate of formula (XIIA), and thereafter if necessary carrying out one or more of the following steps:

(i) converting the group $R^3$ into a different group $R^3$;
(ii) removing any protecting groups;
(iii) converting the group Y into a different group Y;
(iv) converting the product into a salt.

Alternatively, the compound of formula (IX) wherein X is O or S, or a salt thereof, may be prepared by reacting a compound of the formula (XIV):

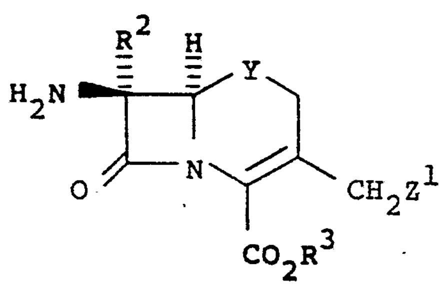

(XIV)

wherein $R^2$, $R^3$ and Y are as defined with respect to formula (I), $Z^1$ is a leaving group, and any reactive groups may be protected, with an acid of the formula (XV) or a salt thereof:

$R^4XH$ (XV)

wherein X is O or S and $R^4$ is as hereinabove defined, and thereafter, if necessary, carrying out one or more of the following steps:

(i) removing any protecting groups;
(ii) converting the group $R^3$ into a different group $R^3$;
(iii) converting the group Y into a different group Y;
(iv) converting the product into a salt.

Suitable leaving groups for $Z^1$ include halogen. Conveniently the salt of the acid (XV) is an alkali metal salt such as the sodium salt.

The compounds of formulae (XI) and (XIII) wherein X is oxygen may be produced by treatment of the corresponding 3-acetoxymethyl cephalosporin compound with a suitable enzyme-containing preparation such as wheat bran or a purified enzyme such as citrus acetyl esterase.

Compounds of formulae (XIV) and (XV) may be prepared by methods known in the art.

The compound of formula (XI) wherein X is NH may be produced by reduction of a 3-azidomethylcephem of formula (XVI):

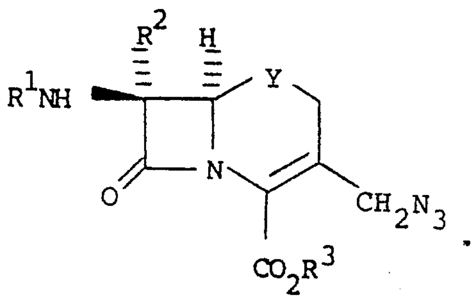

(XVI)

wherein $R^1$, $R^2$, Y and $R^3$ are as hereinbefore defined, with a reducing agent which does not cause appreciable side-reactions, or reduction of a group other than the azido group.

Suitable reducing conditions include hydrogenation in the presence of a catalyst, for example palladium on charcoal, or the Lindlar catalyst.

In a similar manner, the compound of formula (XIII) wherein X is NH may be produced by reduction of a 3-azidomethylcephem of formula (XVII):

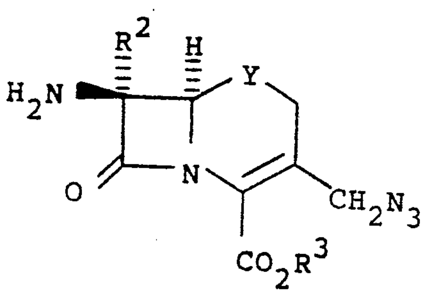

(XVII)

wherein $R^2$, Y and $R^3$ are as hereinabove defined.

The required 3-azidomethylcephem of formula (XVI) or (XVII) may be prepared by treatment of the corresponding 3-acetoxymethylcephem with a source of azide ions such as an alkali metal azide, for example sodium azide. The reaction may suitably be carried out in aqueous solution.

The present invention further provides a process for the preparation of a compound of formula (I) wherein $R^2$ is methoxy or formamido by reacting a compound of formula (XVIII):

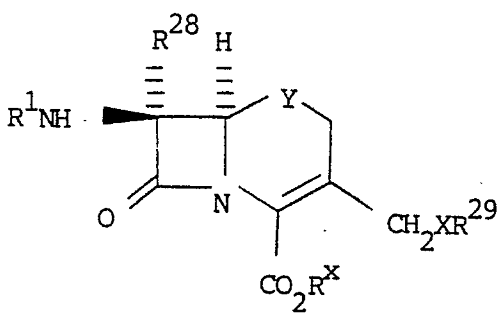

(XVIII)

wherein $R^1$, Y and X are as hereinabove defined, $R^x$ is a readily removable carboxy protecting group, $R^{29}$ is hydrogen or a group $R^4$ as hereinabove defined, $R^{28}$ is a suitable intermediate group known for the production of a methoxy or formamido group, and wherein any reactive groups may be protected; with a reagent known to convert the intermediate group $R^{28}$ to methoxy or formamido; and thereafter if necessary carrying out one or more of the following steps:

(i) removing any protecting groups;
(ii) converting the group $R^x$ into a group $R^3$;
(iii) converting the group Y into a different group Y;
(iv) converting the product into a salt;
(v) converting the group $R^{29}$ into a group $R^4$.

Suitable groups for $R^x$ include ester groups such as those listed hereinabove as suitable for the group $R^3$.

When the group $R^2$ in the compound of formula (I) is methoxy, the intermediate group $R^{28}$ is suitably a group $-SR^{30}$ wherein $R^{30}$ is $C_{1-6}$ alkyl, aryl or benzyl.

Preferably $R^{30}$ is 4-methylphenyl.

Processes suitable for converting the group $-SR^{30}$ into methoxy include:

(a) reaction with chlorine or bromine at -25° to -80° C and subsequently decomposing the resultant halo sulphonium halide with methanol and a base; or

(b) reaction with methanol in the presence of a metal ion such as tellurium (III), lead (IV), silver, copper (II), bismuth (V), mercury, lead, cadmium or thallium salt.

When the group $R^2$ in the required compound of formula (I) is formamido the group $R^{28}$ in the compound of formula (XVIII) is suitably a group $-SR^{30}$ or $SOR^{30}$ wherein $R^{30}$ is as hereinabove defined, or a group $-NH_2$.

Processes suitable for converting the group $SR^{30}$ into formamido involve use of a nucleophilic derivative of formamide as described in European Patent Application Publication No. 0 115 405.

The group $SOR^{30}$ may also be used as an intermediate group $R^{28}$ and converted to a formamido group by direct reaction with a nucleophilic derivative of formamide.

When the group $R^{28}$ is $-NH_2$, the amino group may be formylated to give the required -NHCHO group.

Suitable formylating agents include the reagent 4-formyl-2-methyl-1,3,4-thiadiazolin-5-thione (see H. Yazawa and S. Goto, Tetrahedron Letters, 1985, 26, 3703-3706), or mixed anhydrides such as formic acetic anhydride. The reaction may suitably be carried out in a temperature in the range -50° C to 30° C in aprotic solvent such as, for example, dichloromethane, chloroform, dimethylformamide, tetrahydrofuran, hexamethylphosphoramide, or dimethylsulphoxide, in the presence of a tertiary base. A preferred tertiary base employed in the reaction is a base of the pyridine type, such as pyridine, lutidine or picoline.

A process suitable for preparing compounds of formula (XVIII) wherein $R^{28}$ is $-SR^{30}$ is analogous to that described in Tetrahedron Letters 1973, 273.

A process suitable for preparing compounds of formula (XVIII) wherein $R^{28}$ is $-NH_2$ is analogous to that disclosed in European Patent Application Publication No. 0 071 395.

In a preferred aspect of the above process the group $R^{29}$ in the compound of formula (XVIII) is hydrogen and the group $R^4$ is introduced in step (v), for example by acylation with an acylating derivative of an acid of formula (XII) under the conditions described hereinabove for the conversion of a compound of formula (XI) into a compound of formula (I) or (where appropriate) reaction with an isocyanate of formula (XIIA).

In a particularly preferred process for the preparation of compounds of formula (I) wherein $R^2$ methoxy or formamido and X is oxygen, the group $XR^{29}$ in the compound of formula (XVIII) is hydroxy protected as acetoxy. Deprotection of the hydroxy group is suitably carried out subsequent to the introduction of the methoxy or formamido group at C-7 by enzymatic cleavage, preferably with citrus acetyl esterase.

Compounds of formula (I) wherein the group $R^1$ is a group of the formula:

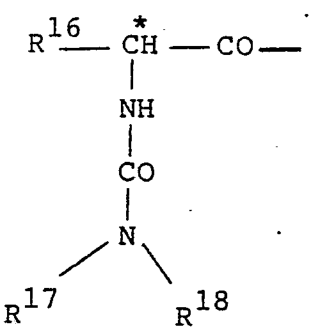

wherein $R^{16}$, $R^{17}$, $R^{18}$, and * are as hereinbefore defined may also be prepared by treating a compound of formula (XIX):

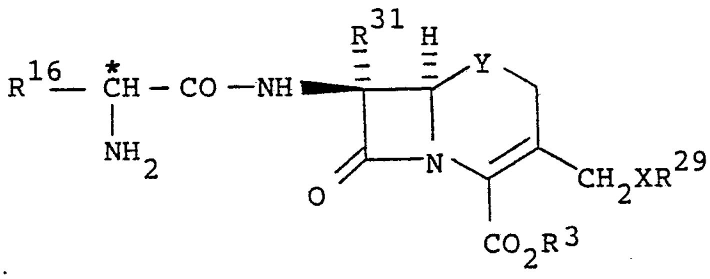

(XIX)

wherein $R^{16}$, *, $R^3$, Y, $R^{29}$, and X are as hereinabove defined and $R^{31}$ is hydrogen, methoxy, formamido, or a group $R^{28}$ as defined for formula (XVIII), and wherein any reactive groups may be protected; with an acylating derivative of an acid of formula (XX):

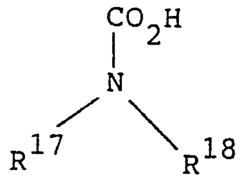

(XX)

wherein $R^{17}$ and $R^{18}$ as hereinabove defined and wherein any reactive groups may be protected; and thereafter if necessary carrying out one or more of the following steps:

(i) converting the group $R^{31}$ into methoxy or formamido;
(ii) removing any protecting groups;
(iii) converting the group $R^{29}$ into a group $R^4$;
(iv) converting the group $R^3$ into a different group $R^3$;
(v) converting the group Y into a different group Y;
(vi) converting the product into a salt.

Compounds within formula (XIX) may be prepared by reacting a compound of formula (IX) with an N-acylating derivative of an acid of formula (XXI):

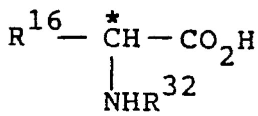

(XXI)

wherein $R^{16}$ and * are as hereinbefore defined and $R^{32}$ is an amino protecting group; and thereafter removing the amino protecting group $R^{32}$.

Compounds within formula (XIX) may also be prepared by reacting a compound of formula (IX) with an N-acylating derivative of an α-azido acid of formula (XXII):

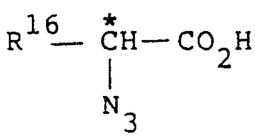

(XXII)

wherein $R^{16}$ and * are as hereinbefore defined; followed by reduction of the azido group to an amino group by conventional methods such as catalytic hydrogenation.

From the foregoing it may be seen that compounds of formula (IX) and protected derivatives thereof are important intermediates and accordingly such compounds form another aspect of the present invention.

Furthermore, compounds of formula (XIII) as hereinabove defined wherein X is NH and $R^2$ is formamido; compounds of formulae (XVI) and (XVII) as hereinabove defined in which $R^2$ is formamido; and compounds of formula (XXIII):

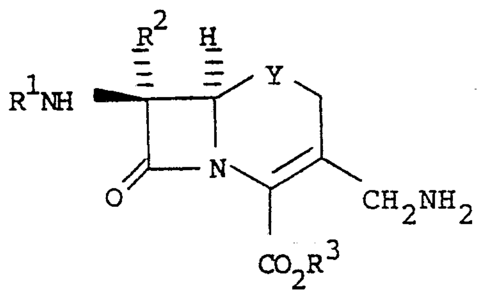

(XXIII)

in which $R^2$ is formamido and $R^1$, Y and $R^3$ are as defined with respect to formula (I) are all novel and valuable intermediates which form yet another aspect of the present invention.

In addition, compounds of formula (XVI) have potent antibacterial activity in their own right.

A preferred sub-class of such antibacterial agents has the formula (XXIV) or a pharmaceutically acceptable salt or pharmaceutically acceptable in vivo hydrolysable ester thereof:

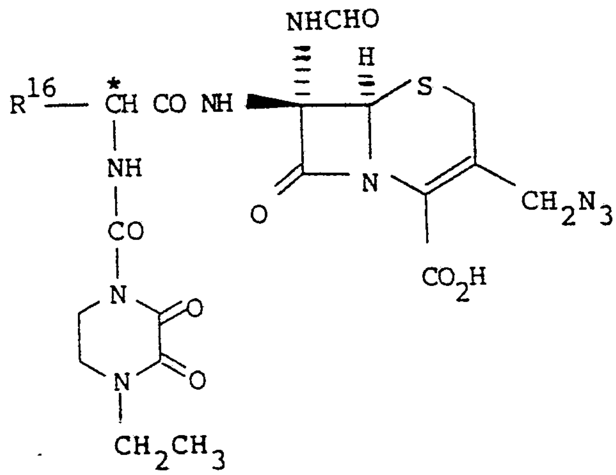

(XXIV)

in which the group $R^{16}$ is as defined with respect to formula (IV) hereinabove.

In compounds of formula (XXIV), $R^{16}$ is preferably phenyl or substituted phenyl, most preferably a 3,4-dihydroxyphenyl group.

Specific compounds within formula (XXIV) include the following and pharmaceutically acceptable salts and pharmaceutically acceptable in vivo hydrolysable esteres thereof:

(6R, 7R)-3-Azidomethyl-7-[(R)-2-(4-ethyl-2,3-dioxopiperazin-1-ylcarbonylamino)-2-phenylacetamido]-7-formamidoceph-3-em-4-carboxylic acid;

(6R,7R)-3-Azidomethyl-7-[(R)-2-(4-ethyl-2,3-dioxopiperazin-1-ylcarbonylamino)-2-(3,4-dihydroxyphenyl)acetamido]-7-formamidoceph-3-em-4-carboxylic acid;

Specific compounds within formula (XVII) include the following:

Sodium (6R, 7S)-7-Amino-3-azidomethyl-7-formamidoceph-3-em-4-carboxylate; and

Benzhydryl (6R, 7S)-7-Amino-3-azidomethyl-7-formamidoceph-3-em-4-carboxylate.

Specific compounds within formula (XXIII) include the following:

Sodium (6R, 7R)-3-Aminomethyl-7-[(R)-2-(4-ethyl-2,3-dioxopiperazin-1-ylcarbonylamino)-2-phenylacetamido]-7-formamidoceph-3-em-4-carboxylate.

Suitable amino protecting groups in compounds in which the amino group may be protected, including compounds of formulae (IX) and (XXI), are those well known in the art which may be removed under conventional conditions without disruption of the remainder of the molecule.

Examples of amino-protecting groups include benzyl optionally substituted in the phenyl ring by one or two substituents selected from $C_{1-4}$ alkyl, $C_{1-4}$ alkoxy, trifluoromethyl, halogen or nitro; $C_{1-4}$ alkylcarbonyl optionally substituted by halogen; $C_{1-4}$ alkoxycarbonyl, for example tert-butoxycarbonyl; benzyloxycarbonyl optionally substituted as for benzyl above; allyloxycarbonyl; trityl or trichloroethoxy- carbonyl.

A particular amino-protecting group is 2-chloroacetyl.

The compounds of formula (Ia) may be prepared by similar processes, to those described hereinabove as suitable for the preparation of a compound of the formula (I), except that each process for the preparation of the compound of formula (Ia) further comprises, if necessary, the step of converting the product into a

pharmaceutically acceptable salt or pharmaceutically acceptable in vivo hydrolysable ester.

The antibiotic compounds of the present invention are active against a wide range of Gram-negative and Gram-positive organisms including E.coli such as, for example ESS, JT4, JT425 and NCTC 10418; Pseudomonas Spp. such as Ps.aeruginosa for example 10662 and Dalgleish; Serratia marcescens US32; Klebsiella aerogenes A; Enterobacter cloacae N1; P.mirabilis such as, for example C977 and 889; P.morganii; P.rettgeri; B.subtilis; Staph.aureus such as, for example Oxford and Russell; N.catarrhalis 1502; Strep.faecalis I; β-Haemolytic Strep.CN10.

The following Examples illustrate the preparation and biological activity of the compounds of the present invention.

The following abbreviations are used:
DMF refers to dimethylformamide,
THF refers to tetrahydrofuran,
TFA refers to trifluoroacetic acid,
EtOAc refers to ethyl acetate.

PREPARATION 1

Diphenylmethyl (6R,7R)-3-(3,4-Diacetoxybenzoyloxy)methyl-7-[(R)-2-(4-ethyl-2,3-dioxopiperazin-1-yl)carbonylamino-2-phenylacetamido]-7-formamidoceph-3-em-4-carboxylate

Sodium (6R,7R)-3-acetoxymethyl-7-[(R)-2-(4-ethyl-2,3-dioxopiperazin-1-yl)carbonylamino-2-phenylacetamido]-7-formamidoceph-3-em-4-carboxylate (200mg, 0.31 mmol) (European Patent Application Publication No. 0 071 395) was dissolved in 0.05M aqueous $KH_2PO_4$ solution (10ml) and the pH adjusted to 7 with 0.05M aqueous NaOH solution. Citrus acetylesterase preparation (ex. Sigma) (1.6ml, equivalent to approx. 6.4mg of protein) was added and the mixture allowed to stand. The pH was maintained at 7 by additions of aqueous $NaHCO_3$ solution for 30 minutes.

After 2 hours the reaction mixture was layered with EtOAc (30ml) and stirred vigorously while adjusting the pH of the aqueous layer to 2.1 with 2.5M aqueous HCl solution. The organic phase was separated and the aqueous phase extracted a further five times with EtOAc (5 × 30ml). The combined organic extracts were dried over $MgSO_4$ and concentrated to 15ml under reduced pressure to provide a solution of (6R,7R)-7-[(R)-2-(4-ethyl-2,3-dioxopiperazin-1-yl)carbonylamino-2-phenylacetamido]-7-formamido-3-hydroxymethylceph-3-em-4-carboxylic acid. The above solution was treated with diphenyldiazomethane (61mg, 0.31mmol) and allowed to stand for 2 hours. After concentration under reduced pressure to a volume of 5ml, dry $CH_2Cl_2$ (25ml)was added. The solution was concentrated, diluted with dry $CH_2Cl_2$ (25ml), concentrated, diluted with dry $CH_2Cl_2$ (25ml), concentrated and finally diluted with dry $CH_2Cl_2$ to provide a solution of diphenylmethyl (6R,7R)-7-[(R)-2-(4-ethyl-2,3-dioxopiperazin-1-yl) carbonylamino-2-phenylacetamido]-7-formamido-3-hydroxymethylceph-3-em-4-carboxylate in $CH_2Cl_2$ (l0ml). The above solution was treated with pyridine (66μl, 64mg, 0.81mmol) and 3,4-diacetoxybenzoyl chloride (0.81mmol, prepared as described below in Preparation 4 (a)) in $CH_2Cl_2$(5ml) and the mixture allowed to stand overnight. After concentration under reduced pressure EtOAc (40ml) was added. The resulting solution was washed with saturated aqueous $NaHCO_3$ solution (3 × 30ml), 0.1M aqueous HCl solution (2 × 30ml) and brine (30ml) then dried over $MgSO_4$. The solvent was removed under reduced pressure and the residue chromatographed on silica gel. Elution with mixtures of ethyl acetate/hexane and methanol/ethyl acetate provided the title compound (24mg) containing some of the corresponding monoacetoxymonohydroxybenzoyl derivative; $\nu_{max}$($CH_2Cl_2$) 3360, 3270, 1790, 1780, 1720 and 1690cm$^{-1}$; $\delta_H$($CD_3COCD_3$) (major rotamer) 10.03 (1H, d, J 6.9Hz), 8.80 (1H, s), 8.53 (1H, s), 8.30 (1H, s), 7.92-7.82 (2H, m), 7.63-7.22 (16H, m), 6.94 (1H, s), 5.73 (1H, d, J 6.9Hz) 5.33 (1H, s), 5.27 and 5.15 (each 1H, d, J 13.8Hz), 4.04 (2H, m), 3.69 (2H, m), 3.50 (2H, q, J 7.1Hz), 3.39 and 3.07 (each 1H, d, J 16.8Hz), 2.32 (3H, s), 2.31 (3H, s), 1.16 (3H, t, J 7.1Hz). [F.A.B. (+ve ion, 3-nitrobenzylalcohol) MNa+ 983].

PREPARATION 2

Diphenylmethyl (6R,7R)-3-(3,4-Diacetoxycinnamoyloxy)methyl-7-[(R)-2-(4-ethyl-2,3-dioxopiperazin-1-yl)carbonylamino-2-phenylacetamido]-7-formamidoceph-3-em-4-carboxylate

By an exactly analogous procedure to that of Prepar ation 1 sodium (6R,7R)-3-acetoxymethyl-7-[(R)-2-(4ethyl-2,3-dioxopiperazin-1-yl)carbonylamino-2-phenylacetamido]-7-formamidoceph-3-em-4-carboxylate (300mg, 0.47mmol) was converted to a solution of diphenylmethyl (6R,7R)-7-[(R)-2-(4-ethyl-2,3-dioxopiperazin-1-yl)carbonylamino-2-phenylacetamido]-7-formamido-3-hydroxymethylceph-3-em-4-carboxylate in $CH_2Cl_2$ (10ml). The above solution was treated with pyridine (95μl, 93mg, 1.175mmol) and 3,4-diacetoxycinnamoyl chloride (1.175mmol, prepared from the corresponding acid by the general procedure described below in Preparation 4(a)) and the mixture allowed to stand overnight. After concentration under reduced pressure EtOAc (40ml) was added. The resulting solution was washed with saturated aqueous $NaHCO_3$ solution (3 × 40ml), 0.1M aqueous HCl solution (2 × 40ml) and brine (40ml) then dried over $MgSO_4$. The solvent was removed under reduced pressure and the residue chromatographed on silica gel. Elution with mixtures of ethyl acetate/hexane and methanol/ethyl acetate provided the title compound (68mg) as a white solid; $\nu_{max}$

($CH_2Cl_2$) 3380, 3260, 1785, 1720, 1685 and 1620cm$^{-1}$; $\delta_H$ ($CD_3COCD_3$) (major rotamer) 10.04 (1H, d, J 6.9Hz), 8.80 (1H, s), 8.53 (1H, s), 8.30 (1H, s), 7.70-7.15 (19H, m), 6.94 (1H, s), 6.55 (1H, d, J 16.OHz), 5.75 (1H, d, J 6.9Hz), 5.33 (1H, s), 5.15 and 5.00 (each 1H, d, J 13.9Hz), 4.07 (2H, m), 3.68 (2H, m), 3.50 (2H, q, J 7.1Hz), 3.34 and 3.14 (each 1H, d, J 16.9Hz), 2.33 (3H, s), 2.32 (3H, s), 1.17 (3H, t, J 7.1Hz).

PREPARATION 3

Sodium (6R,7R)-3-Acetoxymethyl-7-formamido-7-(2,2,2-trichloroethoxy)carbonylaminoceph-3-em-4-carboxylate

t-Butyl (6R,7R)-3-acetoxymethyl-7-formamido-7-(2,2,2- trichloroethoxy)carbonylaminoceph-3-em-4-carboxylate (3.0g, 5.49mmol) (European Patent Application Publication No. 0 071 395) was dissolved in trifluoroacetic acid (TFA) (18ml) and the solution allowed to stand for 40 minutes. The TFA was then removed under reduced pressure and the residue redissolved in toluene (20ml) and the mixture re-evaporated. The crude acid was suspended in water (15ml) and the pH adjusted to 7 with saturated aqueous $NaHCO_3$ solution. The resulting solution was loaded onto a column of Diaion HP20SS. Elution began with water and continued with acetone/water mixtures. The product containing fractions were identified by h.p.l.c. then combined and concentrated. The solution was freeze-dried to afford the title compound (1.89g) as a white solid; $\nu_{max}$ (KBr) 1775, 1736, 1685 and 1610cm$^{-1}$; $\delta_H$ ($D_2O$) (Major rotamer) 8.20 (1H, s), 5.36 (1H, s), 4.98 and 4.70 (each 1H, d, J 12.2Hz), 4.84 (2H, AB), 3.68 and 3.35 (each 1H, d, J 17.7Hz), 2.11 (3H, s). [F.A.B. (+ve ion, thioglycerol) MH+ 512].

PREPARATION 4(a)

3,4-Diacetoxybenzoyl Chloride

To 3,4-diacetoxybenzoic acid (238mg, 1.0mmol) in dry $CH_2Cl_2$ (15ml), containing a catalytic quantity of dry DMF, was added oxalyl chloride (175µl, 254mg, 2.0mmol). The mixture was stirred for 1$^1/_2$ hours, then evaporated under reduced pressure. The residue was taken up in dry $CH_2Cl_2$ and re-evaporated (× 2). The title compound was dried in vacuo and used without further purification.

PREPARATION 4(b)

Diphenylmethyl (6R,7R)-3-(3,4-Diacetoxybenzoyloxy)methyl-7-formamido-7-(2,2,2-trichloroethoxy)carbonylaminoceph-3-em-4-carboxylate

Sodium (6R,7R)-3-acetoxymethyl-7-formamido-7-(2,2,2-trichloroethoxy)carbonylaminoceph-3-em-4-carboxylate (200mg, 0.39mmol) was dissolved in 0.05M aqueous $KH_2PO_4$ solution (10ml) and the pH of the solution adjusted to 7 with 0.05M aqueous NaOH solution. Citrus acetylesterase preparation (ex. Sigma) (1.6ml, equivalent to approx. 6.4mg protein) was added and the mixture allowed to stand overnight. The reaction mixture was layered with ethyl acetate (30ml) and stirred vigorously while adjusting the pH of the aqueous layer to 2.1 with 5M aqueous HCl solution. The organic phase was separated and the aqueous phase extracted a further five times with EtOAc (5 × 30ml). The combined organic extracts were washed once with brine (20ml) and dried over $MgSO_4$. The volume was reduced to 20ml by evaporation under reduced pressure to provide a solution of (6R,7R)-7-formamido-3-hydroxymethyl-7-trichloroethoxycarbonylaminoceph-3-em-4-carboxylic acid.

The above solution was treated with diphenyldiazomethane (79mg, 0.41mmol) and allowed to stand for 1$^1/_2$ hours. After concentration to a volume of 5ml by evaporation under reduced pressure dry $CH_2Cl_2$ (40ml) was added. The solution was again concentrated to 5ml and diluted with dry $CH_2Cl_2$ (40ml). Concentration by evaporation under reduced pressure then provided a solution of diphenylmethyl (6R,7R)-7-formamido-3-hydroxymethyl-7-(2,2,2-trichloroethoxy)carbonlyaminoceph-3- em-4-carboxylate in $CH_2Cl_2$ (15ml).

The above solution was treated with pyridine (81µl, 79mg, 1.0mmol) and 3,4-diacetoxybenzoyl chloride (1.0mmol, prepared as described in Preparation 4 (a)) in $CH_2Cl_2$ (5ml). The mixture was stirred for 2 hours then concentrated under reduced pressure. EtOAc( 40ml) was added and the resulting solution washed with saturated aqueous $NaHCO_3$ solution (3 × 30ml), 0.1M aqueous HCl solution (2 × 30ml) and brine (30ml) then dried over $MgSO_4$. The solvent was removed under reduced pressure and the residue chromatographed on silica gel. Elution with mixtures of ethyl acetate/hexane provided the title compound (112mg) as an amorphous white solid; $\nu_{max}$ (KBr) 3320, 1780, 1725 and 1695cm$^{-1}$; $\delta_H$ ($CDCl_3$) (major rotamer) 8.23 (1H, s), 8.1-7.8 (2H, m), 7.5-7.2 (11H, m), 6.96 (1H, s), 6.55 (1H, s), 5.41 and 5.23 (each 1H, d, J 14.2Hz), 5.23 (1H, s), 4.78 (2H, AA'), 3.44 and 3.30 (each 1H, d, J 16.2Hz), 2.33 (3H, s), 2.32 (3H, s). [F.A.B. (+ve ion, 3-nitrobenzylalcohol/sodium/thioglycerol) [MNa + thioglycerol]+, 964].

PREPARATION 5

Diphenylmethyl (6R,7S)-7-Amino-3-(3,4-dihydroxybenzoyloxy)methyl-7-formamidoceph-3-em-4-carboxylate

Diphenylmethyl (6R,7R)-3-(3,4-diacetoxybenzoyloxy)methyl-7-formamido-7-(2,2,2-trichloroethoxy)carbonylaminoceph-3-em-4-carboxylate (180mg, 0.22mmol) was dissolved in THF (10ml) and 1M aqueous $KH_2PO_4$

solution (3ml) was added. Freshly activated zinc powder (500mg) was added and the pH adjusted to 4 with 2.5M aqueous HCl solution. Further additions of zinc were made to complete the reaction, with the pH maintained at 4 with 2.5M aqueous HCl solution. After 20 minutes the mixture was filtered through a Kieselguhr plug and the filtrate evaporated under reduced pressure. The residue was partitioned between EtOAc and water; the organic phase was separated and washed with saturated aqueous $NaHCO_3$ solution and brine. After drying with $MgSO_4$ and evaporation of the solvent under reduced pressure the residue was chromatographed on silica gel. Elution with mixtures of ethyl acetate/hexane followed by concentration of the product-containing eluant gave the title compound (31mg) as a white solid; $\nu_{max}$ ($CH_2Cl_2$) 3400, 3300, 1775, 1740 and 1690cm$^{-1}$; $\delta_H$($CDCl_3$) (major rotamer) 8.19 (1H, s), 7.3 (13H, m), 6.86 (1H, s), 6.67 (1H, s), 5.40 (1H, s), 5.26 (2H, m), 3.43 and 3.23 (each 1H, d, J 13.8Hz).

## EXAMPLE 1

Sodium (6R,7R)-3-(3,4-Dihydroxybenzoyloxy)methyl-7-[(R)-2-(4-ethyl-2,3-dioxopiperazin-1-yl)carbonylamino-2-phenylacetamido]-7-formamidoceph-3-em-4-carboxylate

Diphenylmethyl (6R,7R)-3-(3,4-diacetoxybenzoyloxy) methyl-7-[(R)-2-(4-ethyl-2,3-dioxopiperazin-1-yl) carbonylamino-2-phenylacetamido]-7-formamido-3-em-4-carboxylate (300mg, 0.31mmol) was dissolved in dry $CH_2Cl_2$ (24ml) and anisole (1.02ml, 1.01g, 9.36mmol) was added. The solution was cooled in an ice bath and trifluoroacetic acid (TFA) (722μl, 1.07g, 9.36mmol) was added. After 1$^1/_2$ hours the TFA and $CH_2Cl_2$ were removed by evaporation under reduced pressure. The residue was triturated with ether providing a yellow solid. The solid was suspended in water and the pH adjusted to 7 by addition of saturated aqueous $NaHCO_3$ solution. The resulting solution was treated with citrus acetylesterase preparation (ex. Sigma) (1.0ml, equivalent to approx. 4mg protein) and allowed to stand for 1 hour. Chromatography on Diaion HP20SS resin involved elution with acetone/water mixtures. The product-containing eluant was concentrated under reduced pressure then lyophilised to give the title compound as a white solid (68mg); $\nu_{max}$ (KBr) 1774, 1705, 1675 and 1609cm$^{-1}$; $\delta_H$($D_2O/CD_3COCD_3$) (major rotamer) 8.18 (1H, s), 7.56-7.36 (7H, m), 6.86 (1H, d, J 7.8Hz), 5.56 (1H, s), 5.32 (1H, s), 5.07 and 4.92 (each 1H, d, J 12.8Hz), 4.15-3.95 (2H, m), 3.8-3.65 (2H, m), 3.54 (2H, q, J 7.2Hz), 3.43 and 3.02 (each 1H, d, J 17.3Hz), 1.22 (3H, t, J 7.2Hz). [F.A.B (+ve ion, thioglycerol) MH+ 733].

## EXAMPLE 2

Sodium (6R,7R)-3-(3,4-Dihydroxycinnamoyloxy)methyl-7-[(R)-2-(4-ethyl-2,3-dioxopiperazin-1-yl)carbonylamino2-phenylacetamido]-7-formamidoceph-3-em-4-carboxylate

Diphenylmethyl (6R,7R)-3-(3,4-diacetoxycinnamoyloxy)methyl-7-[(R)-2-(4-ethyl-2,3-dioxopiperazin-1-yl)carbonylamino-2-phenylacetamido]-7-formamidoceph-3-em-4-carboxylate (60mg, 0.0608mmol) was dissolved in dry $CH_2Cl_2$ (6ml) and anisole (198μl, 197mg, 1.824mmol) was added. The solution was cooled in an ice bath and trifluoroacetic acid (TFA) (141μl, 208mg, 1.824mmol) was added. After 2 hours the TFA and $CH_2Cl_2$ were removed by evaporation under reduced pressure. The residue was triturated with ether providing a solid. The solid was suspended in water (10ml) and the pH adjusted to 7 by addition of saturated aqueous $NaHCO_3$ solution. The resulting solution was treated with citrus acetylesterase preparation (ex. Sigma) (0.5ml, equivalent to approx. 2mg protein) and allowed to stand for 1 hour. Chromatography on Diaion HP20SS resin involved elution with acetone/water mixtures. The product-containing eluant was concentrated under reduced pressure then freeze-dried to give the title compound as a white solid (6.5mg); $\nu_{max}$ (KBr) 1775, 1676, 1626 and 1606cm$^{-1}$; $\delta_H$ ($D_2O$) (major rotamer) 8.11 (1H, s), 7.55-7.3 (8H, m), 7.10 (1H, s), 7.01 and 6.87 (each 1H, d, J 8.1Hz), 6.24 (1H, d, J 15.9Hz), 5.47 (1H, s), 5.26 (1H, s), 3.94 (2H, m), 3.62 (2H, m), 3.45 (2H, m), 3.39 and 2.99 (each 1H, d, J 17.6Hz), 1.13 (3H, t, J 7.1Hz).

## PREPARATION 6

Sodium (6R,7S)-7-Amino-3-azidomethyl-7-formamidoceph-3-em-4-carboxylate

7β-amino-7α-formamidocephalosporanic acid (5.2g) (European Patent Application Publication No. 0 211 656) was suspended in water (30ml) and the pH of the solution adjusted to 7 with saturated aqueous $NaHCO_3$ solution. Sodium azide (2.14g) was added to the solution which was then heated to 55°C with stirring. After 7h at this temperature, the solution was concentrated in vacuo to smaller volume and loaded onto a column of Diaion HP20SS. The column was eluted with water, monitoring fractions by h.p.l.c. Those fractions containing the product were combined and evaporated in vacuo to small volume. The solution was freeze-dried to afford the title 3-azido-methyl compound (3.65g) as a buff-coloured solid; $\nu_{max}$ (KBr) 2107, 1758, 1657 and 1609cm$^{-1}$; $\delta_H$ ($D_2O$) (major rotamer) 3.43 and 3.72 (each 1H, d, J 17.5Hz), 4.10 (2H, AA'), 5.26 (1H, s), 8.22 (.1H, s). [F.A.B. (+ve ion, thioglycerol) MH+, 321].

## PREPARATION 7

Benzhydryl (6R,7S)-7-Amino-3-azidomethyl-7-formamidoceph-3-em-4-carboxylate

Sodium (6R,7S)-7-amino-3-azidomethyl-7-formamido-ceph-3-em-4-carboxylate (85mg) was dissolved in water (2ml), and the solution was adjusted to pH 2 with dilute aqueous HCl. The mixture was evaporated in vacuo to give a solid which was suspended in acetonitrile and again evaporated to dryness. The solid was again suspended in acetonitrile (10ml) and the mixture treated with diphenyldiazomethane (65mg) with stirring.

The solid gradually went up into solution, and a further small quantity of diphenyldiazomethane was added to maintain the red colour of the solution. After 4.5h, acetic acid (3 drops) was added to the solution which, after a further 15min, was evaporated in vacuo The residue was chromatographed on silica gel eluting with ethyl acetate to give the title benzhydryl ester (96mg); $\nu_{max}$ ($CH_2Cl_2$) 3400, 2110, 1785, 1725 and 1700cm$^{-1}$; $\delta_H$($D_6$-acetone) (major rotamer) 2.75 (2H, br), 3.43 and 3.51 (each 1H, d, J 17Hz), 4.06 and 4.32 (each 1H, d, J 13.5Hz), 5.65 (1H, s), 6.96 (1H, s), 7.24-7.64 (10H, m), 8.10 (1H, br s) and 8.27 (1H, d, J 1.2Hz) [F.A.B. (+ve ion, thioglycerol) MH+, 465].

PREPARATION 8

Benzhydryl (6R,7R)-3-Azidomethyl-7-[(R)-2-(4-ethyl-2,3-dioxopiperazin-1-ylcarbonylamino)-2-phenylacetamido]-7-formamidoceph-3-em-4-carboxylate

Benzhydryl (6R,7S)-7-amino-3-azidomethyl-7-formamidoceph-3-em-4-carboxylate (0.407g) was dissolved in dry dichloromethane (20ml) and pyridine (0.173g) was added. To the stirred solution at ice-bath temperature was added dropwise a solution of (R)-[2-(4-ethyl-2,3-dioxopiperazin-1-yl)carbonylamino]-2-phenylacetyl chloride (0.595g) in dichloromethane (10ml). After the addition was complete (10 min) the reaction was stirred for a further 1h. The solution was washed with water, dilute $NaHCO_3$ solution, dilute HCl solution and brine. The organic solution was dried ($MgSO_4$) and evaporated in vacuo and the crude product chromotographed on silica gel eluting with $CHCl_3$ followed by 2%, 4% and 6% MeOH in $CHCl_3$. The title compound was obtained as a white foamy solid (0.623g); $\nu_{max}$ ($CH_2Cl_2$) 3300br, 2120, 1785, 1715sh and 1685cm$^{-1}$; $\delta_H$($d_6$-acetone) (major rotamer) 1.16 (3H, t, J 7Hz), 3.13 and 3.30 (each 1H, d, J 17Hz), 3.47 (2H, q, J 7Hz), 3.67 and 4.03 (each 2H, m), 4.16 and 4.35 (each 1H, d, J 14Hz), 5.32 (1H, s), 5.73 (1H, d, J 7Hz), 6.93 (1H, s), 7.24-7.64 (15H, m), 8.32 (1H, d, J 1Hz), 8.50 (1H, br s), 8.77 (1H, s) and 10.02 (1H, d, J 7Hz) [F.A.B. (+ve ion, 3-nitrobenzyl alcohol/sodium) MNa+, 788].

EXAMPLE 3

Sodium (6R,7R)-3-Azidomethyl-7-[(R)-2-(4-ethyl-2,3-dioxopiperazin-1-ylcarbonylamino)-2-phenylacetamido]-7-formamidoceph-3-em-4-carboxylate

Method A

A stirred solution of benzhydryl (6R,7R)-3-azidomethyl-7-[(R)-2-(4-ethyl-2,3-dioxopiperazin-1-ylcarbonylamino)-2-phenylacetamido]-7-formamidoceph-3-em-4-carboxylate (88mg) in $CH_2Cl_2$ (10ml) at 0°C was treated successively with anisole (373mg) and trifluoroacetic acid (393mg). The solution was allowed to warm to room temperature and after 3.25h was diluted with toluene. The solution was evaporated in vacuo, more toluene was added and the mixture again evaporated in vacuo to yield a solid. The product was suspended in water (5ml) and saturated $NaHCO_3$ solution added until the pH was 6.5. Residual solid was removed by filtration, and the solution freeze-dried to afford the title sodium salt (58mg) as a white solid; $\nu_{max}$ (KBr) 2106, 1772, 1679 and 1612cm$^{-1}$; $\delta_H$($D_2O$) (major rotamer) 1.22 (3H, t, J 7Hz), 3.09 and 3.50 (each 1H, d, J 17.5Hz), 3.54 (2H, q, J 7Hz), 3.72 and 4.05 (each 2H, m), 4.03 (2H, s), 5.33 (1H, s), 5.55 (1H, s), 7.54 (5H, m) and 8.17 (1H, s) [F.A.B. (+ve ion, thioglycerol) MH+ 622].

Method B

A solution of sodium (6R,7S)-7-amino-3-azidomethyl-7-formamido-ceph-3-em-4-carboxylate (0.96g) in water was acidified to (10ml) to pH3 with dilute hydrochloric acid. A white solid precipitated, and the mixture was evaporated in vacuo using ethanol to azeotrope off the water. Toluene was then added and the mixture again evaporated in vacuo to give a solid which was dried in vacuo for 30 min.

The product was then suspended in dry dichloromethane (30ml) under argon and treated with N,O-bistrimethylacetamide (0.894ml) and chlorotrimethylsilane (0.09ml) with stirring. After 1h, the mixture was cooled to 0°C and treated dropwise over 10 min. with a solution of (R)-[2-(4-ethyl-2,3-dioxopiperazin-1-yl)carbonylamino]-2-phenylacetyl chloride (1.63g) in dry dichloromethane (16ml). After 1h, the solution was evaporated in vacuo and the residue dissolved in a mixture of water (10ml) and THF (20ml). After 15 min, the pH of the solution was adjusted to 6.5 by the addition of dilute $NaHCO_3$ solution, and the solution was concentrated to small volume and freeze-dried.

The crude product was redissolved in water and the solution loaded onto a column of HP20SS. The column was eluted with water followed by 2%, 4%, 6%, 8%, 10%, 12.5%, 15% and 20% acetone in water. Fractions were monitored by h.p.l.c. and those containing pure product were combined and concentrated in vacuo to small volume. The aqueous solution was freeze-dried to yield the title cephem as a white solid (1.442g).

EXAMPLE 4

Sodium
(6R,7R)-3-Azidomethyl-7-[(R)-2-(4-ethyl-2,3-dioxopiperazin-1-ylcarbonylamino)-2-(3,4-dihydroxyphenyl)acetamido]-7-formamidoceph-3-em-4-carboxylate

Step A

A suspension of (R)-[2-(4-ethyl-2,3-dioxopiperazin-1-yl)carbonylamino]-2-(3,4-dihydroxyphenyl)acetic acid (0.263g) in dry dichloromethane (10ml) was stirred under argon with N-methyl-N-(trimethylsilyl)trifluoroacetamide (1.18ml) and chlorotrimethylsilane (0.081ml). After 16h, the clear solution was evaporated in vacuo at 35° for 40 min and the resulting oil dried under high vacuum for 10 min. The product was dissolved in dry dichloromethane (10ml) under argon and the solution stirred with thionyl chloride (116mg) for 3.5h. The solution was evaporated in vacuo to give the acid chloride as a yellow solid.

Step B

A solution of sodium (6R,7S)-7-amino-3-azidomethyl-7-formamidoceph-3-em-4-carboxylate (0.16g) in water (5ml) was treated with dilute aqueous HCl to give a pH of 2.5. The resulting suspension was evaporated in vacuo and ethanol was added to the residue. The solvent was again removed under reduced pressure and toluene then added. Evaporation in vacuo gave a solid which was dried under high vacuum for 30 min. The solid was suspended in $CH_2Cl_2$ (10ml) under argon and stirred with N,O-bistrimethylsilylacetamide (0.149ml) and chlorotrimethylsilane (0.015ml) for 2h.

Step C

A solution of the acid chloride from step A in dry dichloromethane (5ml) was added dropwise over 5min. to the mixture obtained in step B which had previously been cooled in an ice-bath. After 1h, the reaction mixture was evaporated to dryness and the residue dissolved in a mixture of water (3.5ml) and THF (10ml). After 20min, the solution was adjusted to pH6.5 with dilute $NaHCO_3$ solution, and then concentrated in vacuo to small volume. The solution was chromatographed on a solumn of HP20SS eluting with water followed by 2%, 5% and 10% acetone in water. Fractions containing the product (h.p.l.c.) were combined and evaporated in vacuo to remove acetone. The resulting aqueous solution was freeze-dried to afford the title cephem (0.213g) as a white solid; $\nu_{max}$(KBr) 2108, 1773, 1676 and 1609cm$^{-1}$; δ($D_2O$) (major rotamer) 1.17 (3H, t, J 7Hz), 3.07 and 3.45 (each 1H, d, J 17.5Hz), 3.49 (2H, q, J 7Hz), 3.68 (2H, m), 3.98 (2H, m), 3.98 (2H, s), 5.28 and 5.30 (each 1H, s), 6.87-7.01 (3H, m), and 8.07 (1H, s), [F.A.B. (+ve ion, thioglycerol) MH+ 654 and MNa+ 676].

PREPARATION 9

Sodium
(6R,7R)-3-Aminomethyl-7-[(R)-2-(4-ethyl-2,3-dioxopiperazin-1-ylcarbonylamino)-2-phenylacetamido]-7-formamidoceph-3-em-4-carboxylate

Method A

To a solution of sodium (6R,7R)-3-azidomethyl-7-[(R)-2-(4-ethyl-2,3-dioxopiperazin-1-ylcarbonylamino)-2-phenylacetamido]-7-formamidoceph-3-em-4-carboxylate (40mg) in a mixture of water (3ml) and THF (4ml) was added 5% palladium on charcoal (60mg). The mixture was shaken in an atmosphere of hydrogen at ambient pressure and temperature for 40min, and then filtered through Kieselguhr. The filtrate was concentrated under reduced pressure to small volume and the solution was chromatographed on HP20SS employing a gradient elution of water to 20% acetone in water. Fractions were monitored by h.p.l.c. and those containing only the desired product were combined and concentrated under reduced pressure. The resulting aqueous solution was freeze-dried to give the title amine (11mg) as a white solid; $\nu_{max}$ (KBr) 1774, 1675 and 1611cm$^{-1}$; $\delta_H$($D_2O$) (major rotamer) 1.16 (3H, t, J 7Hz), 3.10 (1H, d, J 17.5Hz), 3.41-3.75 (7H,m), 3.99 (2H, m), 5.27 (1H, s), 5.48 (1H, s), 7.46 (5H, m) and 8.12 (1H, s); [F.A.B. (+ve ion, thioglycerol) MH+ 574, MNa+ 596].

Method B

The 3-azidomethyl cephem of Example 3 (250mg) was dissolved in a mixture of water (7ml) and THF (15ml). Lindlar catalyst (5% palladium on calcium carbonate, poisoned with lead) (375mg) was added to the solution which was then shaken under hydrogen (ambient temperature and pressure) for 1h. A further quantity (250mg) of Lindlar catalyst was added and the hydrogenation reaction continued for a further 3h. The mixture was then worked-up and the product purified as in method A to afford the title 3-aminomethyl cephem (87mg).

EXAMPLE 5

Sodium (6R,7R)-3-(3,4-Diacetoxybenzoyl)aminomethyl-7-[(R)-2-(4-ethyl-2,3-dioxopiperazin-1-ylcarbonylamino)-2-phenylacetamido]-7-formamidoceph-3-em-4-carboxylate

Sodium (6R,7R)-3-aminomethyl-7-[(R)-2-(4-ethyl-2,3-dioxopiperazin-1-ylcarbonylamino)-2-phenylacetamido]-7- formamidoceph-3-em-4-carboxylate (40mg) was dissolved in a mixture of water (2ml), THF (2ml), and saturated aqueous $NaHCO_3$ solution (0.15ml). To the cooled (ice-bath) stirred solution was added dropwise over 5min a solution of 3,4-diacetoxybenzoyl chloride [prepared from 3,4-diacetoxybenzoic acid (32mg) as described in Preparation 4(a)] in THF (2ml). The pH of the solution was not allowed to fall below 7 by the occasional addition of $NaHCO_3$ solution. After 0.5h at ice-bath temperature and 1.5h at room temperature, the reaction mixture was concentrated in vacuo to remove THF and the aqueous solution was adjusted to pH7 before chromatographing on a column of HP20SS. A gradient elution of water to 30% acetone in water was employed, and fractions were monitored by h.p.l.c. Those containing the product were combined, concentrated in vacuo to small volume and freeze-dried to afford the title acylamino compound (23mg); $\nu_{max}$ (KBr) 1769, 1715, 1677 and 1610cm$^{-1}$; $\delta_H(D_2O)$ (major rotamer) 1.16 (3H, t, J 7Hz), 2.35 (6H, s), 2.87 and 3.28 (each 1H, d, J 17.5Hz), 3.47 (2H, q, J 7Hz), 3.62 (2H, m), 3.94 (2H, m), 4.08 and 4.30 (each 1H, d, J 15Hz), 5.27 (1H, s), 5.47 (1H, s), 7.27-7.70 (8H, m) and 8.12 (1H, s); [F.A.B. (+ve ion, thioglycerol) MH+, 816].

## EXAMPLE 6

Sodium (6R,7R)-3-[(E)-3,4-Diacetoxycinnamoyl]aminomethyl-7-[(R)-2-(4-ethyl-2,3-dioxopiperazin-1-ylcarbonylamino)-2-phenylacetamido]-7-formamido-ceph-3-em-4-carboxylate

Using an exactly analogous procedure to that of Example 5, the 3-aminomethylcephem of Preparation 9 (70mg) was acylated with 3,4-diacetoxycinnamoyl chloride. The latter was prepared from 3,4-diacetoxycinnamic acid (62mg) by the general procedure of Preparation 4(a). After purification by HP20SS chromatography, the title sodium salt was obtained as a freeze-dried solid (45mg); $\nu_{max}$ (KBr) 1768, 1710sh, 1676 and 1611cm$^{-1}$; $\delta_H(D_2O)$ (major rotamer) 1,12 (3H, t, J 7Hz), 2.30 and 2.32 (each 3H, s), 2.86 and 3.30 (each 1H, d, J 17.5Hz), 3.43 (2H, q, 7Hz), 3.55 (2H, m), 3.87 (2H, m), 3.97 and 4.19 (each 1H, d, J 15Hz), 5.25 (1H, s), 5.45 (1H, s), 6.34 (1H, d, J 16Hz), 7.11-7.47 (9H, m), 8.12 (1H, s); [F.A.B. (+ve ion, thioglycerol) MH+ 842, MNa+ 864].

## EXAMPLE 7

Sodium (6R,7R)-3-(3,4-Dihydroxybenzoyl)aminomethyl7-[(R)-2-(4-ethyl-2,3-dioxopiperazin-1-ylcarbonylamino) -2-phenylacetamido]-7-formamido-ceph-3-em-4-carboxylate

Sodium (6R,7R)-3-azidomethyl-7-[(R)-2-(4-ethyl-2,3-dioxopiperazin-1-ylcarbonylamino)-2-phenylacetamido]-7-formamidoceph-3-em-4-carboxylate (300mg) was hydrogenated in the presence of Lindlar catalyst as described in Preparation 9, Method B. The aqueous solution of crude amine, without purification on HP20SS, was acylated wth 3,4-diacetoxybenzoyl chloride (ca. 145mg) as described in Example 5, to provide an aqueous solution of the pure 3-(3,4-diacetoxybenzoyl)aminomethyl cephem after chromatography on HP20SS. This solution (ca. 30ml) was treated with a preparation of citrus acetylesterase (Sigma chemicals) (1ml, equivalent to 4mg protein). The pH of the solution was kept at 7 by the periodic addition of dilute $NaHCO_3$ solution, and the reaction was monitored by h.p.l.c. After 1h the solution was concentrated in vacuo to small volume and loaded onto a column of HP20SS which was eluted with water followed by 2.5%, 5%, 7.5%, 10% and 15% acetone in water. Fractions containing the product (by h.p.l.c) were combined and concentrated to small volume. The solution was freeze-dried to give the title cephem as a white solid (56mg); $\nu_{max}$(KBr) 1774, 1710sh, 1677 and 1598cm$^{-1}$; $\delta_H(D_2O)$ (major rotamer) 1.16 (3H, t, J 7Hz), 2.83 and 3.23 (each 1H, d, J 17Hz), 3.47 (1H, q, J 7Hz), 3.63 (2H, m), 3.97 (2H, m), 4.07 and 4.27 (each 1H, d, J 15Hz), 5.26 (1H, s), 5.47 (1H, s), 6.95 (1H, d, J 8.5Hz), 7.21-7.50 (7H, m) and 8.12 (1H, s). [F.A.B. (+ve ion, thioglycerol) MH+, 732].

## EXAMPLE 8

Sodium (6R,7R)-3-(5-Hydroxy-4-oxo-1,4-dihydropyridin-2-yl)carbonylaminomethyl-7-[(R)-2-(4-ethyl-2,3-dioxopiperazin-1-ylcarbonylamino)-2-phenylacetamido]-7-formamidoceph-3-em-4-carboxylate

A mixture of 5-hydroxy-4-pyridone-2-carboxylic acid (34mg) (Chem. Ber. 1954, Vol. 87, p.1440), N-hydroxybenzotriazole (30mg) and N,N′-dicyclohexylcarbodiimide (46mg) in N,N-dimethylformamide (2ml), was stirred at room temperature for 4h. The precipitated N,N′-dicyclohexylurea was removed by filtration to leave a solution of active ester. To this solution at 0° was added a solution of sodium (6R,7R)-3-aminomethyl-7-[(R)-2-(4-ethyl-2,3-dioxopiperazin-1-ylcarbonylamino)-2-phenylacetamido]-7-formamidoceph-3-em-4-carboxylate (35mg) in N,N- dimethylformamide (0.5ml).

After stirring the reaction mixture at 0° for 1h, and at ambient temperature for 18h, the solvent was removed in vacuo. The residue was suspended in a little water and the suspension was loaded onto a column of Diaion HP20SS. Elution with water followed by 2.5%, 5%, 7.5%, 10%, 15% and 20% acetone in water gave fractions

containing the acylated product (monitored by h.p.l.c.). These were combined, concentrated to small volume, and freeze-dried to afford a solid (5mg) containing ca. 50% of the title cephem; $\nu_{max}$ (KBr) 1775, 1705 and 1677cm$^{-1}$; $\delta_H$ ($D_2O$) inter alia (major rotamer) 1.17 (3H, t, J 7Hz), 2.85 and 3.27 (each 1H, d, J 17.5Hz), 3.48 (2H, q, J 7Hz), 3.68 (2H, m), 3.99 (2H, m), 4.11 and 4.32 (each 1H, d, J 15Hz), 5.27 (1H, s), 5.48 (1H, s), 7.12 (1H, s), 7.46 (5H, m), 7.81 (1H, s), 8.12 (1H, s); [F.A.B. (+ve ion, thioglycerol) MH+, 733].

EXAMPLE 9

Sodium (6R,7R)-3-(2,3-Dihydroxybenzoyl)aminomethyl-7-[(R)-2-(4-ethyl-2,3-dioxopiperazin-1-ylcarbonylamino)-2-phenylacetamido]-7-formamidoceph-3-em-4-carboxylate

A solution of a ca. 40% pure sample of sodium (6R,7R)-3-aminomethyl-7-[(R)-2-(4-ethyl-2,3-dioxopiperazin-1-ylcarbonylamino)-2-phenylacetamido]-7-formamidoceph-3-em-4-carboxylate in a mixture of THF (6ml) and water (3ml) was adjusted to pH 8.5 by the addition of a small quantity of aqueous sodium hydrogencarbonate solution. To this cooled (ice-bath), stirred solution was added dropwise over 2 min. a solution of 2,3-diacetoxybenzoyl chloride [prepared from 2,3-diacetoxybenzoic acid (0.25mmol) by the general procedure of Preparation 4(a)] in THF (1ml). The pH of the solution was kept between 7 and 8.5 by the addition of aqueous sodium hydrogencarbonate solution as necessary. After 1h, the solution was concentrated in vacuo to remove THF and then chromatographed on HP20SS eluting with water followed by acetone-water mixtures (to 25% acetone-water). Fractions containing the product (monitored by h.p.l.c.) were combined, concentrated in vacuo and freeze-dried.

The resulting solid (38mg) was dissolved in water (3ml) and the solution was treated with citrus acetylesterase preparation (0.35ml). The solution was gently agitated, keeping the pH at 7 by the occasional addition of dilute sodium hydrogencarbonate solution. After 1.25h, the solution was filtered and then chromatographed on HP20SS employing a gradient elution starting with water and increasing to 20% acetone in water. Fractions containing the product (monitored by h.p.l.c.) were combined, concentrated in vacuo, and freeze-dried to afford the title cephem (20mg); $\nu_{max}$(KBr) 1774, 1705 and 1674cm$^{-1}$; $\delta_H$ ($D_2O$ + acetone-$d_6$) (major rotamer) 1.18 (3H, t, J 7Hz), 2.86 and 3.29 (each 1H, d, J 17.5Hz), 3.50, 3.68 and 3.99 (each 2H, m), 4.13 and 4.37 (each 1H, d, J 15Hz), 5.28 (1H, s), 5.51 (1H, s), 6.77 and 7.07 (each 1H, m), 7.21-7.51 (6H, m), 8.15 (1H, s); [F.A.B. (+ve ion, thioglycerol), MH+ 732].

PREPARATION 10

Diphenylmethyl [6R,7R]-7-[2-(2-chloroacetylamino-4-thiazolyl)-2-((Z)-methoxyimino)acetamido]-3-hydroxymethylceph-3-em-4-carboxylate

Sodium [6R,7R]-3-acetoxymethyl-7-[2-(2-chloroacetylamino-4-thiazolyl)-2-((Z)-methoxyimino)acetamido-]ceph-3-em-4-carboxylate (0.85g) (J. Antibiotics, 1981, Vol. 34, p.171) was dissolved in potassium dihydrogen phosphate solution (0.1M 17ml) and the pH adjusted to 7.1 with dilute aqueous sodium bicarbonate. The solution was treated with solid supported citrus acetylesterase enzyme overnight. H.p.l.c. analysis showed complete deacetylation. The enzyme was filtered off and washed with a little water. The aqueous solution was concentrated to ca. 10ml and with vigorous stirring acidified to pH 2.0 with dilute hydrochloric acid. The solid was filtered off, washed with a little water and then suspended in acetonitrile (20ml) and water (10ml). Diphenyldiazomethane (450mg) was added and the mixture stirred for 2 hours at room temperature. The mixture was evaporated to dryness and the residue triturated with ethyl acetate. The solid product was collected to give the title compound (0.48g); $\nu_{max}$ (Nujol) 3290, 1770, 1715, 1670, 1650cm$^{-1}$; $\delta_H$ ($CD_3COCD_3$), 3.67 (1H, d, J 18.5Hz), 3.76 (1H, d, J 18.5Hz), 3.95 (3H, s), 4.33 (1H, d, J 14Hz), 4.45 (1H, d, J 14Hz), 4.50 (2H, s), 5.24 (1H, d, J 4.8Hz), 6.04 (1H, dd, J 4.8 and 11Hz), 6.93 (1H, s), 7.2-7.6 (11H, multiplet complex), 8.53 (1H, d, J 11Hz). [F.A.B. (+ve ion, thioglycerol) MH+ 656].

PREPARATION 11

Diphenylmethyl (6R,7R)-7-[2-(2-chloroacetylamino-4-thiazolyl)-2-((Z)-methoxyimino)acetamido]-3-(3,4diacetoxybenzoyloxy)methylceph-3-em-4-carboxylate

Diphenylmethyl [6R,7R]-7-[2-(2-chloroacetylamino-4thiazolyl-2-((Z)-methoxyimino)acetamido]-3-hydroxymethylceph-3-em-4-carboxylate (130mg, 0.2mmol) was suspended in dichloromethane (15ml) and treated with pyridine (40µl). 3,4-Diacetoxybenzoyl chloride prepared as described in Preparation 4(a) (129mg, 0.5mmol) in dichloromethane (10ml) was added to the suspension, and the mixture stirred overnight. The solution was evaporated to dryness and the product purified by chromatography on silica gel eluting with mixtures of ethyl acetate and hexane. Fractions containing the product were combined and evaporated to give the title compound as a white foam (97 mg); $\nu_{max}$($CH_2Cl_2$) 1790 shoulder, 1770, 1720, 1680cm$^{-1}$; $\delta_H$($CDCl_3$) 2.32 (6H, s), 3.49 (1H, d, J 18.5Hz), 3.65 (1H, d, J 18.5Hz), 4.11 (3H, s), 4.17 (2H, AA'), 5.04 (1H, d, J 13.5Hz), 5.14 (1H, d, J 5Hz), 5.34 (1H, d, J 13.5Hz), 6.08 (1H, dd, J 5 and 9Hz), 6.99 (1H, s), 7.2-7.45 (12H, m), 7.50 (1H, d, J 9Hz), 7.8-8.0 (2H, m).

PREPARATION 12

Diphenylmethyl (6R,7S)-7-[(R)-2-(4-ethyl-2,3-dioxopiperazin-1-yl)carbonylamino-2-phenylacetamido]-3-hydroxymethyl-7-methoxyceph-3-em-4-carboxylate

Sodium (6R,7S)-3-acetoxymethyl-7-[(R)-2-(4-ethyl-2,3-dioxopiperazin-1-yl)carbonylamino-2-phenylacetamido]-7-methoxyceph-3-em-4-carboxylate (137mg, 0.21mmol) (Ger. Offen. 2,702,552) in $H_2O$ (10ml) was adjusted to pH 7.0 by addition of saturated aqueous $NaHCO_3$ solution. Citrus acetylesterase preparation (ex. Sigma) (0.14ml, equivalent to approx. 0.56mg of protein) was added and the mixture agitated gently for 1h. A further quantity of the citrus acetylesterase preparation (0.14ml) was added and gentle agitation continued for 2 hours. Ethyl acetate (30ml) was added and the mixture acidified to pH 2.0 with 1M aqueous HCl solution. The organic phase was separated and the aqueous phase further extracted with ethyl acetate (4 × 30ml). The combined organic extracts were washed with brine and dried over $MgSO_4$. Diphenyldiazomethane (41mg, 0.21mmol) was added and the reaction mixture stirred for 4 hours. During this time the volume of the solution was gradually reduced to ca. 25ml under reduced pressure. The final solution was evaporated to dryness and chromatographed on silica gel eluting with ethyl acetate to give the title compound as a white solid (76mg); $\nu_{max}$ ($CH_2Cl_2$) 3390, 3270, 2940, 1780, 1710 and 1690cm$^{-1}$. $\delta_H$ ($CD_3COCD_3$) 9.98 (1H, d, J 6Hz), 8.66 (1H, s), 7.65-7.20 (15H, m), 6.88 (1H, s), 5.73 (1H, d, J 6Hz), 5.07 (1H, s), 4.48 and 4.35 (each 1H, d, J 14Hz), 4.20 (1H, s), 4.11-3.95 (2H, m), 3.79-3.63 (2H, m), 3.55-3.40 (6H, m), 3.23 (1H, d), 1.17 (3H, t, J 7Hz). [F.A.B. (+ve ion, 3-nitrobenzylalcohol-NaOAc) MNa+ 750].

PREPARATION 13

Diphenylmethyl (6R,7S)-3-[(3,4-Diacetoxybenzoyloxy)methyl-7-[(R)-2-(4-ethyl-2,3-dioxopiperazin-1-yl)carbonylamino-2-phenylacetamido]-7-methoxyceph-3-em-4-carboxylate

Diphenylmethyl (6R,7S)-7-[(R)-2-(4-ethyl-2,3-dioxopiperazin-1-yl)carbonylamino-2-phenylacetamido]-3-(hydroxymethyl)-7-methoxyceph-3-em-4-carboxylate (96mg, 0.13mmol) in $CH_2Cl_2$) (10ml) was treated successively with pyridine (26µl, 25mg, 0.32mmol), and a solution of 3,4-diacetoxybenzoyl chloride (prepared from the corresponding acid, as described in Preparation 4(a)) (0.08g, 0.33mmol) in $CH_2Cl_2$ (5ml). After stirring for 18 hours, the mixture was diluted with EtOAc (20ml), washed with M HCl (2 × 25ml) saturated aqueous $NaHCO_3$ solution (2 × 25ml), brine (25ml), dried over $MgSO_4$, and the solvent evaporated under reduced pressure. The residue was chromatographed on silica gel, eluting with ethanol/chloroform (1:50) to give the title compound (53mg) as a white solid; $\nu_{max}$ ($CH_2Cl_2$) 3390, 3270, 1780, 1715 and 1690cm$^{-1}$; $\delta_H$ ($CD_3COCD_3$) 9.94 (1H, d, J 7Hz), 8.72 (1H, s), 7.95-7.73 (2H, m), 7.65-7.15 (16H, m), 6.94 (1H, s), 5.72 (1H, d, J 7Hz), 5.15 (1H, s), 5.28 and 5.08 (each 1H, d, J 13Hz), 4.15-3.90 (2H, m), 3.80-3.60 (2H, m), 3.67 and 3.41 (each 1H, d, J 17Hz), 3.53 (3H, s), 3.50 (2H, q, J 7Hz), 2.32 (6H, s), 1.17 (3H, t, J 7Hz). [F.A.B. (+ve ion, 3-nitrobenzylalcohol-NaOAc) MNa+ 970].

EXAMPLE 10

Sodium (6R,7R)-7-[2-(2-amino-4-thiazolyl)-2-((Z)-methoxyimino)acetamido]-3-(3,4-dihydroxybenzoyloxy)methylceph-3-em-4-carboxylate

Diphenylmethyl (6R,7R)-7-[2-(2-chloroacetylamino-4thiazolyl)-2-((Z)-methoxyiminoacetamido]-3-(3,4-diacetoxybenzoyloxy)methylceph-3-em-4-carboxylate (165mg, 0.19mmol) was dissolved in dichloromethane (18ml) at room temperature and treated with anisole (620µl) and trifluoroacetic acid (440µl). After 2 hours the solutions was diluted with toluene (40ml) and evaporated to dryness in vacuo. The residue was triturated with dry ether (3 × 20ml) stored in vacuo for 30 mins. and then redissolved in water (20ml). The pH was adjusted to 7.2 with dilute aqueous sodium bicarbonate. The solution was treated with citrus acetylesterase enzyme preparation (2ml) maintaining the pH at 7.0 over 2.5 hours and monitoring by h.p.l.c. until the deacetylation was complete. The mixture was filtered through celite and evaporated to dryness by azeotroping with toluene.

The residue was dissolved in water (10ml) and treated with dilute aqueous sodium bicarbonate to pH 7.0 and then sodium N-methyl dithiocarbamate (12mg). The reaction was monitored by h.p.l.c. and required a further batch of sodium N-methyl dithiocarbamate (12mg) for completion. The product was purified on HP20SS resin eluting with water and mixtures of water and acetone. Fractions containing the product were concentrated and freeze dried to give the title compound as a buff amorphous solid (39mg); $\nu_{max}$ (KBr) 1763, 1670, 1603, 1528cm$^{-1}$; $\delta_H$ ($D_2O$) 3.46 (1H, d, J 18Hz), 3.73 (1H, d, J 18Hz), 3.98 (3H, s), 4.92 (1H, d, J 12.5Hz), 5.17 (1H, d, J 12.5Hz), 5.21 (1H, d, J 4.8Hz), 5.81 (1H, d, J 4.8Hz), 6.9-7.0 (2H, m), 7.45-7.55 (2H, m).

EXAMPLE 11

Sodium
(6R,7S)-3-[(3,4-Dihydroxybenzoyloxy)methyl]-7-[(R)-2-(4-ethyl-2,3-dioxopiperazin-1-yl)carbonylamino-2-phenylacetamido]-7-methoxyceph-3-em-4-carboxylate

Diphenylmethyl (6R,7S)-3-[(3,4-diacetoxybenzoyloxy)methyl]-7-[(R)-2-(4-ethyl-2,3-dioxopiperazin-1-yl)carbonylamino-2-phenylacetamido]-7-methoxyceph-3-em-4-carboxylate (65mg, 0.069mmol) in $CH_2Cl_2$ (5ml) was cooled in an ice bath and treated successively with anisole (220µl, 218mg, 2.07mmol) and TFA (160µl, 159mg, 2.07mmol). The reaction mixture was stirred at 0° for 30 minutes, and room temperature for 1.5 hours, then evaporated to dryness under reduced pressure. The residue was diluted with toluene and evaporated, then triturated with diethyl ether to afford a yellow solid. The solid was suspended in water (10ml) and the pH adjusted to 7 by addition of saturated aqueous $NaHCO_3$ solution. The resulting solution was treated with citrus acetylesterase (0.6ml, equivalent to approx. 2.4mg protein) and gently agitated for 1 hour. Further enzyme (0.6ml) was added and the reaction allowed to proceed for 3 hours. The reaction mixture was filtered through celite and chromatographed on Diaion HP20SS resin eluting with acetone/water mixtures. Concentration under reduced pressure of the product-containing eluant followed by lyophilisation gave the title compound as a white solid (18mg); $\nu_{max}$(KBr) 1774, 1708, 1677 and 1610cm$^{-1}$; $\delta_H$ ($D_2O$) 9.87 (1H, d, J 7Hz), 7.28-7.60 (8H, m), 6.92 (1H, d), 5.53 (1H, d, J 7Hz), 5.12 (1H, s), 5.12 and 4.97 (each 1H, d, J 12.5Hz), 4.14-3.90 (2H, m), 3.83-3.62 (2H, m), 3.55 (3H, s), 3.50 (2H, q, J 7Hz), 3.45 and 3.02 (each 1H, d, J 17.5Hz), 1.18 (3H, t, J 7Hz). [F.A.B. (+ve ion, thioglycerol) MH+ 720].

## PREPARATION 14

Sodium
(6R,7R)-3-Acetoxymethyl-7-[(S)-2-(4-ethyl-2,3-dioxopiperazin-1-yl)carbonylamino-2-(2-thienyl)acetamido-7-formamidoceph-3-em-4-carboxylate

(R,S)-2-[(4-ethyl-2,3-dioxopiperazin-1-yl)carbonylamino]-2-(2-thienyl)acetic acid (3.92g, 12.7mmol) in $CH_2Cl_2$ (150ml) under argon was treated with dry DMF (2 drops) and oxalyl chloride (2.0ml, 23mmol) and stirred for 2 hours. The solution was evaporated to dryness and then reevaporated from toluene (50ml). The residue was dissolved in $CH_2Cl_2$ (50ml) and added to a solution of (6R,7S)-3-acetoxymethyl-7-amino-7-formamidocephalosporanic acid (2.0g, 6.35mmol) in $CH_2Cl_2$ (180ml) which had been previously stirred with N,O-bistrimethylsilylacetamide (2.0ml) and trimethylsilyl chloride (0.2ml) for 2 hours.
After the addition, the reaction was stirred for 3 hours and then evaporated to dryness. The residue was dissolved in THF/water (1:1, 100ml) and stirred for 20 minutes. The pH was adjusted to 6.8 with saturated aqueous $NaHCO_3$ solution and the THF removed by evaporation. The product was purified by chromatography on HP20SS eluting with acetone/water mixtures to give the title compound (2.48g) after concentration and freeze drying. The S:R ratio of the product was approximately 5:1; $\nu_{max}$ (KBr) 1774, 1715, 1676 and 1611cm$^{-1}$; $\delta_H$ ($D_2O$) (major rotamer of S-isomer) 8.11 (1H, s), 7.45 (1H, m), 7.25 (1H, m), 7.05 (1H, m), 5.79 (1H, s), 5.27 (1H, s), 4.77 (1H, d, J 13.0Hz), 4.60 (1H, d, J 13.0Hz), 4.00 (2H, m), 3.68 (2H, m), 3.51 and 3.12 (each 1H, d, J 18.0Hz), 3.48 (2H, q, J 7.3Hz), 2.04 (3H, s), 1.16 (3H, t, J 7.3Hz).

## PREPARATION 15

Diphenylmethyl
(6R,7R)-3-(3,4-Diacetoxybenzoyloxy)methyl-7-[(S)-2-(4-ethyl-2,3-dioxopiperazin-1-yl)carbonylamino-2-(2-thienyl)acetamido]-7-formamidoceph-3-em-4-carboxylate

Sodium (6R,7R)-3-acetoxymethyl-7-[(S)-2-(4-ethyl-2,3-dioxopiperazin-1-yl)carbonylamino-2-(2-thienyl)acetamido]-7-formamidoceph-3-em-4-carboxylate (1.0g, 1.6mmol) was dissolved in 0.1M aqueous $KH_2PO_4$ solution (15ml) and treated with aqueous $NaHCO_3$ solution to pH 7.0. A preparation of citrus acetylesterase supported on sepharose was added and the sealed flask tumbled overnight. The enzyme complex was filtered off and washed with 0.1M aqueous $KH_2PO_4$ solution (10ml). The filtrate was treated with a further quantity of citrus acetylesterase preparation to completion, the deacetylation being monitored by h.p.l.c. The mixture was filtered and acidified to pH 2.0 with dilute hydrochloric acid and then extracted exhaustively with EtOAc (20 × 50ml). The organic solution was dried ($MgSO_4$) and concentrated until a hazy precipitate appeared. Diphenyldiazomethane (350mg, 1.78mmol) was added and the esterification monitored by t.l.c.

On completion the mixture was evaporated to dryness and this intermediate was purified by chromatography on silica gel eluting with mixtures of EtOAc/methanol. The intermediate (280mg, 0.38mmol) was dissolved in $CH_2Cl_2$ (30ml), pyridine (62µl, 0.77mmol) was added followed by 3,4-diacetoxybenzoyl chloride (0.77mmol, prepared as described in Preparation 4(a)) in $CH_2Cl_2$ (10ml). The mixture was stirred overnight and then washed with dilute aqueous $NaHCO_3$ solution, dilute hydrochloric acid and brine, dried over $MgSO_4$ and evaporated to dryness. The residue was purified by chromatography on silica gel eluting with EtOAc/methanol mixtures. Fractions containing the product were combined and evaporated to give the title compound as a colourless foam (220mg); $\nu_{max}$ ($CH_2Cl_2$) 1785, 1720 and 1695cm$^{-1}$; $\delta_H$ ($CD_3COCD_3$) (major rotamer) 8.24 (1H, s), 8.0-7.2 (16H, m), 6.92 (1H, s), 5.97 (1H, s), 5.35 (1H, s), 5.24 and 5.06 (each 1H, d, J 13.7Hz), 4.05 (2H, m), 3.7 (2H, m), 3.54 and 3.28 (each 1H, d, J 17.5Hz), 3.48 (2H, q, J 7.2Hz), 2.30 (6H, s) and 1.14 (3H, t, J 7.2Hz).

PREPARATION 16

Diphenylmethyl (6R,7R)-3-[(3,4-Diacetoxyphenyl)acetoxymethyl]-7-[(R)-2-(4-ethyl-2,3-dioxopiperazin-1-yl)carbonylamino-2-phenylacetamido]-7-formamidoceph-3-em-4-carboxylate

Diphenylmethyl (6R,7R)-7-[(R)-2-(4-ethyl-2,3-dioxopiperazin-1-yl)carbonylamino-2-phenylacetamido]-7-formamido-3-(hydroxymethyl)ceph-3-em-4-carboxylate (200mg, 0.27mmol) (obtained as described in Preparation 18) in $CH_2Cl_2$ (10ml) was cooled to 0°C and treated successively with pyridine (65μl, 64mg, 0.81mmol) and a solution of 3,4-(diacetoxyphenyl)acetyl chloride [prepared from the corresponding acid by the procedure described in Preparation 4(a), 220mg, 0.87mmol] in $CH_2Cl_2$ (5ml). The reaction mixture was stirred overnight, diluted with EtOAc (40ml), washed with 1M HCl (2 × 30ml), saturated aqueous $NaHCO_3$ solution (2 × 30ml), brine (30ml), dried over $MgSO_4$ and evaporated under reduced pressure. The residue was chromatographed on silica gel, eluting with ethanol/chloroform mixtures to afford the title compound (219mg) as a yellow foam; $\nu_{max}$ ($CH_2Cl_2$) 3280, 3050, 1795 sh, 1770, 1715, 1690cm$^{-1}$; $\delta_H$ ($CD_3COCD_3$) (major rotamer) 10.04 (1H, d, J 7Hz), 8.76 (1H, s), 8.53 (1H, s), 8.29 (1H, s), 7.67-7.13 (18H, m), 6.92 (1H, s), 5.74 (1H, d, J 7Hz), 5.29 (1H, s), 5.07 and 4.88 (each 1H, d, J 14Hz), 4.12-3.95 (2H, m), 3.75-3.58 (4H, m), 3.50 (2H, q, J 7Hz), 3.26-3.06 (2H, ABq, J 17Hz), 2.27 (6H, s), 1.16 (3H, t, J 7Hz); [F.A.B. (+ve ion, 3-nitrobenzylalcohol-NaOAc) MNa+ 997].

PREPARATION 17

Diphenylmethyl (6R,7R)-7-[(R)-2-(4-Ethyl-2,3-dioxopiperazin-1-yl)carbonylamino-2-phenylacetamido]-7-formamido-3-(3,4,5-triacetoxybenzoyloxy)methylceph-3-em-4-carboxylate

Diphenylmethyl (6R,7R)-7-[(R)-2-(4-ethyl-2,3-dioxopiperazin-1-yl)carbonylamino-2-phenylacetamido]-7-formamido-3-(hydroxymethyl)ceph-3-em-4-carboxylate (140mg, 0.19mmol) (obtained as described in Preparation 18) in $CH_2Cl_2$ (10ml) was cooled in an ice-bath and treated successively with pyridine (110μl, 108mg, 1.36mmol) and a solution of 3,4,5-triacetoxybenzoyl chloride (prepared from the corresponding acid, 280mg, 0.95mmol according to the general procedure of Preparation 4(a)) in $CH_2Cl_2$ (5ml). The reaction was stirred at room temperature overnight, diluted with EtOAc (30ml), washed with M HCl (2 × 25ml), saturated aqueous $NaHCO_3$ solution (2 × 25ml), brine (25ml), dried over $MgSO_4$, and the solvent evaporated under reduced pressure. Chromatography on silica gel eluting with ethanol/ethyl acetate (1:50) gave the title compound as a white solid (81mg); $\nu_{max}$ ($CH_2Cl_2$) 3280, 3160, 1785, 1715, and 1690cm$^{-1}$; $\delta_H$ ($CD_3COCD_3$) (major rotamer) 10.02 (1H, d), 8.79 (1H, s), 8.51 (1H, s), 8.30 (1H, d, J 1Hz), 7.69-7.16 (17H, m), 6.94 (1H, s), 5.73 (1H, d, J 7Hz), 5.63 (1H, s), 5.27 and 5.15 (each 1H, d, J 14Hz), 4.09-3.98 (2H, m), 3.75-3.64 (2H, m), 3.50 (2H, q, J 7Hz), 3.38 and 3.12 (each 1H, d, J 17Hz), 2.34, 2.32, 2.31 (each 3H, s), 1.16 (3H, t, J 7Hz). [F.A.B. (+ve ion, 3-nitrobenzylalcohol-NaOAc) MNa+ 1041].

EXAMPLE 12

Sodium (6R,7R)-3-(3,4-Dihydroxybenzoyloxy)methyl-7-[(S)-2-(4-ethyl-2,3-dioxopiperazin-1-yl)carbonylamino-2-(2-thienyl)acetamido]-7-formamidoceph-3-em-4-carboxylate

Diphenylmethyl (6R,7R)-3-(3,4-diacetoxybenzoyloxy) methyl-7-[(S)-2-(4-ethyl-2,3-dioxopiperazin-1-yl)carbon ylamino-2-(2-thienyl)acetamido]-7-formamidoceph-3-em-4-carboxylate (220mg, 0.23mmol) in $CH_2Cl_2$ (20ml) was treated with anisole (750μl, 6.9mmol) and trifluoroacetic acid (520μl, 6.9mmol). The reaction was monitored by t.l.c. for disappearance of starting material. On completion, toluene (30ml) was added and the mixture evaporated to dryness. The residue was triturated with ether (3 × 20ml) to removed anisole. The solid was dissolved in water and the pH adjusted to 7.0 with aqueous $NaHCO_3$ solution. Citrus acetyl esterase preparation was added. The deacetylation was monitored by h.p.l.c. and the mixture filtered and freeze dried. The product was purified by chromatography on HP20SS resin eluting with water/acetone mixtures. Fractions containing the product were combined, concentrated and freeze dried to give the title compound as a white amorphous solid (76mg); $\nu_{max}$(KBr) 1775, 1710, 1680 and 1600cm$^{-1}$; $\delta_H$ ($D_2O$) (major rotamer) 8.12 (1H, s), 7.45-6.85 (6H, m), 5.78 (1H, s), 5.27 (1H, s), 5.05 and 4.84 (each 1H, d, J 13.5Hz), 3.95 (2H, m), 3.63 (2H, m), 3.50 and 3.13 (each 1H, d, J 17.1Hz), 3.45 (2H, q, J 7.2Hz) and 1.14 (3H, t, J 7.2Hz); [F.A.B. (+ve ion, thioglycerol) MH+ 739].

EXAMPLE 13

Sodium (6R,7R)-3-[(3,4-dihydroxyphenyl)acetoxymethyl]-7-[(R)-2-(4-ethyl-2,3-dioxopiperazin-1-yl)carbonylamino-2-phenylacetamido]-7-formamidoceph-3-em-4-carboxylate

Diphenylmethyl (6R,7R)-3-[(3,4-diacetoxyphenyl)acetoxymethyl]-7-[(R)-2-(4-ethyl-2,3-dioxopiperazin-1-yl)carbonylamino-2-phenylacetamido]-7-formamidoceph-3-em-4-carboxylate (220mg, 0.23mmol) in $CH_2Cl_2$ (l0ml) was cooled to 0°C and treated with anisole (740μl, 733mg, 7.07mmol) and TFA (520μl, 771mg,

6.80mmol). The mixture was stirred at 0°C for 30 minutes, room temperature for 1.5 hours, then evaporated to dryness. The residue was diluted with toluene, evaporated under reduced pressure and triturated with diethyl ether to give a yellow solid. The solid was suspended in water and the pH was adjusted to 7 by addition of saturated aqueous $NaHCO_3$ solution. The solution obtained was treated with citrus acetylesterase preparation (2.2ml, equivalent to approx. 8.8mg protein) and gently agitated for 1 hour, further enzyme (2ml) was added and the reaction allowed to proceed for 1.5 hours. The mixture was filtered through celite and chromatographed on Diaion HP20SS, eluting with acetone/water mixtures to give the title compound as a white freeze-dried solid (43mg); $\nu_{max}$ (KBr) 1775, 1710, 1677 and 1607cm$^{-1}$; $\delta_H$ ($D_2O$) 8.10 (1H, s), 7.57-7.25 (5H, m), 6.82 (1H, d, J 8Hz), 6.76 (1H, d, J 2Hz), 6.64 (1H, dd, J 8Hz, J$^1$ 2Hz), 5.47 (1H, s), 5.17 (1H, s), 4.85-4.59 (2H, ABq, J 12Hz), 4.10-3.80 (2H, m), 3.61 (2H, m), 3.51 (2H, s), 3.46 (2H, q, J 7Hz), 3.16 and 2.82 (each 1H, d, J 17.5Hz), 1.14 (3H, t, J 7Hz). [F.A.B. (+ve ion, thioglycerol) MH+ 747].

## EXAMPLE 14

### Sodium (6R,7R)-7-[(R)-2-(4-Ethyl-2,3-dioxopiperazin-1-yl)carbonylamino-2-phenylacetamido]-7-formamido-3-(3,4,5-trihydroxybenzoyloxy)methylceph-3-em-4-carboxylate

Diphenylmethyl (6R,7R)-7-[(R)-2-(4-Ethyl-2,3-dioxopiperazin-1-yl)carbonylamino-2-phenylacetamido]-7-formamido-3-(3,4,5-triacetoxybenzoyloxy)methylceph-3-em-4-carboxylate (110mg, 0.1mmol) in $CH_2Cl_2$ (l0ml) was cooled in an ice bath and treated successively with anisole (320µl, 318mg, 2.95mmol) and TFA (230µl, 339mg, 2.97mmol). The reaction mixture was stirred at 0° for 20 minutes, then at room temperature for 2.5 hours and evaporated to dryness under reduced pressure. The residue was diluted with toluene and evaporated, then triturated with diethyl ether to give a yellow solid. The solid was suspended in water and the pH adjusted to 7 by addition of dilute aqueous $NaHCO_3$ solution. The resulting solution was treated with citrus acetylesterase preparation (1.2ml equivalent to approx. 4.8mg protein) and gently agitated for 1 hour. Further esterase (1.2ml) was added and the reaction allowed to proceed for 2.5 hours. The mixture was filtered through celite and freeze-dried. Chromatography on Diaion HP20SS eluting with acetone/water mixtures afforded the title compound as a freeze-dried solid (11.4mg); $\nu_{max}$ (KBr) 1775, 1710, and 1677cm$^{-1}$; $\delta_H$($D_2O$) (major rotamer) 8.10 (1H, s), 7.58-7.22 (5H, m), 7.06 (2H, m), 5.45 (1H, s), 5.25 (1H, s), 4.79 (2H), 4.05-3.80 (2H, m), 3.70-3.55 (2H, m), 3.54-3.30 (3H, m), 3.00 (1H, d, J 15.5Hz), 1.13 (3H, m). [F.A.B. (+ve ion, thioglycerol) MH+ 749].

## PREPARATION 18

### Diphenylmethyl (6R,7R)-7-[(R)-2-(4-ethyl-2,3-dioxopiperazin-1-yl)carbonylamino-2-phenylacetamido]-7-formamido-3-(hydroxymethyl)ceph-3-em-4-carboxylate

Sodium (6R,7R)-3-acetoxymethyl-7-[(R)-2-(4-ethyl-2,3-dioxopiperazin-1-yl)carbonylamino-2-phenylacetamido]-7-formamidoceph-3-em-4-carboxylate (1.0g, 1.566mmol) was dissolved in 0.1M aqueous $KH_2PO_4$ solution (15ml) and the pH adjusted to 7 with saturated aqueous $NaHCO_3$ solution. A preparation of citrus acetylesterase supported on sepharose was added and the sealed flask tumbled overnight. Analysis by HPLC then indicated complete deacetylation. The enzyme was removed by filtration and the solid washed with 0.1M aqueous $KH_2PO_4$ solution (10ml). The combined aqueous phases were layered with EtOAc (100ml) and the pH adjusted to 2 with 5M aqueous HCl solution. Sodium chloride was added and the phases separated. The aqueous phase was re-extracted with a further 9 × 100ml EtOAc. The combined extracts were dried over $MgSO_4$ and treated with diphenyldiazomethane (456mg, 2.35mmol). The volume of solution was reduced periodically over 2$^1/_2$ hours when tlc indicated esterification to be complete.

The mixture was evaporated under reduced pressure and the residue chromatographed on silica gel. Elution with EtOAc/methanol mixtures gave the title compound as a white solid (685mg); $\nu_{max}$ (KBr) 1785, 1715 and 1676cm$^{-1}$; $\delta_H$ ($CD_3COCD_3$) (major rotamer) 10.07 (1H, d, J 6.9Hz), 8.73 (1H, s), 8.52 (1H, s), 8.30 (1H, d, J 1.1Hz), 7.68-7.20 (15H, m), 6.90 (1H, s), 5.76 (1H, d, J 6.9Hz), 5.29 (1H, s), 4.50 (2H, m), 4.05 (2H, m), 3.60 (2H, m), 3.51 (2H, q, J 7.1Hz), 3.35 and 3.01 (each 1H, d, J 16.0Hz), 1.17 (3H, t, J 7.1Hz); [F.A.B. (+ve ion, thioglycerol) MH+ 741].

## PREPARATION 19

### 3,4-Diacetoxyphenyl Isocyanate

A solution of sodium azide (78mg, 1.2mmol) in water (2ml) was cooled in an ice bath and crude 3,4-diacetoxybenzoyl chloride (1.0mmol, prepared as described in Preparation No. 4(a)) in acetone (2ml) was added. After 20 minutes the cooling bath was removed and stirring continued for a further 1 hour. The mixture was extracted with benzene (3 × 10ml) and the combined extracts dried over $MgSO_4$. The resulting solution was heated for 3$^1/_2$ hours, allowed to stand overnight, then heated for a further 3 hours. Rearrangement of the acid azide to isocyanate was monitored by IR. The mixture was concentrated to approx. 10ml and $CH_2Cl_2$ (5ml) added. The resulting solution of crude 3,4-diacetoxyphenyl isocyanate was used with further purification.

PREPARATION 20

Diphenylmethyl (6R,7R)-3-[(3,4-Diacetoxyphenyl)carbamoyloxymethyl]-7-[(R)-2-(4-ethyl-2,3-dioxopiperazin-1-yl)carbonylamino-2-phenylacetamido]-7-formamidoceph-3-em-4-carboxylate

Diphenylmethyl (6R,7R)-7-[(R)-2-(4-ethyl-2,3-dioxopiperazin-1-yl)carbonylamino-2-phenylacetamido]-7-formamido-3-(hydroxymethyl)ceph-3-em-4-carboxylate (61mg, 0.082mmol) was added to the solution of crude 3,4-diacetoxyphenyl isocyanate from Preparation No. 21. The mixture was allowed to stand for 89 hours. The solvents were evaporated and the residue partitioned between water (10ml) and EtOAc (30ml). The organic phase was separated, washed with 0.5M aqueous HCl solution, saturated aqueous $NaHCO_3$ solution and brine then dried over $MgSO_4$.

The crude product was chromatographed on silica gel. Elution with EtOAc/methanol mixtures provided the title compound as an off-white solid (69mg); $\nu_{max}$ (KBr) 1775, 1717, 1684 and 1613cm$^{-1}$; $\delta_H$ ($CD_3COCD_3$) (major rotamer) 10.02 (1H, d, J 7.0Hz), 8.30 (1H, s), 7.7-7.1 (17H, m), 6.93 (2H, m), 5.73 (1H, d, J 7.0Hz), 5.30 (1H, s), 5.08 and 4.95 (each 1H, d, J 13.8Hz), 4.02 (2H, m), 3.70 (2H, m), 3.50 (2H, m), 3.32 and 3.12 (each 1H, d, J 16.8Hz), 2.28 and 2.26 (each 3H, s), 1.17 (3H, t, J 7.1Hz). [F.A.B. (+ve ion, thioglycerol) MH+ 976].

PREPARATION 21

Diphenylmethyl (6R,7R)-3-(2,3-Diacetoxycinnamoyloxy)methyl-7-[(R)-2-(4-ethyl-2,3-dioxopiperazin-1-yl)carbonylamino-2-phenylacetamido]-7-formamidoceph-3-em-4-carboxylate

Diphenylmethyl (6R,7R)-7[(R)-2-(4-ethyl-2,3-dioxopiperazin-1-yl)carbonylamino-2-phenylacetamido]-7-formamido-3-(hydroxymethyl)ceph-3-em-4-carboxylate (100mg, 0.135mmol) was dissolved in dry $CH_2Cl_2$ (5ml) and pyridine (27μl, 27mg, 0.337mmol, 2.5eq.) was added. Crude 2,3-diacetoxycinnamoyl chloride (prepared from the corresponding acid (89mg, 0.337mmol, 2.5eq.) by the procedure described in Preparation No. 4(a)) in dry $CH_2Cl_2$ (2ml) was added. The resulting solution was stirred for 19 h. After evaporation under reduced pressure the residue was partitioned between EtOAc and 0.5M aqueous HCl solution. The organic phase was separated, washed twice with 0.5M aqueous HCl solution, twice with saturated aqueous $NaHCO_3$ solution and with brine. After drying over $MgSO_4$ and evaporation under reduced pressure the residue was chromatographed on silica gel. Elution with EtOAc/methanol mixtures provided the title compound as an off-white solid (103mg); $\nu_{max}$ (KBr) 1779, 1716, 1690 and 1636cm$^{-1}$; $\delta_H$($CD_3COCD_3/D_2O$ exchanged) (major rotamer) 8.29 (1H, s), 7.85-7.25 (19H, m), 6.94 (1H, s), 6.60 (1H, d, J 16.1Hz), 5.70 (1H, s), 5.37 (1H, s), 5.14 and 4.97 (each 1H, d, J 13.8Hz), 4.08 (2H, m), 3.75 (2H, m), 3.52 (2H, q, J 7.2Hz), 3.35 and 3.10 (each 1H, d, J 17.1Hz), 2.40 and 2.32 (each 3H, s), 1.17 (3H, t, J 7.2Hz); [F.A.B. (+ve ion, 3-nitrobenzylalcohol-NaOAc) MNa+ 1009].

EXAMPLE 15

Sodium (6R,7R)-3-[(3,4-Dihydroxyphenyl)carbamoyloxymethyl]-7-[(R)-2-(4-ethyl-2,3-dioxopiperazin-1-yl)carbonylamino-2-phenylacetamido]-7-formamidoceph-3-em-4-carboxylate

Diphenylmethyl (6R,7R)-3-[(3,4-Diacetoxyphenyl)carbamoyloxymethyl]-7-[(R)-2-(4-ethyl-2,3-dioxopiperazin-1- yl)carbonylamino-2-phenylacetamido]-7-formamidoceph-3-em-4-carboxylate (55mg, 0.0564mmol) was dissolved in dry $CH_2Cl_2$ (3ml) and anisole (184μl, 183mg, 1.69mmol, 30eq.) was added. TFA (130μl, 192mg, 1.69mmol, 30eq.) was added and the solution allowed to stand for 2 hours. The mixture was then evaporated under reduced pressure and the residue triturated with ether providing a yellow solid. The solid was suspended in water and dissolution achieved by adjusting the pH to 7.2 with saturated aqueous $NaHCO_3$ solution. Citrus acetylesterase preparation (ex. Sigma) (0.5ml, equivalent to approx. 2mg of protein) was added and the deacetylation reaction monitored by HPLC. After 30 minutes the mixture was chromatographed on a column of Diaion HP20SS resin. Elution with acetone/water mixtures gave a series of fractions which were examined by HPLC.

The product-containing eluant was concentrated then lyophilised to give the title compound as a pink solid (17mg); $\nu_{max}$ (KBr) 1773, 1710, 1679 and 1611cm$^{-1}$; $\delta_H$($D_2O/CD_3COCD_3$) (major rotamer) 8.13 (1H, s), 7.55-7.35 (5H, m), 6.92 (1H, s), 6.82 (1H, d, J 6.8Hz), 6.69 (1H, dd, J 6.8 and 2Hz), 5.49 (1H, s), 5.27 (1H, s), 4.86 and 4.70 (each 1H, d, J 12.9Hz), 3.99 (2H, m), 3.67 (2H, m), 3.48 (2H, q, J 7.2Hz), 3.38 and 2.99 (each 1H, d, J 17.5Hz), 1.17 (3H, t, J 7.2Hz); [F.A.B. (+ve ion, thioglycerol) MH+ 748].

EXAMPLE 16

Sodium (6R,7R)-3-(2,3-Dihydroxycinnamoyloxy)methyl-7-[(R)-2-(4-ethyl-2,3-dioxopiperazin-1-yl)carbonylamino-2-phenylacetamido]-7-formamidoceph-3-em-4-carboxylate

Diphenylmethyl (6R,7R)-3-(2,3-Diacetoxycinnamoyloxy)methyl-7-[(R)-2-(4-ethyl-2,3-dioxopiperazin-1-yl)carbonylamino-2-phenylacetamido]-7-formamidoceph-3-em- 4-carboxylate (94mg, 0.0952mmol) was

dissolved in dry $CH_2Cl_2$ (8ml) and anisole (310µl, 309mg, 2.86mmol, 30eq.) was added. TFA (220µl, 326mg, 2.86mmol, 30eq.) was added and the solution allowed to stand for 1 1/2 hours. The mixture was evaporated under reduced pressure and then re-evaporated from toluene. The residue was trituated with ether. The resulting cream solid was dissolved in water (10ml) by adjusting the pH to 7.2 with saturated aqueous $NaHCO_3$ solution. Citrus acetylesterase preparation (ex. Sigma) (0.5ml, equivalent to approx. 2mg protein) was added; a further 0.25ml of enzyme preparation was added after 75 minutes.

The deacetylation was monitored by HPLC. The mixture was chromatographed on Diaion HP20SS resin eluting with acetone/water mixtures. The product-containing eluant was concentrated under reduced pressure and freeze-dried to give the title compound as a white solid (36mg); $\nu_{max}$ 1775, 1705, 1685 and 1625cm$^{-1}$; $\delta_H$ ($D_2O/CD_3COCD_3$) (major rotamer) 8.22 (1H, s), 8.00 (1H, d, J 16.1Hz), 7.65-7.35 (5H, m), 7.15 (1H, d, J 7.8Hz), 7.00 (1H, d, J 7.8Hz), 6.86 (1H, t, J 7.8Hz), 6.61 (1H, d, J 16.1Hz), 5.61 (1H, s), 5.36 (1H, s), 5.07 and 4.97 (each 1H, d, J 12.5Hz), 4.09 (2H, m), 3.77 (2H, m), 3.58 (2H, q, J 7.2Hz), 3.41 and 3.01 (each 1H, d, J 17.3Hz), 1.25 (3H, t, J 7.2Hz); [F.A.B. (+ve ion, thioglycerol) MH+ 759].

PREPARATION 22

2-Hydroxymethyl-5-(4-methoxybenzyloxy)-4-pyrone

Kojic acid (Fluka Chemie AG) (10.0g, 70.4mmol) and 4-methoxybenzyl chloride (10.5ml, 12.1g, 77.4mmol) were dissolved in dry DMF (50ml) and freshly powdered $K_2CO_3$ (19.5g, 140.7mmol) was added. The heterogeneous mixture was stirred for 2 hours then heated at 60°C for a further 3 hours. When cool the mixture was poured into water (300ml) and the aqueous phase extracted with $CH_2Cl_2$ (3 × 80ml). The combined organic phase was washed with water and brine, then dried over $MgSO_4$. Evaporation under reduced pressure gave a solid residue which was recrystallised from $CHCl_3$ to give the title compound as a cream-coloured crystalline solid (10.9g); $\nu_{max}$ (KBr) 3308, 1653, 1642, 1615 and 1588cm$^{-1}$; $\delta_H$($CDCl_3$) 7.51 (1H, s), 7.30 (2H, d, J 8.6Hz), 6.88 (2H, d, J 8.6Hz), 6.51 (1H, s), 4.96 (2H, s), 4.64 (2H, d, J 6.0Hz), 3.87 (1H, t, J 6.0Hz), and 3.80 (3H, s).

PREPARATION 23

5-(4-Methoxybenzyloxy)-4-pyrone-2-carboxylic acid

2-Hydroxymethyl-5-(4-methoxybenzyloxy)-4-pyrone (5.0g, 19.1mmol) was dissolved in acetone (250ml) with gentle warming and the resulting solution cooled in an ice bath. Jones reagent [prepared from concentrated sulphuric acid (4.7ml), water (15ml), and $CrO_3$ (5.37g)] was added dropwise over 5 minutes. The mixture was stirred for 1 hour, the cooling bath removed and stirring continued for a further 1 hour. Methanol (50ml) was then added. The green precipitate was removed by filtration through Kieselguhr and the filtrate evaporated to dryness under reduced pressure. The solid residue was triturated with methanol and the product collected by filtration and washed with acetone and ether.

The title compound was thus obtained as a pale green solid (1.4g); $\nu_{max}$ (KBr) 1740, 1617 and 1595cm$^{-1}$; $\delta_H$($CDCl_3/CD_3COCD_3$) 7.82 (1H, s), 7.35 (2H, m), 7.15 (1H, s), 6.90 (1H, s), 5.06 (2H, s), and 3.81 (3H, s).

PREPARATION 24

5-(4-Methoxybenzyloxy)-4-oxo-1,4-dihydropyridine-2-carboxylic acid

5-(4-Methoxybenzyloxy)-4-pyrone-2-carboxylic acid (1.05g, 3.80mmol) was suspended in .880 ammonia solution (30ml) and the mixture heated to reflux for 2 hours. A further 15ml of .880 ammonia solution was then added and heating continued for 1 hour. After cooling the solution was acidified to pH 1 using 5M aqueous HCl. The resulting off-white solid was obtained by filtration and washed with acetone and then ether. After drying in vacuo the title compound was obtained as a beige solid (813mg); $\nu_{max}$ (KBr) 1658 and 1590cm$^{-1}$; $\delta_H$ ($CD_3SOCD_3/D_2O$) 7.91 (1H, s), 7.39 (2H, d, J 8.6Hz), 7.22 (1H, s), 6.96 (2H, d, J 8.6Hz), 5.11 (2H, s), and 3.76 (3H, s).

PREPARATION 25

4-Methoxybenzyl 4,5-Di(4-methoxybenzyloxy)pyridine-2-carboxylate

5-(4-Methoxybenzyloxy)-4-oxo-1,4-dihydropyridine-2-carboxylic acid (700mg, 2.54mmol) was dissolved in DMF (30ml) and 4-methoxybenzyl chloride (1.72ml, 1.99g, 12.72mmol) was added. Freshly powdered $K_2CO_3$ (3.52g, 25.4mmol) was added and the heterogeneous mixture stirred and heated to 60°C for 18 hours. The mixture was then diluted with EtOAc (150ml) and water (150ml). The organic phase was separated and washed three times with water, once with 0.5M aqueous HCl, once with saturated aqueous sodium hydrogen carbonate then with water and brine and dried over $MgSO_4$. The solvent was removed under reduced pressure and the residue chromatographed on silica gel. Elution with ethyl acetate/hexane mixtures provided the title compound as a white crystalline solid (794mg); $\nu_{max}$ (KBr) 1702 and 1612cm$^{-1}$; $\delta_H$ ($CDCl_3$) 8.24 (1H, s), 7.72 (1H, s), 7.42-7.26 (6H, m), 6.92-6.85 (6H, m), 5.33 (2H, m), 5.17 (2H, s), 5.15 (2H, s), 3.81 (3H, s), 3.80 (3H, s), and 3.79 (3H, s).

PREPARATION 26

4,5-Di(4-Methoxybenzyloxy)pyridine-2-carboxylic acid

4-Methoxybenzyl 4,5-di(4-methoxybenzyloxy)pyridine-2-carboxylate (300mg, 0.588mmol) was dissolved in THF (15ml) and 2M aqueous potassium hydroxide (6ml) was added. The mixture was stirred and heated to reflux for 90 minutes. The THF was removed by evaporation under reduced pressure and the concentrated aqueous residue diluted with water and acidified to pH 1 with concentrated aqueous HCl.
The resulting white precipitate was separated by filtration and dried over $P_2O_5$ in vacuo to give the title compound (247mg); $\nu_{max}$ 1732 and 1612cm$^{-1}$; $\delta_H(CD_3SOCD_3)$ 8.42 (1H, s), 7.92 (1H, s), 7.39 (4H, m), 6.96 (4H, m), 5.37 (2H, s), 5.28 (2H, s), 3.76 (3H, s), and 3.75 (3H, s). [F.A.B. (+ve ion, Thioglycerol) MH+ 396].

PREPARATION 27

Diphenylmethyl (6R,7R)-3-[4,5-Di(4-methoxybenzyloxy)pyridin-2-yl]carbonyloxymethyl-7-[(R)-2-(4-ethyl-2,3-dioxopiperazin-1-yl)carbonylamino-2-phenylacetamido]-7-formamidoceph-3-em-4-carboxylate

4,5-Di(4-methoxybenzyloxy)pyridine-2-carboxylic acid (51mg, 0.130mmol) was suspended in dry $CH_2Cl_2$ and dry diisopropylethylamine (19mg, 0.130mmol) was added. The resulting solution was cooled to -60° C and methanesulphonyl chloride (15mg, 0.130mmol) was added. The flask was transferred to an ice bath and stirred for 30 minutes.
Diphenylmethyl (6R,7R)-7-[(R)-2-(4-ethyl-2,3-dioxopiperazin-1-yl)carbonylamino-2-phenylacetamido]-7-formamido-3-(hydroxymethyl)ceph-3-em-4-carboxylate (80mg, 0.108mmol) was dissolved in dry $CH_2Cl_2$ (2ml) and pyridine (10mg, 0.130mmol) was added. The above solution of mixed anhydride was then added and the mixture allowed to stand for 20 hours. The $CH_2Cl_2$ was evaporated under reduced pressure and the residue partitioned between EtOAc and 0.5M aqueous HCl. The organic phase was separated, washed twice with 0.5M aqueous HCl, three times with saturated aqueous sodium hydrogen carbonate, twice with brine and then dried over $MgSO_4$. Evaporation under reduced pressure gave a residue which was purified by column chromatography. Elution with ethyl acetate/methanol mitures gave the title compound as an off-white solid (38mg); $\nu_{max}$ (KBr) 1788, 1715 and 1689cm$^{-1}$; $\delta_H$ $(CD_3COCD_3)$ (major rotamer) 10.02 (1H, d, J 7Hz), 8.76 (1H, s), 8.54 (1H, s), 8.32 (1H, s), 8.29 (1H, s), 7.72 (1H, s), 7.65-7.20 (19H, m), 7.0-6.85 (4H, m), 5.74 (1H, d, J 7Hz), 5.26 (2H, s), 5.24 (2H, s), 5.25-5.10 (2H, m), 4.05 (2H, m), 3.79 (3H, s), 3.78 (3H, s), 3.66 (2H, m), 3.50 (2H, m), 3.37 and 3.15 each (1H, d, J 16.8Hz), and 1.17 (3H, t, J 7Hz). [F.A.B. (+ve ion, 3-Nitrobenzyl alcohol/sodium) MNa+ 1140].

EXAMPLE 17

(6R,7R)-7-[(R)-2-(4-Ethyl-2,3-dioxopiperazin-1-yl)carbonylamino-2-phenylacetamido]-7-formamido-3-(5-hydroxy-4-oxo-1,4-dihydropyridin-2-yl)carbonyloxymethylceph-3-em-4-carboxylic acid

Diphenylmethyl (6R,7R)-3-[4,5-di(4-methoxybenzyl)oxy]pyridin-2-ylcarbonyloxymethyl-7-[(R)-2-(4-ethyl-2,3-dioxopiperazin-1-yl)carbonylamino-2-phenylacetamido]-7-formamidoceph-3-em-4-carboxylate (110mg, 0.098mol) was dissolved in dry $CH_2Cl_2$ and anisole (638mg, 5.90mmol) was added. TFA (673mg, 5.90mmol) was added and the mixture allowed to stand for 50 minutes. The $CH_2Cl_2$ was removed under reduced pressure and the residue triturated with ether to give the title compound as a pale green solid; $\nu_{max}$ (KBr) 1785, 1710 and 1676cm$^{-1}$; [F.A.B. (+ve ion, Thioglycerol) MH+ 712].

PREPARATION 28

Diphenylmethyl (6R,7R)-3-[(E)-[4,5-di-(4-Methoxybenzyloxypyridin-2-yl]propenoyloxymethyl]-7-[(R)-2-(4-ethyl-2,3-dioxopiperazin-1-ylcarbonylamino)-2-phenylacetamido]-7-formamidoceph-3-em-4-carboxylate.

To a suspension of (E)-3-[4,5-di-(4-methoxybenzyloxy)pyridin-2-yl]propenoic acid (162mg), prepared as described in Preparation 43 below, in $CH_2Cl_2$ (6ml) was added diisopropylethylamine (49.6mg). The resulting solution was cooled to -70°C and methanesulphonyl chloride (29.8µl) was added with stirring. The solution was then stirred for 30 minutes in an acetone-ice bath. To this solution was added a solution of diphenylmethyl (6R,7R)-7-[(R)-2-(4-ethyl-2,3-dioxopiperazin-1-ylcarbonylamino)-2-phenylacetamido]-7-formamido-3-(hydroxymethyl)ceph-3-em-4-carboxylate (111mg) and pyridine (30.4mg) in $CH_2Cl_2$ (5ml). Stirring was continued at room temperature for 18 hours.
The solution was evaporated in vacuo and the residue partitioned between ethyl acetate and dilute $NaHCO_3$ solution. The organic layer was washed with water and brine, then dried ($MgSO_4$) and evaporated in vacuo to give a crude product which was chromatographed on silica eluting with ethyl acetate followed by 2% and 5% ethanol in ethyl acetate. The relevant fractions were combined and evaporated to give the title cephem (25mg); $\nu_{max}$ $(CH_2Cl_2)$ 1790, 1720 and 1695cm$^{-1}$; [F.A.B. (+ve ion, Thioglycerol), MH+, 1144].

EXAMPLE 18

(6R,7R)-7-[(R)-2-(4-Ethyl-2,3-dioxopiperazin-1-ylcarbonylamino)-2-phenylacetamido]-7-formamido-3-[(E)-3-(5-hydroxy-4-oxo-1,4-dihydropyridin-2-yl)prop-2-enoyloxymethyl]ceph-3-em-4-carboxylic acid

A solution of diphenylmethyl (6R,7R)-3-[(E)-[4,5-di(4-methoxybenzyloxy)pyridin-2-yl]propenoyloxymethyl]-7-[(R)-2-(4-ethyl-2,3-dioxopiperazin-1-ylcarbonylamino)-2-phenylacetamido]-7-formamidoceph-3-em-4-carboxylate (20mg) in $CH_2Cl_2$ (5ml) was stirred with anisole (113mg) and trifluoroacetic acid (120mg). After 1 hour, the solution was evaporated in vacuo, and the residue then twice diluted with $CH_2Cl_2$ (10ml) followed by further evaporation. Ether was added to the residue and the mixture triturated and then filtered to afford the title cephem acid as a solid (7mg); [F.A.B. (+ve ion, Thioglycerol), MH+ 738].

PREPARATION 29

Diphenylmethyl (6R,7R)-3-(3,4-Diacetoxybenzoyloxy)methyl-7-(2-phenylacetamido)ceph-3-em-4-carboxylate

Diphenylmethyl (6R,7R)-3-hydroxymethyl-7-(2-phenylacetamido)ceph-3-em-4-carboxylate (900mg; 1.75mmol) (J. Antibiotics, 1981, Vol. 34, p.1300) was suspended in dry dichloromethane (20ml) at room temperature under argon. Pyridine (346mg; 4.4mmol) was added followed by a solution of 3,4-diacetoxybenzoyl chloride [prepared from 3,4-diacetoxybenzoic acid (1.04g; 4.4mmol) as described in Preparation 4(a)] in dry dichloromethane (10ml). After 2.5h, the volatiles were removed under reduced pressure and the residue treated with ethyl acetate (50ml) and water (25ml). The phases were separated and the organic phase washed with water (25ml), saturated aqueous sodium hydrogen carbonate (25ml), water (25ml), saturated aqueous copper (II) sulphate (25ml), water (25ml), saturated brine (25ml) and dried over $MgSO_4$. Evaporation under reduced pressure gave a yellow foam, which was purified by chromatography through silica gel (<230 mesh ASTM), eluting with 25% ethyl acetate in cyclohexane, to give the title compound (513mg), m.p. 164-166°C (EtOAc-$Et_2O$); $\nu_{max}$ (KBr) 3317, 1780, 1723, 1710 and 1653cm$^{-1}$. $\delta_H$ $(CD_3)_2CO$] 2.32 (6H, s), 3.65 and 3.71 (2H, ABq, J 14Hz), 3.81 and 3.93 (2H, ABq, J 18Hz), 5.03 and 5.29 (2H, ABq, J 13Hz), 5.20 (1H, d, J 5Hz), 5.94 (1H, dd, J 5 and 9Hz), 6.97 (1H, s), 7.22-7.56 (15H, m), 7.81-7.94 (3H, m), 8.08 (1H, d, J 9Hz). [F.A.B. (+ve ion, thioglycerol) MH+ 735].

PREPARATION 30

Diphenylmethyl (6R,7R)-7-amino-3-(3,4-diacetoxybenzoyloxy)methylceph-3-em-4-carboxylate

Diphenylmethyl (6R,7R)-3-(3,4-Diacetoxybenzoyloxy)methyl-7-(2-phenylacetamido)ceph-3-em-4-carboxylate (2.39g; 3.26mmol) in dry dichloromethane (50ml), under argon, at 0-5°C was treated with N-methylmorpholine (723mg; 7.16mmol) then phosphorus pentachloride (813mg; 3.91mmol). Cooling was removed immediately and the mixture stirred until all the phosphorus pentachloride had dissolved. After an additional 10 minutes, methanol (20ml) was added and the mixture stirred for 30 minutes before being evaporated to dryness under reduced pressure. The residue was treated with ethyl acetate (50ml) and water (25ml) and the pH adjusted to 8.0 with saturated aqueous sodium hydrogen carbonate. The phases were separated, the aqueous phase further extracted with ethyl acetate (20ml), the extracts combined, washed with water (20ml), saturated brine (20ml), dried over $MgSO_4$ and evaporated to dryness under reduced pressure. The residue was triturated under diethyl ether and the title compound (1.46g) collected by filtration, washed with a little diethyl ether and dried in vacuo, m.p. 149°C; $\nu_{max}$ (KBr) 3411, 1.775 and 1717cm$^{-1}$; $\delta_H$ ($CDCl_3$) 2.34 (6H, s), 3.44 and 3.62 (2H, ABq, J 18Hz), 4.81 (1H, d, J 5Hz), 4.96 (1H, d, J 5Hz), 4.98 and 5.26 (2H, ABq, J 13Hz), 6.99 (1H, s), 7.22-7.47 (11H, m), 7.79-7.94 (2H, m). [F.A.B. (+ve ion, thoglycerol) MH+ 617].

PREPARATION 31

Diphenylmethyl (6R,7R)-7-[(Z)-2-(2-Aminothiazol-4-yl)-2-cyclopentyloxyiminoacetamido]-3-(3,4-diacetoxybenzoyloxy)methylceph-3-em-4-carboxylate

(Z)-2-(2-Aminothiazol-4-yl)-2-cyclopentyloxyiminoacetic acid (125mg; 0.49mmol) (British Pat. Applications Nos. 8 518 869 and 8 610 910) was dissolved in dry N,N-dimethylformamide (2ml) under argon and treated with ethyldiisopropylamine (139mg; 0.49mmol) and cooled to -50°C. Methanesulphonyl chloride (56mg; 0.49mmol) was added, the mixture allowed to warm to -20°C and stirred at -20°C for 20 min before cooling to -40°C. To this solution was added a solution of diphenylmethyl (6R,7R)-3-(3,4-diacetoxybenzoyloxy)methyl-7-aminoceph3-em-4-carboxylate (100mg; 0.16mmol) and pyridine (39mg; 0.44mmol) in dry dichloromethane (10ml) at 0-5°C under argon. The mixture was allowed to regain room temperature over 15 min. then stirred for 30 mins. After evaporation to dryness under reduced pressure the residue was treated with ethyl acetate (50ml). The ethyl acetate solution was washed with water (5 × 20ml), saturated brine (20ml), dried over $MgSO_4$ and evaporated to dryness under reduced pressure. The crude foam was purified by chromatography through silica (<230 mesh ASTM), eluting with 50% ethyl acetate in cyclohexane, giving the title compound (47mg) as a foam; $\nu_{max}$ ($CH_2Cl_2$) 3380, 1780 and 1725cm$^{-1}$; $\delta_H$≪ [$(CD_3)_2CO$] 1.0-2.0 (8H, m), 2.32 (6H, s), 3.77 and 3.91 (2H, ABq, J 18Hz), 4.71-4.80 (1H, m), 5.03 and 5.31 (2H, ABq, J 13Hz), 5.32 (1H, d, J 5Hz), 6.08 (1H, dd, J 5 and 9Hz), 6.74 (2H, br s), 6.83 (1H, s), 6.99 (1H, s), 7.24-7.60 (11H, m), 7.82-7.96 (2H, m), 8.45 (1H, d, J 9Hz).

PREPARATION 32

Diphenylmethyl (6R,7R)-7-[(R)-2-Azido-2-phenylacetamido]-3-(3,4-diacetoxybenzoyloxy)methylceph-3-em-4-carboxylate

(R)-2-Azido-2-phenylacetic acid (Dynamit Nobel) (390mg; 2.2mmol) was dissolved in dry dichloromethane (20ml) under argon at room temperature and the solution treated with oxalyl chloride (0.22ml) then dry N,N-dimethylformamide (0.2ml). After 1h, the volatiles were removed under reduced pressure and the residue redissolved in tetrahydrofuran (10ml). This solution was added to a solution containing diphenylmethyl (6R,7R)-7-amino-3-(3,4-diacetoxybenzoyloxy)methylceph- 3-em-4-carboxylate (450mg; 0.73mmol) and pyridine (174mg; 2.2mmol) in tetrahydrofuran (10ml). After 1h, the reaction mixture was diluted with ethyl acetate (50ml) and water (20ml). The phases were separated, the organic phase washed with water (20ml), 5M aqueous HCl solution (20ml), water (20ml), saturated, aqueous sodium hydrogen carbonate (20ml), water (20ml), saturated brine and dried over $MgSO_4$. Evaporation under reduced pressure followed by chromatography through silica (<230 mesh ASTM), eluting with ethyl acetate in cyclohexane (25-30% ethyl acetate gradient), gave the title compound (291mg) as a foam; $\nu_{max}(CH_2Cl_2)$, 2220, 1785, 1725 and 1700cm$^{-1}$. $\delta_H[(CD_3)|$ 2.32 (6H, s), 3.70 and 3.84 (2H, ABq, J 18Hz), 5.01 and 5.27 (2H, ABq, J 13Hz), 5.22 (1H, d, J 5Hz), 5.26 (1H, s), 5.98 (1H, dd, J 5 and 9Hz), 6.98 (1H, s), 7.25-7.60 (16H, m), 7.81-7.94 (2H, m), 8.85 (1H, d, J 9Hz). [F.A.B. (+ve ion, 1,4-dithioerythreitol/1,4-dithio-R,S- threitol) MNa+ 798].

PREPARATION 33

Diphenylmethyl (6R,7R)-7-[(Z)-2-(2-t-Butoxycarbonylpropan-2-yloxyimino)-2-(2-tritylaminothiazol-4-yl)acetamido]-3-(3,4-diacetoxybenzoyloxy)methylceph-3-em-4-carboxylate

Diphenylmethyl (6R,7R)-7-amino-3-(3,4-diacetoxybenzoyloxy)methylceph-3-em-4-carboxylate (270mg; 0.44mmol) and N,N'-dicyclohexylcarbodiimide (198mg; 0.96mmol) were dissolved in tetrahydrofuran (10ml) under argon at room temperature. This solution was treated with a solution of (Z)-2-(2-t-butoxycarbonylpropan-2-yloxyimino)-2-(2tritylaminothiazol-4-yl)acetic acid (500mg; 0.88mmol) in tetrahydrofuran (10ml) at 0-5°C. Cooling was removed immediately and the mixture stirred at room temperature for 1h. 1-Hydroxybenztriazole (118mg; 0.88mmol) was then added and the mixture stirred a further 2h. The precipitate was removed by filtration, washed with tetrahydrofuran (10ml) and the filtrate evaporated to dryness under reduced pressure. The residue was purified by chromatography through silica (<230 mesh ASTM), eluting with 25% ethyl acetate in cyclohexane, giving the title compound (140mg) as a foam; $\nu_{max}$ $(CH_2Cl_2)$ 3400, 1790, and 1730cm$^{-1}$; $\delta_H[(CD_3)_2CO]$ 1.43 (9H, s), 1.45 (6H, s), 2.33 (6H, s), 3.76 and 3.90 (2H, ABq, J 18Hz), 5.04 and 5.34 (2H, ABq, J 13Hz), 5.30 (1H, d, J 5Hz), 6.01 (1H, dd, J 5 and 9Hz), 6.83 (1H, s), 6.98 (1H, s), 7.21-7.96 (26H, m), 8.30 (1H, d, J 9Hz).

PREPARATION 34

Diphenylmethyl (6R,7R)-7-[2-(2-chloroacetylamino-4-thiazolyl)-2-([Z]-methoxyimino)acetamido]-3-(3,4-diacetoxycinnamoyloxy)methylceph-3-em-4-carboxylate

Diphenylmethyl (6R,7R)-7-[2-(2-chloroacetylamino-4-thiazolyl)-2-([Z]-methoxyimino)acetamido]-3-hydroxymethylceph-3-em-4-carboxylate (120mg; 18mmol) was suspended in dichloromethane (15ml) and treated with pyridine (40µl) and 3,4-diacetoxycinnamoyl chloride (140mg; 0.5mmol) overnight. The solution was evaporated to dryness and the product purified by chromatography on silica gel eluting with mixtures of ethyl acetate and hexane. Fractions containing the product were combined and evaporated to give the title compound as a white foam (110mg); $\nu_{max}$ $(CH_2Cl_2)$ 1790 (sh), 1770, 1715, 1685cm$^{-1}$; $\delta_H$ $(CDCl_3 + D_2O)$ 2.27 (6H, 2s), 3.63 (1H, d, J 5Hz), 3.81 (1H, d, J 18.5Hz), 4.14 (3H, s), 4.41 (2H, s), 4.90 (1H, d, J 13.3Hz), 5.11 (1H, d, J 13.3Hz), 5.28 (1H, d, J 4.9Hz), 6.01 (1H, d, J 4.9Hz), 6.50 (1H, d, J 16Hz), 6.93 (1H, s), 7.2-7.6 (14H, m), 7.63 (1H, d, J 16Hz).

EXAMPLE 19

Sodium (6R,7R)-3-(3,4-Dihydroxybenzoyloxy)methyl-7-(2-phenylacetamido)ceph-3-em-4-carboxylate

Diphenylmethyl (6R,7R)-3-(3,4-Diacetoxybenzoyloxy)methyl-7-(2-phenylacetamido)ceph-3-em-4-carboxylate (200mg; 0.27mmol) was dissolved in dry dichloromethane (10ml) under argon and cooled to 0-5°C. Anisole (883mg; 8.2mmol) then trifluoroacetic acid (932mg; 8.2mmol) were added and the cooling removed. After 1h, the volatiles were removed by evaporation under reduced pressure, the residue diluted with toluene then re-evaporated under reduced pressure. The residue was partitioned between ethyl acetate and saturated aqueous sodium hydrogen carbonate. The phases were separated and the aqueous phase freeze-dried. The resulting feeze-dried solid was redissolved in water (10ml), pH adjusted to 7.2 with saturated aqueous sodium hydrogen carbonate and the mixture treated with citrus acetyl esterase preparation (ex. Sigma) (0.5ml). After 2h of gentle agitation, the mixture was chromatographed through HP20SS, eluting with mixtures of

tetrahydrofuran in water (0-30% tetrahydrofuran gradient). The relevant fractions were combined, concentrated under reduced pressure and freeze-dried giving the title compound (96mg); $\nu_{max}$ (KBr) 3400, 3270, 1751, 1653, and 1603cm$^{-1}$; $\delta_H$ ($D_2O$) 3.34-3.72 (4H, m), 4.66-4.88 (2H, m), 5.05 (1H, d, J 4Hz), 5.61 (1H, d, J 4Hz), 6.78-6.89 (1H, m), 7.23-7.51 (7H, m). [F.A.B. (+ve ion, thioglycerol) MH+ 507].

EXAMPLE 20

Sodium (6R,7R)-7-[(Z)-2-(2-aminothiazol-4-yl)-2-cyclopentyloxyiminoacetamido]-3-(3,4-dihydroxybenzoyloxy)methylceph-3-em-4-carboxylate

Diphenylmethyl (6R,7R)-7-[(Z)-2-(2-Aminothiazol-4-yl)-2-cyclopentyloxyiminoacetamido]-3-(3,4-diacetoxybenzoyloxy)methylceph-3-em-4-carboxylate (47mg; 0.055mmol) was dissolved in dry dichloromethane (5ml) and cooled to 0-5°C. Anisole (177mg; 1.64mmol) then TFA (188mg; 1.65mmol) were added and the cooling removed immediately. After 3h, the volatiles were removed under reduced pressure, toluene added to the residue then re-evaporated under reduced pressure. The residue was treated with EtOAc (20ml) and saturated, aqueous sodium hydrogen carbonate (10ml). The phases were separated, the organic phase extracted with water (l0ml), the aqueous extracts combined, washed with diethyl ether (10ml) and freeze-dried. The resulting powder was redissolved in water (10ml) and stirred with citrus acetyl esterase preparation (ex. Sigma; 0.1ml) at pH 7.0-7.5 until h.p.l.c. analysis showed that no starting material remained. The crude reaction mixture was chromatographed through HP20SS resin, eluting with mixtures of tetrahydrofuran in water (0-30% tetrahydrofuran gradient). The relevant fractions were combined, concentrated under reduced pressure than freeze-dried to give the title compound (11mg); $\nu_{max}$ (KBr) 1758, 1653, and 1616cm$^{-1}$; $\delta_H$ ($D_2O$) 1.14-1.92 (8H, m), 3.47 and 3.71 (2H, ABq, J 18Hz), 4.85 and 5.11 (2H, ABq, J 14Hz), 5.20 (1H, d, J 5Hz), 5.79 (1H, d, J 5Hz), 6.78 (1H, d, J 8Hz), 6.96 (1H, s), 7.38-7.51 (2H, m). [F.A.B. (+ve ion, glycerol/$(CH_3)_2SO$) MH+ 626].

EXAMPLE 21

Sodium (6R,7R)-7-[(R)-2-azido-2-phenylacetamido]-3-(3,4-dihydroxybenzoyloxy)methylceph-3-em-4-carboxylate

Diphenylmethyl (6R,7R)-7-[(R)-2-Azido-2-phenylacetamido]-3-(3,4-diacetoxybenzoyloxy)methylceph-3-em-4-carboxylate (291mg; 0.37mmol) was dissolved in dry dichloromethane (10ml) under argon and cooled to 0-5°C. Anisole (1.22g; 11.3mmol) then TFA (1.28g; 11.23mmol) were added and the cooling removed. After 1h, the volatiles were removed under reduced pressure, dichloromethane (2 × 10ml) added and re-evaporated. The residue was treated with ethyl acetate (25ml) and water (25ml). The pH of the mixture was adjusted to 7.5 with saturated, aqueous sodium hydrogen carbonate and the phases separated. The aqueous phase was freeze-dried. The resultant powder was suspended in water (10ml), the pH adjusted to 7.2 with 1M aqueous HCl solution, the mixture stirred and treated with citrus acetyl esterase preparation (ex. Sigma; 0.3ml). After 0.5h, a further portion of enzyme (0.3ml) was added and the mixture stirred for a further 2h before h.p.l.c. analysis indicated that no starting material remained. This mixture was freeze-dried. The freeze-dried solid was resuspended in water (10ml) and the mixture chromatographed through HP20SS resin, eluting with mixtures of tetrahydrofuran in water (0-50% tetrahydrofuran gradient). The relevant fractions were combined, concentrated under reduced pressure and freeze-dried to give the title compound (12.5mg) as a white solid; $\nu_{max}$ (KBr) 2113, 1762, 1689 and 1600cm$^{-1}$; $\delta_H$ ($D_2O$) 3.29 and 3.59 (2H, ABq, J 18Hz), 4.80- 5.10 (3H, m), 5.37 (1H, s), 5.63 (1H, d, J 5Hz), 6.91 (1H, d, J 8Hz), 7.41-7.1 (8H, m). [F.A.B. (+ve ion, glycerol/$(CH_3)_2SO$) MH+ 548].

EXAMPLE 22

Sodium (6R,7R)-7-[(Z)-2-(2-Aminothiazol-4-yl)-2-(2-carboxypropan-2-yloxyimino)acetamido]-3-(3,4-dihydroxybenzoyloxy)methylceph-3-em-4-carboxylate

Diphenylmethyl (6R,7R)-7-[(Z-)2-(2-t-Butoxycarbonylpropan-2-yloxyimino)-2-(2-tritylaminothiazol-4-yl)acetamido]-3-(3,4-diacetoxybenzoyloxy)methylceph-3-em-4-carboxylate (140mg; 0.12mmol) was dissolved in TFA (5ml) under argon at 0-5°C and the mixture stirred at 0-5°C for 0.5h then at room temperature for 0.5h. The volatiles were removed under reduced pressure and toluene (10ml) added to the residue. The toluene was then removed under reduced pressure and the residue treated with diethyl ether (10ml) and saturated, aqueous sodium hydrogen carbonate (10ml). The phases were separated, the aqueous phase washed with diethyl ether (10ml) and freeze-dried. The resulting, freezedried solid was re-suspended in water (10ml), stirred and treated with citrus acetyl esterase preparation (ex. Sigma; 0.2ml) between pH 6.5-7.5 (glacial acetic acid was added when necessary). When h.p.l.c. analysis showed no starting material remaining, the reaction mixture was subjected to chromatography through HP20SS resin. Elution was with mixtures of tetrahydrofuran in water (0-50% tetrahydrofuran gradient). The relevant fractions were combined, concentrated under reduced pressure and freeze-dried to give the title compound (39.2mg); $\nu_{max}$ (KBr) 1763, 1666 sh and 1597cm$^{-1}$; $\delta_H$[$(CD_3)_2CO$ + $D_2O$] 1.47 and 1.49 (each 3H, 2s), 3.47 and 3.71 (2H, ABq, J 18Hz), 4.94 and 5.20

(2H, ABq, J 13Hz), 5.22 (1H, d, J 5Hz), 5.81 (1H, d, J 5Hz), 6.91 (1H, d, J 8Hz), 6.94 (1H, s), 7.43-7.53 (2H, m). [F.A.B. (+ve ion, thioglycerol) MH+ 666].

EXAMPLE 23

Sodium (6R,7R)-7-[2-(2-amino-4-thiazolyl)-2-([Z]-methoxyimino)acetamido]-3-(3,4-dihydroxycinnamoyloxy)methylceph-3-em-4-carboxylate

Diphenylmethyl ( 6R,7R)-7-[2-(2-chloroacetylamino-4-thiazolyl)-2-([Z]-methoxyimino)acetamido]-3-(3,4-diacetoxycinnamoyloxy)methylceph-3-em-4-carboxylate (100mg; 0.11mmol) was dissolved in dichloromethane (10ml) at room temperature and treated with anisole (370μl) and trifluoroacetic acid (260μl). The disappearance of starting material was monitored by t.l.c. on silica plates eluting with a 3:1 mixture of ethyl acetate:hexane. When the reaction was complete the solution was diluted with toluene (30ml) and evaporated to dryness under reduced pressure. The residue was triturated with a 1:1 mixture of ether: hexane (3 × 20ml), dried in vacuo for 30 minutes and then dissolved in water (20ml) by the addition of dilute aqueous sodium hydrogen carbonate to pH 7.2. The solution was treated with citrus acetyl esterase enzyme preparation (ex. Sigma, 1 ml) maintaining the pH at 7.0 over 2.5 hours and monitoring by h.p.l.c. until the deacetylation was complete. The mixture was filtered through celite and evaporated to dryness by azeotroping with toluene. The residue was dissolved in water (20ml) and treated with dilute aqueous sodium hydrogen carbonate to pH 7.0. Sodium N-methyl dithiocarbamate (30mg, 2 equiv.) was then added. The reaction was monitored by h.p.l.c. The aqueous solution was washed with ethyl acetate and the product purified on HP20SS resin eluting with water and mixtures of water and acetone. Fractions containing product were combined, concentrated and freeze-dried to give the title compound as white amorphorus solid (11mg); $\nu_{max}$ (KBr) 1763, 1670, 1617, 1527cm$^{-1}$; $\delta_H$ [$D_2O$ + $CD_3COCD_3$] 3.42 (1H, d, J 17.7Hz), 3.69 (1H, d, J 17.7Hz), 3.96 (3H, s), 4.85 (1H, d), 5.03 (1H, d, J 12.4Hz), 5.22 (1H, s (br)), 5.80 (1H, s (br)), 6.30 (1H, d, J 16 Hz), 6.97 (1H, s), 6.8-7.15 (3H, m), 7.58 (1H, d, J 16Hz). [F.A.B. (+ve ion, thioglycerol) MH+ 598].

PREPARATION 35

Diphenylmethyl (6R,7R)-3-[(3,4-Diacetoxyphenyl)carbamoyloxy]methyl-7-[2-(2-furyl)-2-(Z)-methoxyiminoacetamido]ceph-3-em-4-carboxylate

Sodium (6R,7R) 3-acetoxymethyl-7-[2-(2-furyl)-2-(Z)-methoxyiminoacetamido]ceph-3-em-4-carboxylate (222mg, 0.5mmol) (British Patent No. 1 399 086) was dissolved in 0.05M aqueous $KH_2PO_4$ solution (15ml) and the pH of the solution adjusted to 7.2 by addition of saturated aqueous $NaHCO_3$ solution. Citrus acetylesterase preparation (ex. Sigma) (0.75ml) was added and the mixture allowed to stand for 2 hours. The aqueous phase was then layered with EtOAc and the mixture stirred vigorously while adjusting the pH to 2 by addition of 5M aqueous HCl solution. The mixture was shaken and the phases separated. The aqueous phase was re-extracted a further three times with EtOAc. The combined extracts were dried over $MgSO_4$ and then treated with diphenyldiazomethane (107mg, 0.55mmol).

The resulting solution was concentrated to one third volume and allowed to stand for 4 hours. Further concentration under reduced pressure was followed by dilution with $CH_2Cl_2$ (50ml), re-concentration and redilution with $CH_2Cl_2$ to a final volume of 20ml. 3,4-Diacetoxyphenyl isocyanate (5mmol) (prepared as described in Preparation 19) in benzene (10ml) was added and the solution allowed to stand for 44 hours. The solvents were evaporated under reduced presure and the residue partitioned between EtOAc and 0.5M aqueous HCl solution. The organic phase was separated and washed twice with 0.5M aqueous HCl solution, three times with saturated aqueous $NaHCO_3$ solution and with brine then dried over $MgSO_4$. The solvent was evaporated under reduced pressure and the residue chromatographed on silica gel. Elution with EtOAc/hexane mixtures gave the title compound as a cream-coloured solid (187mg); $\nu_{max}$ 1773, 1733, 1687 and 1611cm$^{-1}$; $\delta_H$ ($CDCl_3$) 7.52-7.05 (15H, m), 6.98 (1H, s), 6.92 (1H, d, J 3.5Hz), 6.48 (1H, dd, J 3.5 and 1.8Hz), 6.00 (1H, dd, J 9.0 and 4.9Hz), 5.09 (1H, d, J 4.9Hz), 5.15 and 4.84 (each 1H, d, J 13.5Hz), 4.09 (3H, s), 3.63 and 3.45 (each 1H, d, J 18.7Hz), 2.27 (6H, 2s). [F.A.B. (+ve ion, 3-Nitrobenzylalcohol/sodium) MNa+ 805].

PREPARATION 36

4-Methoxybenzyl (6R,7R)-7-Amino-3-(3,4-diacetoxybenzoyloxy)methylceph-3-em-4-carboxylate

4-Methoxybenzyl (6R,7R)-7-amino-3-chloromethylceph-3-em-4-carboxylate (409mg, 1.11mmol) (available from Otsuka Chemical Co.) was dissolved in dry DMF (l0ml) and NaI (165mg, 1.11mmol) was added. The solution was stirred and the reaction vessel protected from the light. Sodium 3,4-diacetoxybenzoate (577mg, 2.22mmol) was added and the mixture stirred vigorously for 1$^1/_2$ hours. The mixture was then partitioned between EtOAc and saturated aqueous $NaHCO_3$ solution. The organic phase was separated, washed twice with saturated aqueous $NaHCO_3$ solution once with water and once with brine then dried over $MgSO_4$. Evaporation of the solvent under reduced pressure gave a solid residue which was purified by silica gel chromatography. Elution with EtOAc/hexane mixtures gave the product as an oil which solidified upon trituration with ether/hexane to provide the title compound as a pale yellow solid (240mg); $\nu_{max}$ 3425, 3346,

1763 and 1718cm$^{-1}$; $\delta_H(CDCl_3)$ 7.91 (1H, dd, J 8.6 and 2Hz), 7.83 (1H, d, J 2Hz), 7.38-7.27 (3H, m), 6.87 (2H, d, J 8.7Hz), 5.35 and 5.02 (each 1H, d, J 13.3Hz), 5.25 (2H, s), 4.94 (1H, d, J 5.1Hz), 4.77 (1H, d, J 5.1Hz), 3.80 (3H, s), 3.61 and 3.43 (each 1H, d, J 18.3Hz), 2.34 (6H, s). [F.A.B. (+ve ion, 3-Nitrobenzylalcohol/ sodium) MNa+ 593].

PREPARATION 37

4-Methoxybenzyl (6R,7R)-7-[3-(2-Chloro-6-fluorophenyl)-5-methylisoxazol-4-yl]carbonylamino-3-(3,4-diacetoxybenzoyloxy)methylceph-3-em-4-carboxylate

4-Methoxybenzyl ( 6R,7R)-7-amino-3-(3,4-diacetoxybenzoyloxy)methylceph-3-em-4-carboxylate (100mg, 0.18mmol) was dissolved in dry $CH_2Cl_2$ (5ml) and pyridine (29mg, 0.36mmol) was added. 3-(2-Chloro-6-fluorophenyl)-5-methylisoxazole-4-carbonyl chloride (98mg, 0.36mmol) (see U.K. Patent No. 978,299) was added. After 2 hours the solvent was evaporated under reduced pressure and the residue partitioned between EtOAc and 0.5M aqueous HCl solution. The organic phase was separated, washed twice with 0.5M aqueous HCl solution, three times with saturated aqueous $NaHCO_3$ solution, once with brine and then dried over $MgSO_4$. Evaporation of the solvent under reduced pressure gave a solid residue which was purified by column chromatography. Elution with EtOAc/hexane mixtures gave the title compound as an off-white solid (114mg); $\nu_{max}$ 1777, 1724 and 1677cm$^{-1}$; $\delta_H$ ($CDCl_3$) 7.91 (1H, dd, J 8.5 and 2Hz), 7.82 (1H, d, J 2Hz), 7.52-7.14 (6H, m), 6.88 (2H, m), 5.96-5.84 (2H, m), 5.38 and 5.07 (each 1H, d, J 13.6Hz), 5.24 and 5.17 (each 1H, d, J 11.8Hz), 4.90 (1H, d, J 4.7Hz), 3.80 (3H, s), 3.53 and 3.31 (each 1H, d, J 18.3Hz), 2.81 (3H, s), 2.33 (6H, s). [F.A.B. (+ve ion, Thioglycerol/3-nitrobenzylalcohol/sodium) MNa+ 830].

PREPARATION 38

Diphenylmethyl (6R,7R)-3-(3,4-Diacetoxybenzoyloxy)methyl-7-(2-(Z)-methoxyimino-2-phenylacetamido)ceph-3-em-4-carboxylate

Sodium (6R,7R)-3-acetoxymethyl-7-(2-(Z)-methoxyimino-2-phenylacetamido)ceph-3-em-4-carboxylate (290mg, 0.64mmol) (British Patent No. 1 399 086) was dissolved in 0.1M aqueous $KH_2PO_4$ solution and the pH adjusted to 7 by addition of saturated aqueous $NaHCO_3$ solution. Citrus acetylesterase preparation (1.0ml, equivalent to approx. 4mg of protein) was added and the mixture gently agitated for 1 hour. A further quantity of citrus acetylesterase (0.5ml, equivalent to approx. 2mg of protein) was added and the reaction allowed to proceed for another 1.5 hours. The mixture was layered with EtOAc (30ml) and acidified to pH 2.0 with 1M aqueous HCl solution. The organic phase was separated and the aqueous phase extracted with EtOAc (3 x 30ml). The combined organic extracts were washed with brine, dried over $MgSO_4$ and concentrated to 15ml under reduced pressure to provide a solution of (6R,7R)-3-hydroxymethyl-7-(2-(z)-methoxyimino-2-phenylacetamido)ceph-3-em-4-carboxylic acid. The solution was treated with diphenyldiazomethane (123mg, 0.64mmol) and stirred for 2 hours. The mixture was concentrated to 5ml under reduced pressure, then diluted with $CH_2Cl_2$ (25ml) and concentrated to 5ml twice. Dilution with $CH_2Cl_2$ (5ml) gave a solution of diphenylmethyl (6R,7R)-3-hydroxymethyl-7-(2-(Z)-methoxyimino-2-phenylacetamido)ceph-3-em-4-carboxylate. The solution was treated with pyridine (0.13ml, 1.6mmol) and a solution of 3,4-diacetoxybenzoyl chloride (1.6mmol, prepared as described in Preparation 4(a)) in $CH_2Cl_2$ (10ml). The mixture was stirred at room temperature overnight and diluted with EtOAc (30ml). The solution was washed with 0.5M aqueous HCl solution (2 × 30ml), saturated aqueous $NaHCO_3$ solution (2 × 30ml), brine (30ml), and dried over $MgSO_4$. The solvent was removed under reduced pressure and the residue chromatographed on silica gel. Elution with mixtures of ethyl acetate/hexane provided the title compound (142mg); $\nu_{max}$ ($CH_2Cl_2$) 3390, 1780, 1725 and 1685cm$^{-1}$; $\delta_H(CD_3COCD_3)$ 8.68 (1H, d, J 8.5Hz), 8.20-7.23 (18H, m), 6.98 (1H, s), 6.14 (1H, dd, J 8.5 and 5Hz), 5.35 (1H, d, J 5Hz), 5.30 and 5.03 (each 1H, d, J 13Hz), 3.97 (3H, s), 3.99 and 3.77 (each 1H, d, J 18Hz), 2.32 (3H, s), 2.31 (3H, s). [F.A.B. (+ve ion, 3-Nitrobenzylalcohol/NaOAc) MNa+ 800].

PREPARATION 39

Diphenylmethyl (6R,7R)-3-(3,4-Diacetoxybenzoyloxy)methyl-7-(2-(E)-methoxyimino-2-(2-thienyl)acetamido]ceph-3-em-4-carboxylate

Sodium (6R,7R)-3-acetoxymethyl-7-[2-(E)-methoxyimino-2-(2-thienyl)acetamido]ceph-3-em-4-carboxylate (280mg, 0.61mmol) (British Patent No. 1 399 086) was dissolved in 0.1M aqueous $KH_2PO_4$ and the pH adjusted to 7 by addition of saturated aqueous $NaHCO_3$ solution. Citrus acetylesterase preparation (1.0ml, equivalent to approx. 4mg of protein) was added and the mixture gently agitated for 3 hours with addition of further citrus acetylesterase (0.5ml) after 2 hours. The mixture was layered with EtOAc (30ml) and acidified to pH 2.0 with 1M aqueous HCl solution. The layers were separated and the aqueous phase extracted with EtOAc (4 × 30ml). The combined organic extracts were washed with brine (30ml) and dried over $MgSO_4$. The solution was concentrated to 50ml under reduced pressure to give a solution of (6R,7R)-3-hydroxymethyl-7-[2-(E)-methoxyimino-2-(2-thienyl)acetamido)ceph-3-em- 4-carboxylic acid. The solution was treated with diphenyldiazomethane (124mg, 0.64mmol), stirred for 3 hours and then evaporated to 5ml. The mixture was diluted with $CH_2Cl_2$ (25ml) and evaporated to low volume (5ml). This procedure was repeated and then $CH_2Cl_2$ (25ml) added to give a solution of diphenylmethyl (6R,7R)-3-hydroxymethyl-7-[2-(E)-methoxyimino-2-(2-thienyl)acetamido]ceph-3-em-4-carboxylate. The solution was cooled to 0° and treated with pyridine (0.13ml, 1.6mmol), followed by a solution of 3,4- diacetoxybenzoyl chloride (1.6mmol, prepared as described in Preparation 4(a)) in $CH_2Cl_2$ (5ml). The reaction was stirred at room temperature overnight and diluted with EtOAc (30ml). The solution was washed with 1M aqueous HCl solution (2 × 40ml), saturated aqueous $NaHCO_3$ (2 × 40ml), saturated brine (40ml) and dried over $MgSO_4$. The solvent was removed under reduced pressure and the residue chromatographed on silica gel. Elution with ethyl acetate/hexane mixtures gave the title compound (180mg); $\nu_{max}$ ($CH_2Cl_2$) 3050, 1775, 1725 and 1695cm$^{-1}$; $\delta_H$ ($CD_3COCD_3$) 8.72 (1H, d, J 8.5Hz), 8.20-7.20 (12H, m), 7.15-7.06 (1H, m), 6.98 (1H, s), 6.10 (1H, dd, J 8.5 and 5Hz), 5.35 (1H, d, J 5Hz), 5.32 and 5.04 (each 1H, d, J 13Hz), 3.94 (3H, s), 3.93 and 3.77 (each 1H, d, J 18Hz), 2.32 (3H, s), 2.31 (3H, s). [F.A.B. (+ve ion, 3-Nitrobenzylalcohol/NaOAc) MNa+ 806].

EXAMPLE 24

Sodium (6R,7R)-3-[(3,4-Dihydroxyphenyl)carbamoyloxy]methyl-7-[2-(2-furyl)-2-(Z)-methoxyiminoacetamido]ceph-3-em-4-carboxylate

Diphenylmethyl (6R,7R)-3-[(3,4-diacetoxyphenyl)carbamoyloxy]methyl-7-[2-(2-furyl)-2-(Z)-methoxyiminoacetamido]ceph-3-em-4-carboxylate (150mg, 0.19mmol) was dissolved in dry $CH_2Cl_2$ (10ml) and anisole (622mg, 5.75mmol) was added. The solution was cooled in an acetone/ice bath and TFA (655mg, 5.75mmol) was added. After 1$^1/_2$ hours the volatiles were evaporated under reduced pressure and the residue treated with toluene and re-evaporated. Water (20ml) was added and the pH adjusted to 7 with saturated aqueous $NaHCO_3$ solution. The resulting solution was extracted twice with ether. The aqueous phase was treated with citrus acetylesterase preparation (ex. Sigma) (0.5ml). After 2 hours the product was purified by HP20SS chromatography. Elution with acetone/water mixtures gave a series of fractions which were analysed by HPLC. The product-containing eluant was concentrated and freeze-dried to give the title compound as a pink solid (37mg); $\nu_{max}$ (KBr) 1765, 1710, 1680 and 1605cm$^{-1}$; $\delta_H$($D_2O$) 7.65 (1H, s), 6.95 (5H, m), 5.78 (1H, d, J 4.5Hz), 5.17 (1H, d, J 4.5Hz), 4.95 and 4.70 (each 1H, d, J 12.5Hz), 3.96 (3H, s), 3.65 and 3.40 (each 1H, d J 17.8Hz).

Example 25

Sodium (6R,7R)-7-[3-(2-Chloro-6-fluorophenyl)-5-methylisoxazol-4-yl]carbonylamino-3-(3,4-dihydroxybenzoyloxy)methylceph-3-em-4-carboxylate

4-Methoxybenzyl (6R,7R)-7-[3-(2-chloro-6-fluorophenyl)-5-methylisoxazol-4-yl]carbonylamino-3-(3,4-diacetoxybenzoyloxy)methylceph-3-em-4-carboxylate (100mg, 0.126mmol) was dissolved in dry $CH_2Cl_2$ (5ml) and anisole (408mg, 3.77mmol) was added. The solution was stirred under argon and cooled in an acetone/ice bath. TFA (430mg, 3.77mmol) was added and the mixture stirred for 3 hours. Volatile components were evaporated under reduced pressure and the residue triturated with ether/hexane. The resulting solid was suspended in water (20ml) and the pH adjusted to 7.2 with saturated aqueous $NaHCO_3$ solution. Citrus acetylesterase preparation (ex. Sigma) (0.5ml) was added and the mixture allowed to stand for 170 minutes. After filtration through Kieselguhr the resulting aqueous solution was freeze-dried.

The product was dissolved in water (15ml) and the pH adjusted to 7 by addition of aqueous $NaHCO_3$ solution. Chromatography on HP20SS resin was achieved using acetone/water mixtures as eluant. Product-containing fractions were combined and freeze-dried to give the title compound as a white amorphous solid (25mg); $\nu_{max}$(KBr) 1763, 1674 and 1609cm$^{-1}$; $\delta_H$ ($CD_3COCD_3/D_2O$) 7.65-7.4 (4H, m), 7.32 (1H, t, J 8.8Hz), 6.96 (1H, d, J 8.9Hz), 5.78 (1H, d, J 4.7Hz), 5.14 (1H, d, J 4.7Hz), 5.22 and 5.00 (each 1H, d, J 12.7Hz), 3.68 and 3.41 (each 1H, d, J 17.7Hz), 2.77 (3H, s). [F.A.B. (+ve ion, DMSO/glycerol/thioglycerol) MH+ 626].

EXAMPLE 26

Sodium (6R,7R)-3-(3,4-Dihydroxybenzoyloxy)methyl-7-(2-(Z)-methoxyimino-2-phenylacetamido)ceph-3-em-4-carboxylate

Diphenylmethyl (6R,7R)-3-(3,4-diacetoxybenzoyloxy)methyl-7-(2-(Z)-methoxyimino-2-phenylacetamido)ceph-3-em-4-carboxylate (260mg, 0.33mmol) in $CH_2Cl_2$ (5ml) was treated with anisole (1.07ml, 1.06g, 9.9mmol), and TFA (0.76ml, 1.12g, 9.9mmol). The reaction was stirred at room temperature for 1 hour, then the TFA and $CH_2Cl_2$ were removed by evaporation under reduced pressure. The residue was suspended in water (15ml) and dilute aqueous $NaHCO_3$ solution was added to give a solution at pH 7.0. The solution was washed with $Et_2O$ (3 × 15ml) and any residual traces of $Et_2O$ removed by evaporation under reduced pressure. Citrus acetylesterase preparation (0.5ml, equivalent to approx. 2mg of protein) was added to the solution, which was gently agitated for 1.5 hours. Chromatography on Diaion HP20SS resin eluting with water/acetone mixtures

and lyophilisation gave the title compound as an off-white solid (15.7mg); $\nu_{max}$ (KBr) 1764, 1675 and 1602cm$^{-1}$; $\delta_H$ ($D_2O$) 7.50-7.30 (7H, m), 6.91 (1H, d), 5.84 (1H, d, J 4.5Hz), 5.21 (1H, d, J 4.5Hz), 5.10 and 4.87 (each 1H, d, J 13Hz), 4.00 (3H, s), 3.71 and 3.44 (each 1H, d, J 18Hz). [F.A.B. (+ve ion, Thioglycerol) MH+ 550].

EXAMPLE 27

Sodium (6R,7R)-3-(3,4-Dihydroxybenzoyloxy)methyl-7-[2-(E)-methoxyimino-2-(2-thienyl)acetamido]ceph-3-em-4-carboxylate

Benzhydryl (6R,7R)-3-(3,4-diacetoxybenzoyloxy)methyl-7-[2-(E)-methoxyimino-2-(2-thienyl)acetamido-]ceph-3-em-4- carboxylate (180mg, 0.23mmol) in $CH_2Cl_2$ (10ml) was treated with anisole (0.74ml, 0.74g, 6.9mmol) and TFA (0.70ml, 1.04g, 6.9mmol). The reaction mixture was stirred for 1.5h, then the TFA and $CH_2Cl_2$ were removed by evaporation under reduced pressure. The residue was suspended in water (15ml) and dilute aqueous $NaHCO_3$ solution was added to give a solution at pH 7.0. The solution was washed with $Et_2O$ (3 × 15ml) and residual traces of $Et_2O$ removed by evaporation under reduced pressure. Citrus acetylesterase preparation (0.5ml, equivalent to approx. 2mg of protein) was added to the mixture, which was then gently agitated for 2 hours. Chromatography on Diaion HP20SS resin eluting with water/acetone mixtures, followed by lyophilisation gave the title compound as an off white solid (18mg); $\nu_{max}$(KBr) 1763, 1676 and 1603cm$^{-1}$; $\delta_H$ ($D_2O$) 7.59 (1H, d, J 5Hz), 7.55-7.43 (2H, m), 7.36 (1H, d, J 3.5Hz), 7.15 (1H, dd, J 5 and 4Hz), 6.93 (1H, d, J 8Hz), 5.84 (1H, d, J 4.5Hz), 5.23 (1H, d, J 4.5Hz), 5.12 and 4.89 (each 1H, d, J 12.5Hz), 3.97 (3H, s), 3.74 and 3.47 (each 1H, d, J 18Hz). [F.A.B. (+ve ion, Thioglycerol) MH+ 556].

PREPARATION 40

2-Hydroxymethyl-5-(4-methoxybenzyloxy)-4-oxo-1,4-dihydropyridine

A suspension of 2-hydroxymethyl-5-(4-methoxybenzyloxy)-4-pyrone (3.28g) in concentrated aqueous ammonia solution (75ml) was stirred and heated under reflux. The starting material gradually dissolved to briefly give a clear solution, after which the product began to precipitate out. Further quantities of ammonia solution (10ml) were added after 1h and 2h. After 3h the mixture was allowed to cool and then filtered. The resulting solid was washed with acetone followed by ether and dried to afford the title pyridone (2.87g); $\nu_{max}$ (KBr) 1612, 1529 and 1489cm$^{-1}$; $\delta_H$ ($CD_3SOCD_3$) 3.70 (3H, s), 4.32 (2H, s), 4.93 (2H, s), 6.30 (1H, s), 6.93 and 7.36 (each 2H, d, J 8Hz), and 7.46 (1H, s).

PREPARATION 41

Methyl (E)-3-[5-(4-Methoxybenzyloxy)-4-oxo-1,4-dihydropyridin-2-yl]propenoate

(a) 2-Hydroxymethyl-5-(4-methoxybenzyloxy)-4-oxo-1, 4-dihydropyridine (2.20g) was suspended in DMF (100ml) and the mixture warmed gently until a clear solution was obtained. Active manganese dioxide (22g) was added, and the mixture was stirred at ambient temperature for 6h, before filtering over Celite. The filtrate was evaporated in vacuo to yield crude 5-(4-methoxybenzyloxy)-4-oxo-1,4-dihydropyridine-2-carboxaldehyde as a solid.

(b) The aldehyde was suspended in $CH_2Cl_2$ (120ml) and to the stirred suspension was added carbomethoxymethylenetriphenylphosphorane (2.45g). An immediate clear solution was obtained, from which a solid began to precipitate on continued stirring. After 2h the solid was obtained by filtration, and the filtrate was evaporated in vacuo. $CH_2Cl_2$ was added to the residue, and a second crop of solid obtained by filtration. The combined solids (1.244g) consisted of the title ester; $\nu_{max}$ (KBr) 1727, 1657, 1609 and 1533cm$^{-1}$; $\delta_H$($CD_3SOCD_3$) 3.72 (3H, s), 3.75 (3H, s), 5.11 (2H, s), 6.68 (1H, d, J 16Hz), 6.94 (2H, d, J 8.5Hz), 7.01 (1H, br s), 7.39 (2H, d, J 8.5Hz), 7.48 (1H, d, J 16Hz), 8.05 (1H, br s).

PREPARATION 42

Methyl (E)-3-[4,5-di(4-Methoxybenzyloxy)pyridin-2-yl]propenoate

A mixture of methyl (E)-3-(4-methoxybenzyloxy)-4-oxo-1,4-dihydropyridin-2-yl]propenoate (1.26g), 4-methoxybenzyl chloride (1.35ml) and potassium carbonate (2.76g) was heated at 60-65°C in DMF (40ml) with stirring. After 18h the mixture was allowed to cool and then partitioned between ethyl acetate (200ml) and water (100ml). The organic solution was washed with water (5 × 100ml) and brine (50ml) and then dried ($MgSO_4$) and evaporated in vacuo. The crude product was chromatographed on silica eluting successively with 40%, 50% and 60% ethyl acetate in hexane. Fractions containing the title compound were combined and evaporated to afford a solid (1.67g); $\nu_{max}$ ($CH_2Cl_2$) 1715, 1645, 1615, 1580 and 1515cm$^{-1}$; $\delta_H$ ($CDCl_3$) 3.70, 3.80 and 3.82 (each 3H, s), 5.15 (4H, s) 6.70 and 7.56 (each 1H, J 16Hz), 6.91 and 7.37 (each 4H, m), 6.99 (1H, s) and 8.20 (1H, s).

PREPARATION 43

(E)-3-[4,5-Di(4-methoxybenzyloxy)pyridin-2-yl]propenoic acid

A mixture of methyl (E)-3-[4,5-di(4-methoxybenzyloxy) pyridin-2-yl]propenoic acid (1.63g), 2M aqueous KOH solution (36ml) and THF (90ml) were stirred and heated under reflux. After 2.5h the solution was cooled and concentratedin vacuo to remove THF. The aqueous solution was acidified to pH 1 with 5M aqueous HCl and the resultant solid obtained by filtration, washing it with acetone and ether. The washings were evaporated in vacuo and the residue triturated with ether to give a second crop of solid, again isolated by filtration. The combined solids were dried to give the title acid (1.445g); $\nu_{max}$ (KBr) 1705, 1611, 1586 and 1545cm$^{-1}$; $\delta_H$ ($CD_3SOCD_3$) 3.75 and 3.76 (each 3H, s), 5.19 and 5.27 (each 2H, s), 6.95 (5H, m) 7.36 and 7.43 (each 2H, d, J 8.6Hz), 7.60 (1H, d, J 15.9Hz), 7.84 (1H, s), 8.38 (1H, s) [F.A.B. (+ve ion, 3-Nitrobenzyl alcohol) MH+, 422].

PREPARATION 44

Sodium (E)-3-[4,5-Di(4-methoxybenzyloxy)pyridin-2-yl]propenoate

(E)-3-[4,5-di(4-methoxybenzyloxy)pyridin-2-yl]propenoic acid (631.5mg) was dissolved in DMF (5ml) with warming. A solution of 1.47M sodium 2-ethylhexanoate in methyl isobutyl ketone (1.02ml) was added with stirring, followed by ether (ca. 35ml) until a heavy white precipitate was observed. The mixture was filtered and the solid washed with ether and immediately dried in vacuo to afford the title sodium salt (561mg); $\nu_{max}$ (KBr) 1645, 1611, 1585 and 1515cm$^{-1}$.

PREPARATION 45

4-Methoxybenzyl (6R,7R)-7-Amino-3-[(E)-[4,5-di(4-methoxybenzyloxy)pyridin-2-yl]propenoyloxymethyl]ceph-3-em-4-carboxylate

The hydrochloride salt of p-methoxybenzyl (6R,7R)-7-amino-3-chloroceph-3-em-4-carboxylate (112mg) was partitioned between ethyl acetate (20ml) and dilute aqueous $NaHCO_3$ solution (20ml). The organic solution was washed with brine (5ml), dried ($MgSO_4$) and evaporated in vacuo. The resulting amine was dissolved in DMF (5ml) and the solution was treated successively with sodium iodide (41.5mg) and sodium (E)-3-[4,5-di(4-methoxybenzyloxy)pyridin-2-yl]propenoate (246mg). The mixture was stirred in the absence of light for 2h and then partitioned between ethyl acetate (50ml) and dil. $NaHCO_3$ solution (50ml). The organic phase was washed further with dil. $NaHCO_3$ (2 × 50ml) and brine (20ml), then dried ($MgSO_4$) and evaporated in vacuo. The crude product was chromatographed on silica eluting with a gradient of 70% ethyl acetate-hexane to ethyl acetate. Fractions containing the title cephem were combined and evaporated to yield a foam (34mg); $\nu_{max}$ ($CH_2Cl_2$) 1780, 1720, 1615 and 1515cm$^{-1}$; $\delta_H$ ($CDCl_3$) 3.40 and 3.58 (each 1H, d, J 18.5Hz), 3.77, 3.81 and 3.82 (each 3H, s), 4.75 and 4.92 (each 1H, d, J 5.1Hz), 4.93 and 5.22 (each 1H, d, J ca. 13Hz), 5.16 (4H, s), 5.23 (2H, centre of AA′), 6.71 and 7.54 (each 1H, d, J 15.4Hz), 6.87, 6.89 and 6.93 (each 2H, d, J 8.8Hz), 7.00 (1H, s), 7.34 (6H, m), and 8.20 (1H, s); [F.A.B. (+ve ion, Thioglycerol) MH+, 754].

PREPARATION 46

4-Methoxybenzyl (6R,7R)-7-[(Z)-2-[2-(2-Chloroacetamido)thiazol-4-yl]-2-methoxyiminoacetamido]-3-[(E)-[4,5-di(4-methoxybenzyloxy)pyridin-2-yl]propenoyloxymethyl]ceph-3-em-4-carboxylate

A solution of 4-methoxybenzyl (6R,7R)-7-amino-3-[(E)-[4,5-di(4-methoxybenzyloxy)pyridin-2-yl]propenoyloxymethyl]ceph-3-em-4-carboxylate (95mg) in dry $CH_2Cl_2$ (10ml) was cooled in an ice-bath. Pyridine (24mg) was added followed by (Z)-[2-(2-chloroacetamido)thiazol-4-yl]-2-methoxyiminoacetyl chloride hydrochloride (53mg) with stirring. After 10 min, the solution was diluted with $CH_2Cl_2$ (20ml) and washed with water (30ml) and brine (15ml). The solution was dried ($MgSO_4$) and evaporated in vacuo to yield a crude product which was chromatographed on silica eluting with a gradient of 60% to 80% ethyl acetate in hexane. Fractions containing the title cephem were combined and evaporated to give a foam (90mg); $\nu_{max}$ (KBr) 1784, 1713, 1680, 1611, 1542 and 1512cm$^{-1}$; $\delta_H$ ($CDCl_3$) 3.46 and 3.61 (each 1H, d, J 18.6Hz), 3.79, 3.80 and 3.82 (each 3H, s), 4.10 (3H, s), 4.25 (2H, s), 4.98 (1H, d, J 13.8Hz), 5.08 (1H, d, J 4.9Hz), 5.15-5.30 (7H, m), 6.02 (1H, dd, J 4.9 and 9.1Hz), 6.69 (1H, d, J 15.7Hz), 6.87-6.94 (6H, m), 6.99 (1H, s), 7.31-7.37 (7H, m), 7.42 (1H, d, J 9Hz), 7.55 (1H, d, J 15.7Hz), 8.20 (1H, s), 10.12 (1H, s); [F.A.B. (+ve ion, Thioglycerol) MH+ 1013].

EXAMPLE 28

(6R,7R)-7-[(Z)-2-[2-(2-Chloroacetamido)thiazol-4-yl] 2-methoxyiminoacetamido]-3-[(E)-3-(5-hydroxy-4-oxo-1,4-dihydropyridin-2-yl)propenoyloxy]methylceph-3-em-4-carboxylic acid

A solution of 4-methoxybenzyl (6R,7R)-7-[(Z)-2-[2-(2-chloroacetamido)thiazol-4-yl]-2-methoxyiminoacetamido]-3-[(E)-[4,5-di(4-methoxybenzyloxy)pyridin-2-yl]propenoy loxymethyl]ceph-3-em-4-carboxylate (85mg) in $CH_2Cl_2$ (5ml) was cooled in an ice-bath and treated with anisole (544mg) and trifluoroacetic acid (0.388ml) with stirring. After 30 min at ice-bath temperature and 45 min at room temperature, the solution was evaporated in vacuo. The residue was twice re-diluted with $CH_2Cl_2$ (10ml) followed by evaporation, and then

triturated with ether to afford the title acid as a solid (54.5mg); $\nu_{max}$ (KBr) 1781, 1715, 1675 and 1542cm$^{-1}$; $\delta_H$ ($CD_3SOCD_3$) ca. 3.6 (2H, m, partially masked by $H_2O$ signal), 3.90 (3H, s), 4.38 (2H, s) 4.85 and 5.16 (each 1H, d, J 12.9Hz), 5.20 (1H, d, J 4.9Hz), 5.87 (1H, dd, J 4.9 and 8.3Hz), 6.74 (1H, d, J 15.9Hz), 7.13 (1H, s), 7.46 (1H, s), 7.52 (1H, d, J 15.9Hz), 7.90 (1H, s) and 9.73 (1H, d, J 8.3Hz) [F.A.B. (+ve ion, Thioglycerol) MH+, 653].

EXAMPLE 29

Sodium (6R,7R)-7-[(Z)-2-(2-Aminothiazol-4-yl)-2-methoxyiminoacetamido]-3-[(E)-3-(5-hydroxy-4-oxo-1,4-dihydropyridin-2-yl)propenoyloxymethyl]ceph-3-em-4-carboxylate

(6R,7R)-7-[(Z)-2-[2-(2-chloroacetamido)thiazol-4-yl]-2-methoxyiminoacetamido]-3-[(E)-3-(5-hydroxy-4-oxo-1,4-dihydropyridin-2-yl)propenoyloxy]methylceph-3-em-4-carboxylic acid (45mg) was suspended in water (5ml) and dilute $NaHCO_3$ was added until the solution was at pH 7.5. Sodium N-methyldithiocarbamate (18mg) was added and the mixture stirred for 2h. A further quantity (4.5mg) of sodium N-methyldithiocarbamate was added and stirring continued for 1h. The solution was washed with ethyl acetate (2 × 30ml), and then concentrated in vacuo to small volume, before loading onto a column of HP20SS. This was eluted with water followed by 50% methanol-water. The methanol-water eluant was concentrated in vacuo and the resulting aqueous solution freeze-dried to afford the title sodium salt (28mg); $\nu_{max}$ (KBr) 1762, 1705, 1609 and 1533cm$^{-1}$; [F.A.B. (+ve ion, Thioglycerol), MH+, 599].

PREPARATION 47

4-Methoxybenzyl (6R,7R)-7-Amino-3-[4,5-di(4-methoxybenzyloxy)pyridin-2-yl]carbonyloxymethylceph-3-em-4-carboxylate

4-Methoxybenzyl (6R,7R)-7-amino-3-chloromethylceph-3-em-4-carboxylate (0.5mmol) was dissolved in dry DMF (10ml) and the flask protected from the light. NaI (75mg, 0.5mmol) was added followed by sodium 4,5-di(4-methoxybenzyloxy)pyridine-2-carboxylate (230mg, 0.55mmol) prepared by the general procedure of Preparation 44. The heterogeneous mixture was stirred for 22 hours. EtOAc (60ml) was then added and the mixture washed twice with saturated aqueous $NaHCO_3$ solution and with brine (20ml) then dried over $MgSO_4$. Evaporation of the solvent under reduced presure gave an orange/ brown oil which was subjected to silica gel column chromatography. Elution with EtOAc/hexane mixtures gave the title compound as a yellow brown solid after trituration with ether/hexane (55mg); $\nu_{max}$(KBr) 1776, 1717, 1611 and 1576cm$^{-1}$; $\delta_H$ ($CDCl_3$) 8.25 (1H, s), 7.70 (1H, s), 7.45-7.2 (6H, m), 7.0-6.8 (6H, m), 5.43 and 5.12 (each 1H, d, J 13.5Hz), 5.23 (2H, s), 5.21 (2H, s), 5.19 (2H, s), 4.92 (1H, d, J 4.9Hz), 4.75 (1H, d, J 4.9Hz), 3.82 (3H, s), 3.80 (3H, s), 3.79 (3H, s), 3.62 and 3.43 (each 1H, d, J 18.5Hz). [F.A.B. (+ve ion, Thioglycerol) MH+ 728].

PREPARATION 48

4-Methoxybenzyl (6R,7R)-7-[(Z)-2-[2-(2-Chloroacetamido) thiazol-4-yl]-2-methoxyiminoacetamido]-3-[4,5-di(4-methoxybenzyloxy)pyridin-2-yl]carbonyloxymethylceph-3-em-4-carboxylate

A solution of 4-methoxybenzyl (6R,7R)-7-amino-3-[4,5- di(4-methoxybenzyloxy)pyridin-2-yl]carbonyloxymethylceph-3-em-4-carboxylate (68mg, 0.093mmol) in dry $CH_2Cl_2$ (4ml) was cooled in an ice bath. Pyridine (18mg, 0.23mmol) was added followed by (Z)-[2-(2-chloroacetamido)thiazol-4-yl]-2-methoxyiminoacetyl chloride hydrochloride (37mg, 0.11mmol) with stirring. After 15 minutes the $CH_2Cl_2$ was removed by evaporation under reduced pressure. The residue was partitioned between EtOAc and saturated aqueous $NaHCO_3$ solution. The organic phase was separated and washed with saturated aqueous $NaHCO_3$ solution, twice with 0.5M aqueous HCl solution and once with brine. After drying over $MgSO_4$ evaporation under reduced pressure gave a residue which was purified by silica gel column chromatography. Elution with EtOAc/hexane mixtures provided the title compound as a cream coloured foam (58mg); $\nu_{max}$ (KBr) 1785, 1720, 1684 and 1611cm$^{-1}$; $\delta_H$ ($CDCl_3$) 10.1 (1H, s (br)), 8.25 (1H, s), 7.70 (1H, s), 7.43-7.23 (8H, m), 6.96-6.85 (6H, m), 6.02 (1H, dd, J 9.1 and 4.9Hz), 5.48 and 5.15 (each 1H, d, J 13.7Hz), 5.23 (2H, s), 5.20 (2H, s), 5.18 (2H, s), 5.07 (1H, d, J 4.9Hz), 4.27 (2H, s), 4.11 (3H, s), 3.82 (3H, s), 3.80 (6H, s), 3.65 and 3.47 (each 1H, d, J 18.5Hz). [F.A.B. (+ve ion, Thioglycerol) MH+ 987].

EXAMPLE 30

(RS)1-Acetoxyethyl (6R,7R)-7-[2-(2-amino-4-thiazolyl)-2(Z)-methoxyiminoacetamido]-3-[3,4-dihydroxybenzoyloxy] methylceph-3-em-4-carboxylate

Sodium (6R,7R)-7-[2-(2-amino-4-thiazolyl)-2-(Z)-methoxyiminoacetamido]-3-[3,4-dihydroxybenzoyloxy-]methylceph-3-em-4-carboxylate (115mg, 0.2mmol) was dissolved in DMF (5ml) and treated with 1-bromoethyl acetate (67mg). The solution was stirred for 30 mins and partitioned between ethyl acetate and water. The organic phase was washed with water ( × 3) and brine and then dried over $MgSO_4$. The product was purified by chromatography on silica gel eluting with mixtures of ethyl acetate and hexane. Fractions containing the

product were combined and evaporated to give the title compound as a white foam in 22% yield; $\nu_{max}$ (THF) 1790, 1765, 1715, 1690cm$^{-1}$; $\delta_H$ [$CD_3COCD_3$] 1.52 (3H, 2d, J 5.5Hz), 2.09 (3H, s), 3.71 (1H, 2d, J 18.5Hz), 3.87 (1H, 2d, J 18.5Hz), 3.91 (3H, s), 4.87 and 4.91 (1H, 2d, J 12.5Hz), 5.02 (1H, 2d, J 12.5Hz), 5.2-5.3 (1H, 2m), 5.97-6.07 (1H, 2m), 6.85-6.95 (2H, m), 7.00 and 7.10 (1H, 2q, J 5.5Hz), 7.40-7.55 (2H, m). [F.A.B. (+ve ion, Thioglycerol) MH+ 636].

Example 31

(6R,7R)-7-[(Z)-2-[2-(2-Chloroacetamido)thiazol-4-yl]-2-methoxyiminoacetamido]-3-(5-hydroxy-4-oxo-1,4-dihydropyridin-2-yl)carbonyloxymethylceph-3-em-4-carboxylic acid

A stirred solution of 4-methoxybenzyl (6R,7R)-7-[(Z)-2-[2-(2-chloroacetamido)thiazol-4-yl]-2-methoxyiminoacetamido]-3-[4,5-di(4-methoxybenzyloxy)pyridin-2-yl]carbonyloxymethylceph-3-em-4-carboxylate (50mg, 0.051mmol) and anisole (330µl, 329mg, 3.04mmol) in dry $CH_2Cl_2$ (3ml) was cooled in an ice bath. TFA (234µl, 347mg, 3.04mmol) was added and the solution stirred for 45 minutes. Volatile components were than removed by evaporation under reduced pressure and the oily residue triturated with ether to give the title compound as a cream coloured solid (34mg); $\nu_{max}$ (KBr) 1781, 1736, 1676, 1623 and 1550cm$^{-1}$; $\delta_H$ ($CD_3SOCD_3$) 12.95 (1H, s), 9.75 (1H, d, J 8.2Hz), 7.92 (1H, s), 7.47 (1H, s), 7.38 (1H, s), 5.88 (1H, dd, J 8.2 and 4.9Hz), 5.22 (1H, d, J 4.9Hz), 5.25 and 4.96 (each 1H, d, J 13.0Hz), 4.40 (2H, s, 3.91 (3H, s) 3.4 (2H m, partially obscured by HOD signal). [F.A.B. (+ve ion, Thioglycerol) MH+ 627].

PREPARATION 49

3,4-Diacetoxythiobenzoic acid

3,4-Diacetoxybenzoyl chloride (2g) was dissolved into dichloromethane (100ml) and treated with pyridine (0.64ml). The reaction was cooled to -20°C and $H_2S$ gas passed through the solution. After 3 hours argon was substituted for $H_2S$ and passed through the solution for 2 hours. The solvent was evaporated under vacuum and the residue taken up into a small quantity of tetrahydrofuran and neutralised with sodium bicarbonate solution. The aqueous solution was extracted with ethyl acetate (×2) and finally acidified to pH 2 with dilute HCl and further extracted with ethyl acetate. This last extract was evaporated to a yellow oil which slowly solidified at 0°C. Thus the title compound was obtained in 63% yield); $\nu_{max}$ ($CH_2Cl_2$) 1773 and 1677cm$^{-1}$.

PREPARATION 50

(6R,7R) p-Methoxybenzyl 7-Amino-3-(3,4-diacetoxybenzoylthiomethyl)-ceph-3-em-4-carboxylate

p-Methoxybenzyl 7-amino-3-chloromethyl-ceph-3-em-4-carboxylate (0.3917g) was dissolved into dichloromethane (5ml) and treated sequentially with 3,4-diacetoxythiobenzoic acid (0.29g) and pyridine (0.09ml). The reaction was diluted with ethyl acetate after four hours. Subsequent washings with brine (×2), dilute hydrochloric acid and finally a sodium bicarbonate solution, were followed by drying ($MgSO_4$) and evaporation. The title compound was thus obtained in 95% yield (0.6g); $\nu_{max}$ ($CH_2Cl_2$) 1773 and 1720cm$^{-1}$; $\delta_H$ ($CDCl_3$) 1.5-1.9 (2H, br s, disappears on $D_2O$ shake), 2.33 (3H, s), 2.34 (3H, s), 3.36 and 3.63 (2H, ABq, J 18.5z), 3.81 (3H, s), 3.98 and 4.31 (2H, ABq, J 13.5Hz), 4.72 (1H, d, J 5Hz), 4.90 (1H, d, J 5Hz), 5.24 and 5.28 (2H, ABq, J 11.8Hz), 6.90 (2H, d, J 8.7Hz), 7.32 (1H, d, J 8.5Hz), 7.39 (2H, d, J 8.7Hz), 7.80 (1H, d, J 2Hz), and 7.86 (1H, dd, J 8.5 and 2Hz), m/e (FAB, thioglycerol) 587 (32%, MH+).

PREPARATION 51

(6R,7R) p-Methoxybenzyl 7-(2-[2-(Chloroacetylamino)thiazol-4-yl]-2[Z]-methoximinoacetamido)-3-(3,4-diacetoxybenzoylthiomethyl)ceph-3-em-4-carboxylate

The ester from Preparation 50 (0.4268g) was dissolved into dichloromethane (12ml) and treated sequentially with 2-[2-(chloroacetylamino)thiazol-4-yl]-2[Z]-methoximinoacetyl chloride (0.27g) and pyridine (0.13ml). The reaction was diluted with ethyl acetate after 1 hour and washed with brine (×2). Evaporation and chromatography on silica, eluting with toluene/ethyl acetate (1:1), afforded the title compound in 32% yield (0.35g); $\nu_{max}$ (KBr) 3313, 1775, 1717 and 1663cm$^{-1}$; $\delta_H$ ($CDCl_3$) 2.34 (6H, s), 3.43 and 3.67 (2H, ABq, J 18.6Hz), 3.83 (3H, s), 4.02 and 4.35 (2H, ABq, J 13.5Hz), 4.11 (3H, s), 4.28 (2H, s), 5.07 (1H, d, J 4.9Hz), 5.24 and 5.27 (2H, ABq, J 12.2Hz), 5.99 (1H, dd, J 4.9 and 9.2Hz), 6.90 (2H, d, J 8.7Hz), 7.27 (1H, d, J 9.2Hz), 7.32 (1H, d, J 8.5Hz), 7.38 (2H, d, J 8.7Hz), 7.39 (1H, s), 7.80 (1H, d, J 2.1Hz), 7.86 (1H, dd, J 8.5 and 2.1Hz), addition of $D_2O$ caused the doublet at δ7.27 to disappear and the doublet of doublets at δ5.99 to collapse to a doublet (J 4.9Hz).

EXAMPLE 32

Minimum inhibitory Concentration (MIC) values of compounds of the invention against Enterobacter cloacae P99 and Pseudomonas aeruginosa 10662 were determined by serial dilution in agar. The plates were inoculated with $10^4$ colony forming units and incubated overnight at 37°C. The MIC values recorded in Tables 1 and 2 were the lowest concentration of antibiotic to inhibit growth.

Comparative data for cefotaxime (compound A) and 7β-[D-2-[(4-ethyl-2,3-dioxopiperazin-1-yl) carbonylamino]-2-phenylacetamido]-7α-formamidocephalosporanic acid, sodium salt (compound B), disclosed in European Patent Application No. 82303821.1 (Publication Number 0 071 395: Example 36), are also given.

Table 1

| Organism | MIC (µg/ml) | | | | |
|---|---|---|---|---|---|
| | Example 1 | Example 3 | Example 6 | Example 7 | Compound B |
| *Enterobacter cloacae* P99 | 0.12 | 4 | 0.5 | 0.25 | 16 |
| *Pseudomonas aeruginosa* 10662 | ≤0.03 | 4 | 0.12 | ≤0.03 | 16 |

Table 2

| Organism | MIC (µg/ml) | |
|---|---|---|
| | Example 10 | Compound A |
| *Enterobacter cloacae* P99 | 4 | ≥128 |
| *Pseudomonas aeruginosa* 10662 | 0.12 | 16 |

**Claims**

1. A compound of the formula (I) or a salt thereof:

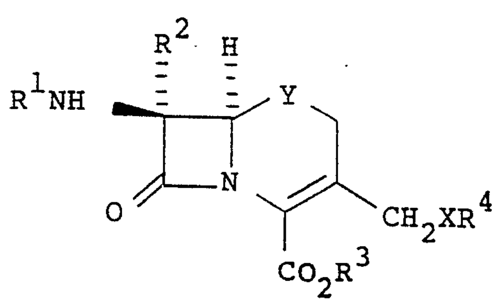

(I)

wherein $R^1$ is an acyl group, in particular that of an antibacterially active cephalosporin; $R^2$ is hydrogen, methoxy, or formamido; $R^3$ is hydrogen or a readily removable carboxy protecting group; Y is S, SO, $SO_2$, O or $CH_2$; X is oxygen, sulphur, or -NH- and $R^4$ is a group of the formula-CO-Z-$R^5$ wherein Z is -CH=CH-, $-(CH_2)_n-$or -NH-; n is 0, 1 or 2; and $R^5$ is:

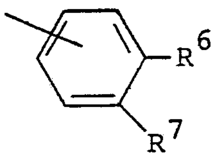 , 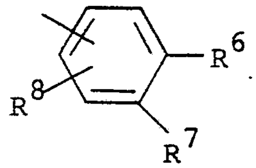 or 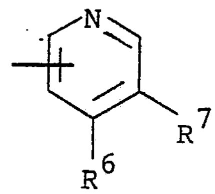

wherein $R^6$ and $R^7$ are the same or different, each representing hydroxy or protected hydroxy and $R^8$ is hydroxy, amino, halogen or carboxy.

2. A compound as claimed in Claim 1 having the formula (Ia) or a pharmaceutically acceptable salt or pharmaceutically acceptable in vivo hydrolysable ester thereof:

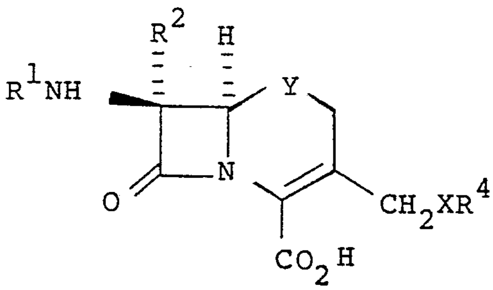

(Ia)

3. A compound as claimed in Claim 1 or Claim 2 in which Y is sulphur.

4. A compound as claimed in any one of Claims 1 to 3 in which the group $R^1$ is a group of the formula $R^{11}CO$- wherein $R^{11}CO$ has one of the subformulae

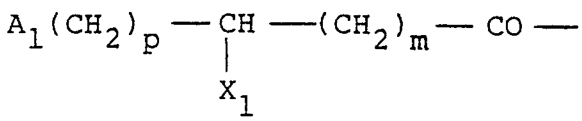

(a)

$A_2CO—$ (b)

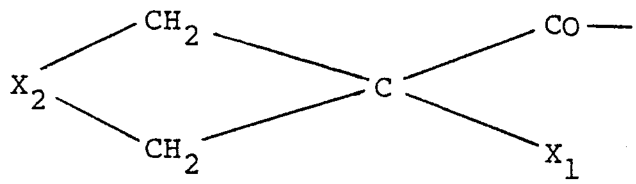

(c)

$A_2—X_3—(CH_2)_p—CO—$ (d)

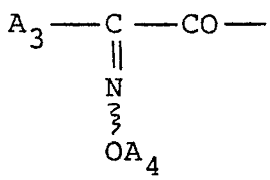

(e)

wherein p is 0, 1 or 2; m is 0, 1 or 2; $A_1$ is $C_{1-6}$alkyl, substituted $C_{1-6}$ alkyl, $C_{3-6}$ cycloalkyl, cyclohexenyl, cyclohexadienyl, an optionally substituted aromatic group, a $C_{1-6}$ alkylthio group or $C_{1-6}$ alkyloxy; $X_1$ is a hydrogen or halogen atom, a carboxylic acid, carboxylic ester, sulphonic acid, azido, tetrazolyl, hydroxy, acyloxy, amino, ureido, acylamino, heterocyclylamino, guanidino or acylureido group; $A_2$ is an aromatic group, a substituted alkyl group; or a substituted dithietane; $X_2$is a $CH_2OCH_2$, $CH_2SCH_2$ or alkylene group; $X_3$ is an oxygen or sulphur atom; $A_3$ is an aryl or heteroaryl group; and $A_4$ is hydrogen, $C_{1-6}$ alkyl, $C_{3-6}$ cycloalkyl, arylaminocarbonyl, $C_{1-6}$ alkylaminocarbonyl, $C_{1-6}$alkanoyl, $C_{1-6}$ alkoxycarbonyl, $C_{2-6}$ alkenyl, carboxy $C_{1-6}$ alkyl, $C_{1-6}$ alkylsulphonyl, di-$C_{1-6}$alkylphosphatomethyl, or aryl.

5. A compound as claimed in Claim 4 in which $R^{11}CO$ is a group of the subformula:

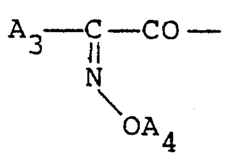

having the *syn* configuration wherein $A_3$ and $A_4$ are as defined in Claim 4 and in which $R^2$ is hydrogen.

6. A compound as claimed in Claim 4 or Claim 5 in which $A_3$ is phenyl, 2-aminothiazol-4-yl, fur-2-yl, thien-2-yl or 2-(2-chloroacetamido)thiazol-4-yl.

7. A compound as claimed in any one of Claims 4 to 6 in which $A_4$ is methyl, cyclopentyl or 2-carboxypropan-2-yl.

8. A compound as claimed in Claim 4 in which $R^{11}$ is a group of the subformula (f) or (g):

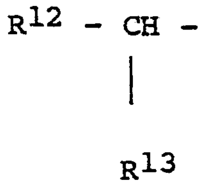

(f)

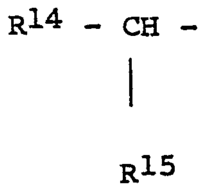

(g)

wherein $R^{12}$ is a phenyl, thienyl or phenoxy group; $R^{13}$ is a hydrogen atom or methyl group; $R^{14}$ is a phenyl, substituted phenyl, substituted thiazolyl, thienyl or cyclohexadienyl group; and $R^{15}$ is a hydroxyl, carboxylic acid group or lower alkyl or phenyl, tolyl or indanyl ester thereof, amino or a substituted amino group,
and $R^2$ is methoxy or formamido.

9. A compound as claimed in Claim 8 having the formula (VI):

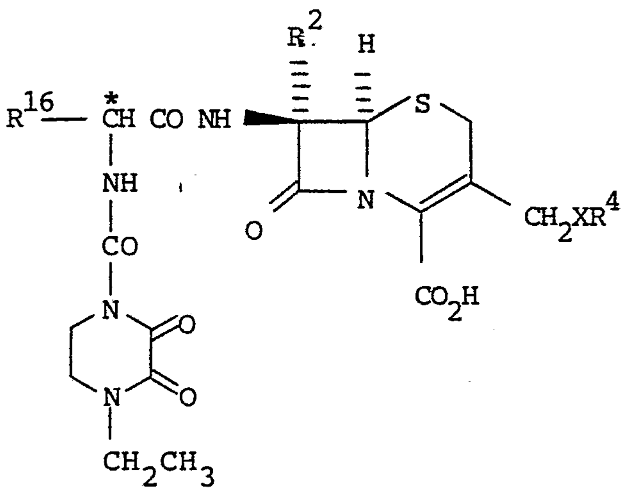

(VI)

in which $R^{16}$ is phenyl, thien-2-yl, thien-3-yl, 4-hydroxyphenyl or 4-acetoxyphenyl and X and $R^4$ are as defined with respect to formula (I) in Claim 1.

10. A compound as claimed in any one of Claims 1 to 9 in which $R^4$ is 2,3-dihydroxybenzoyl, 3,4-dihydroxybenzoyl, or (5-hydroxy-4-oxo-1,4-dihydropyridin-2-yl)carbonyl in which the hydroxy groups are optionally protected.

11. A compound of the formula (Ia) as defined in Claim 2 selected from the following or a pharmaceutically acceptable salt or pharmaceutically acceptable in vivo hydrolysable ester thereof:
(6R, 7R)-3-(3,4-Dihydroxybenzoyloxy)methyl-7-[(R)-2-(4-ethyl-2,3-dioxopiperazin-1-yl)carbonylamino-2-phenylacetamido]-7-formamidoceph-3-em-4-carboxylic acid;
(6R, 7R)-3-(3,4-Dihydroxycinnamoyloxy)methyl-7-[(R)-2-(4-ethyl-2,3-dioxopiperazin-1-yl)carbonylamino-2-phenylacetamido]-7-formamidoceph-3-em-4-carboxylic acid;
(6R,7R)-3-(3,4-Diacetoxybenzoyl)aminomethyl-7-[(R)-2-(4-ethyl-2,3-dioxopiperazin-1-ylcarbonylamino)-2-phenylacetamido]-7-formamidoceph-3-em-4-carboxylic acid;
(6R, 7R)-3-[(E)-3,4-Diacetoxycinnamoyl]aminomethyl-7-[(R)-2-(4-ethyl-2,3-dioxopiperazin-1-yl-carbonylamino)-2-phenylacetamido]-7-formamido-ceph-3-em-4-carboxylic acid;
(6R,7R)-3-(3,4-Dihydroxybenzoyl)aminomethyl-7-[(R)-2-(4-ethyl-2,3-dioxopiperazin-1-ylcarbonylamino)-2-phenylacetamido]-7-formamido-ceph-3-em-4-carboxylic acid;
(6R,7R)-3-(5-Hydroxy-4-oxo-1,4-dihydropyridin-2-yl)carbonylaminomethyl-7-[(R)-2-(4-ethyl-2,3-dioxopiperazin-1-ylcarbonylamino)-2-phenylacetamido]-7-formamidoceph-3-em-4-carboxylic acid;
(6R,7R)-3-(2,3-Dihydroxybenzoyl)aminomethyl-7-[(R)-2-(4-ethyl-2,3-dioxopiperazin-1-ylcarbonylamino)-2-phenylacetamido]-7-formamidoceph-3-em-4-carboxylic acid;

[6R, 7R]-7-[2-(2-amino-4-thiazolyl)-2((Z)-methoxyimino)acetamido]-3-(3,4-dihydroxybenzoyloxy) methylceph-3-em-4-carboxylic acid;
(6R, 7S)-3-[(3,4-Dihydroxybenzoyloxy)methyl]-7-[(R)-2-(4-ethyl-2,3-dioxopiperazin-1-yl)carbonylamino-2-phenylacetamido]-7-methoxyceph-3-em-4-carboxylic acid;
(6R, 7R)-3-(3,4-Dihydroxybenzoyloxy)methyl-7-[(S)-2-(4-ethyl-2,3-dioxopiperazin-1-yl)carbonylamino--2-(2-thienyl)acetamido]-7-formamidoceph-3-em-4-carboxylic acid;
(6R, 7R)-3-[(3,4-dihydroxyphenyl)acetoxymethyl]-7-[(R)-2-(4-ethyl-2,3-dioxopiperazin-1-yl)carbonylamino-2-phenylacetamido]-7-formamidoceph-3-em-4-carboxylic acid;
(6R, 7R)-7-[(R)-2-(4-Ethyl-2,3-dioxopiperazin-1-yl)carbonylamino-2-phenylacetamido]-7-formamido--3-(3,4, 5-trihydroxybenzoyloxy)methylceph-3-em-4-carboxylic acid;
(6R,7R)-3-[(3,4-Dihydroxyphenyl)carbamoyloxymethyl]-7-[(R)-2-(4-ethyl-2,3-dioxopiperazin-1-yl)carbonylamino-2-phenylacetamido]-7-formamidoceph-3-em-4-carboxylic acid;
(6R,7R)-3-(2,3-Dihydroxycinnamoyloxy)methyl-7-[(R)-2-(4-ethyl-2,3-dioxopiperazin-1-yl)carbonylamino-2-phenylacetamido]-7-formamidoceph-3-em-4-carboxylic acid;
(6R,7R)-7-[(R)-2-(4-Ethyl-2,3-dioxopiperazin-1-yl) carbonylamino-2-phenylacetamido]-7-formamido--3-(5-hydroxy-4-oxo-1,4-dihydropyridin-2-yl)carbonyloxymethylceph-3-em-4-carboxylic acid;
(6R,7R)-7-[(R)-2-(4-Ethyl-2,3-dioxopiperazin-1-ylcarbon ylamino)-2-phenylacetamido]-7-formamido-3-[(E)-3-(5-hydroxy-4-oxo-1,4-dihydropyridin-2-yl)prop-2-enoyloxymethyl]ceph-3-em-4-carboxylic acid;
[6R,7R]-3-(3,4-Dihydroxybenzoyloxy)methyl-7-(2-phenylacetamido)ceph-3-em-4-carboxylic acid;
[6R,7R]-7-[Z-2-(2-aminothiazol-4-yl)-2-cyclopentyloxyiminoacetamido]3-(3,4-dihydroxybenzoyloxy)methylceph-3-em-4-carboxylic acid;
[6R,7R]-7-[(R)-2-azido-2-phenylacetamido]-3-(3,4-dihydroxybenzoyloxy)methylceph-3-em-4-carboxylic acid;
[6R,7R]-7-[Z-2-(2-Aminothiazol-4-yl)-2-(2-carboxypropan-2-yloxyimino)acetamido]-3-(3,4-dihydroxybenzoyloxy)methylceph-3-em-4-carboxylic acid;
[6R,7R]-7-[2-(2-amino-4-thiazolyl)-2-([Z]-methoxyimino)acetamido]-3-(3,4-dihydroxycinnamoyloxy)methylceph-3-em-4-carboxylic acid;
(6R,7R)-3-[(3,4-Dihydroxyphenyl)carbamoyloxy]methyl-7-[2-(2-furyl)-2-(Z)-methoxyiminoacetamido-]ceph-3-em-4-carboxylic acid;
(6R,7R)-7-[3-(2-Chloro-6-fluorophenyl)-5-methylisoxazol-4-yl]carbonylamino-3-(3,4-dihydroxybenzoyloxy)methylceph-3-em-4-carboxylic acid;
(6R,7R)-3-(3,4-Dihydroxybenzoyloxy)methyl-7-(2-(Z)-methoxyimino-2-phenylacetamido)ceph-3-em-4-carboxylic acid;
(6R,7R)-3-(3,4-Dihydroxybenzoyloxy)methyl-7-[2-(E)-methoxyimino-2-(2-thienyl)acetamido]ceph-3-em-4-carboxylic acid;
(6R,7R)-7-[(Z)-2-[2-(2-Chloroacetamido)thiazol-4-yl]2-methoxyiminoacetamido]-3-[(E)-3-(5-hydroxy-4-oxo-1,4-dihydropyridin-2-yl)propenoyloxy]methylceph-3-em-4-carboxylic acid;
(6R,7R)-7-[(Z)-2-(2-Aminothiazol-4-yl)-2-methoxyiminoacetamido]-3-[(E)-3-(5-hydroxy-4-oxo-1,4-dihydropyridin-2-yl)propenoyloxymethyl]ceph-3-em-4-carboxylic acid; and
(6R,7R)-7-[(Z)-2-[2-(2-Chloroacetamido)thiazol-4-yl]-2-methoxyiminoacetamido]-3-(5-hydroxy-4-oxo-1,4-dihydro pyridin-2-yl)carbonyloxymethylceph-3-em-4-carboxylic acid.

12. A process for the preparation of a compound of formula (I):

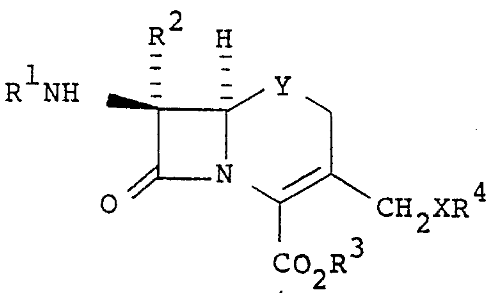

(I)

wherein $R^1$ is an acyl group, in particular that of an antibacterially active cephalosporin; $R^2$ is hydrogen, methoxy, or formamido; $R^3$ is hydrogen or a readily removable carboxy protecting group; Y is S, SO, $SO_2$, O or $CH_2$; X is oxygen, sulphur, or -NH- and $R^4$ is a group of the formula CO Z $R^5$ wherein Z is -CH=CH-, $-(CH_2)n-$ or -NH-; n is 0, 1 or 2; and $R^5$ is:

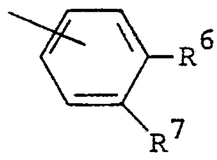 , 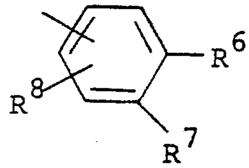 or 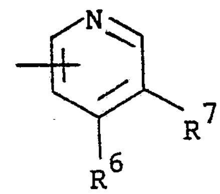

wherein $R^6$ and $R^7$ are the same or different, each representing hydroxy or protected hydroxy and $R^8$ is hydroxy, amino, halogen or carboxy,
which process comprises treating a compound of formula (XI):

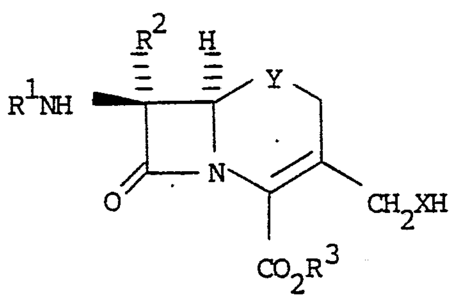

(XI)

wherein $R^1$, $R^2$, $R^3$, Y and X are as hereinbefore defined and any reactive groups may be protected; with either

a) (when Z is not NH) an acylating derivative of an acid of formula (XII):

$R^4$ OH (XII)

wherein $R^4$ is as defined in respect of formula (I) and any reactive groups may be protected; or

b) (when Z is NH) an isocyanate of the formula (XIIA):

$R^5$ NCO (XIIA)

wherein $R^5$ is as defined in respect of formula (I) and any reactive groups may be protected;

and after step a) or step b), if necessary, carrying out one or more of the following steps:

(i) converting the group $R^3$ into a different group $R^3$;
(ii) removing any protecting groups;
(iii) converting the group Y into a different group Y;
(iv) converting the product into a salt.

13. A process for the preparation of a compound of formula (I):

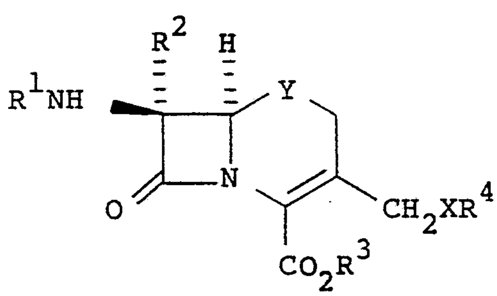

(I)

wherein $R^1$ is an acyl group, in particular that of an antibacterially active cephalosporin; $R^2$ is hydrogen, methoxy, or formamido; $R^3$ is hydrogen or a readily removable carboxy protecting group; Y is S, SO, $SO_2$, O or $CH_2$; X is oxygen, sulphur, or -NH- and $R^4$ is a group of the formula CO Z $R^5$ wherein Z is -CH=CH-, $-(CH_2)n-$ or -NH-; n is 0, 1 or 2; and $R^5$ is:

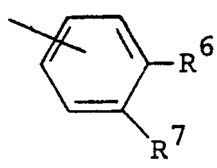 , 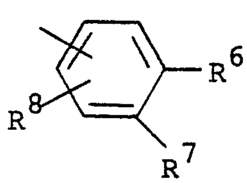 or 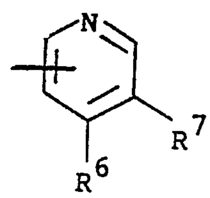

wherein $R^6$ and $R^7$ are the same or different, each representing hydroxy or protected hydroxy and $R^8$ is hydroxy, amino, halogen or carboxy,
which process comprises treating a compound of formula (IX) or a salt thereof:

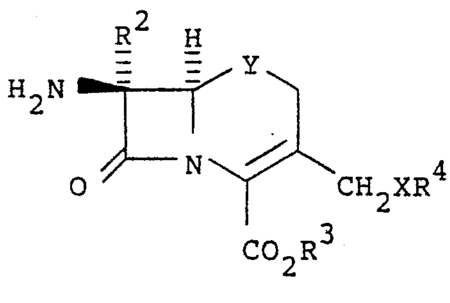

(IX)

wherein $R^2$, $R^3$, Y, X, and $R^4$ are as hereinbefore defined, wherein any reactive groups may be protected, and wherein the amino group is optionally substituted with a group which permits acylation to take place; with an N-acylating derivative of an acid of formula (X):

$R^1OH$ (X)

wherein $R^1$ is as defined with respect to formula (I) and wherein any reactive groups may be protected; and thereafter, if necessary or desired, carrying out one or more of the following steps:

i) removing any protecting groups;
ii) converting the group $R^3$ into a different group $R^3$;
iii) converting the group Y into a different group Y;
iv) converting the product into a salt.

14. A compound of the formula (XXIV) or a pharmaceutically acceptable salt or pharmaceutically acceptable in vivo hydrolysable ester thereof:

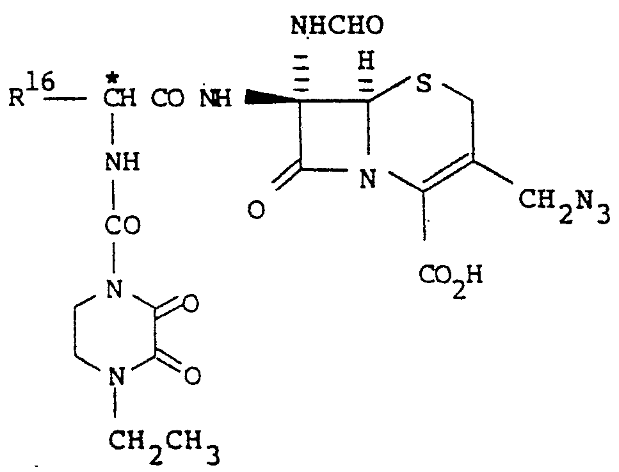

(XXIV)

in which $R^{16}$ is phenyl, substituted phenyl, cyclo cyclohexenyl, cyclohexadienyl, or a 5- or 6- membered heterocyclic ring containing up to three hetero-atoms selected from oxygen, sulphur or nitrogen, optionally substituted with hydroxy, amino, halogen, $C_{1-6}$alkylamino, di($C_{1-6}$ alkyl) amino, or $C_{1-6}$ alkoxy.

15. The use of a compound of formula (Ia) as defined in Claim 2 or a compound of formula (XXIV) as defined in Claim 14, or a pharmaceutically acceptable salt or pharmaceutically acceptable in vivo hydrolysable ester thereof, in the manufacture of a pharmaceutical composition for the treatment of

bacterial infections in humans and animals.